(19) Europäisches Patentamt
European Patent Office
Office européen des brevets

(11) EP 4 656 773 A1

(12) **EUROPEAN PATENT APPLICATION**
published in accordance with Art. 153(4) EPC

(43) Date of publication:
**03.12.2025 Bulletin 2025/49**

(21) Application number: **24747244.2**

(22) Date of filing: **22.01.2024**

(51) International Patent Classification (IPC):
***C25B 11/085*** *(2021.01)* ***C07B 61/00*** *(2006.01)*
***C07C 1/02*** *(2006.01)* ***C07C 11/04*** *(2006.01)*
***C07D 471/04*** *(2006.01)* ***C07D 471/22*** *(2006.01)*
***C07F 1/08*** *(2006.01)* ***C25B 1/04*** *(2021.01)*
***C25B 3/03*** *(2021.01)* ***C25B 3/26*** *(2021.01)*
***C25B 9/00*** *(2021.01)* ***C25B 11/032*** *(2021.01)*
***C25B 11/052*** *(2021.01)* ***C25B 11/054*** *(2021.01)*
***C25B 11/065*** *(2021.01)* ***C25B 11/069*** *(2021.01)*

(52) Cooperative Patent Classification (CPC):
**B01J 31/22; C07B 61/00; C07C 1/02; C07C 11/04; C07D 471/04; C07D 471/22; C07F 1/08; C25B 1/04; C25B 3/03; C25B 3/26; C25B 9/00; C25B 11/032; C25B 11/052; C25B 11/053; C25B 11/054;** (Cont.)

(86) International application number:
**PCT/JP2024/001599**

(87) International publication number:
**WO 2024/157916 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.01.2023 JP 2023009665**
**04.10.2023 JP 2023173256**

(71) Applicant: **Sumitomo Chemical Company, Limited**
**Tokyo 103-6020 (JP)**

(72) Inventors:
- **MASE, Kentaro**
  **Osaka-shi, Osaka 554-8558 (JP)**
- **ABE, Masanori**
  **Osaka-shi, Osaka 554-8558 (JP)**
- **KAMIKAWA, Takashi**
  **Osaka-shi, Osaka 554-8558 (JP)**

(74) Representative: **Vossius & Partner Patentanwälte Rechtsanwälte mbB**
**Siebertstrasse 3**
**81675 München (DE)**

# (54) ETHYLENE PRODUCTION METHOD, CARBON DIOXIDE REDUCTION ELECTRODE, AND CARBON DIOXIDE REDUCTION APPARATUS

(57) An object of the present invention is to provide an ethylene production method having high ethylene selectivity, a carbon dioxide reduction electrode, and a carbon dioxide reduction apparatus. The present invention relates to an ethylene production method including a step of reacting carbon dioxide with water in the presence of a carbon dioxide reduction catalyst that is a multinuclear copper complex and has a copper interatomic distance of 2.8 Å or less as determined by density functional theory, a carbon dioxide reduction electrode, and a carbon dioxide reduction apparatus.

[Fig. 2]

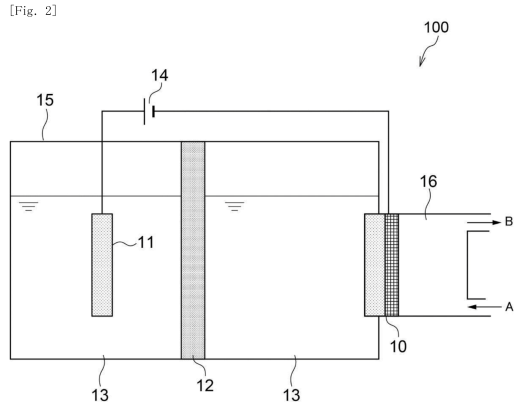


EP 4 656 773 A1

(52) Cooperative Patent Classification (CPC): (Cont.)
**C25B 11/065; C25B 11/069; C25B 11/085;**
**C25B 13/08**

**Description**

TECHNICAL FIELD

The present disclosure relates to an ethylene production method, a carbon dioxide reduction electrode, and a carbon dioxide reduction apparatus.

BACKGROUND ART

In a method for producing ethylene by reducing carbon dioxide, a carbon dioxide reduction catalyst that is a copper complex may be used.

For example, Patent Document 1 proposes "Carbon compound reduction catalyst containing a metal complex containing a ligand having a phosphorus atom and a metal halide salt having two metal atoms, at least one of which is Cu".

PRIOR ART DOCUMENT

PATENT DOCUMENT

Patent Document 1: JP-A-2021-109157

SUMMARY OF THE INVENTION

PROBLEMS TO BE SOLVED BY THE INVENTION

In an ethylene production method using a carbon dioxide reduction catalyst that is a copper catalyst, a large amount of by-products are likely to be generated, and ethylene selectivity tends to be low. Therefore, development of an ethylene production method having high ethylene selectivity is required.

An object of an embodiment of the present disclosure is to provide an ethylene production method having high ethylene selectivity.

An object of another embodiment of the present disclosure is to provide a carbon dioxide reduction electrode having high ethylene selectivity.

An object of still another embodiment of the present disclosure is to provide a carbon dioxide reduction apparatus having high ethylene selectivity.

MEANS FOR SOLVING THE PROBLEMS

Means for solving the above problems includes the following means.

<1> An ethylene production method including a step of reacting carbon dioxide with water in the presence of a carbon dioxide reduction catalyst that is a multinuclear copper complex and has a copper interatomic distance of 2.8 Å or less as determined by density functional theory.

<2> The ethylene production method according to <1>, in which the carbon dioxide reduction catalyst is a multinuclear copper complex in which a copper atom and two oxygen atoms are coordinate-bonded.

<3> An ethylene production method including a step of reacting carbon dioxide with water in the presence of a carbon dioxide reduction catalyst represented by the following Formula (1).

[Chem. 1]

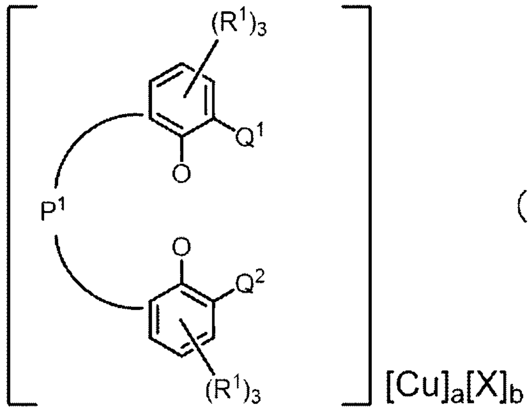

(1)

In Formula (1),

$R^1$ represents a hydrogen atom or a substituent, and a plurality of $R^1$ may be the same as or different from each other. Two adjacent $R^1$ may be bonded to each other to form a ring. $P^1$ represents a divalent group containing one or more aromatic rings. $Q^1$ and $Q^2$ represent a monovalent group containing one or more aromatic rings, and $Q^1$ and $Q^2$ may be bonded to each other to form a ring structure. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

<4> The ethylene production method according to <3>, in which $P^1$ is a divalent group represented by the following Formula ($P^a$) or the following Formula ($P^b$).

[Chem. 2]

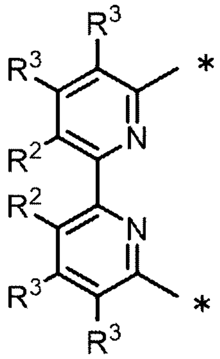

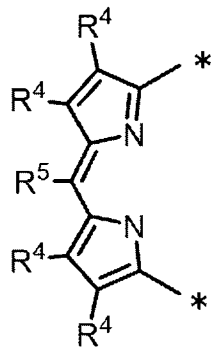

($P^a$) ($P^b$)

In Formula ($P^a$), $R^2$ and $R^3$ each independently represent a hydrogen atom or a substituent, and two adjacent $R^2$ and two adjacent $R^3$ may be bonded to each other to form a ring structure. In Formula ($P^b$), $R^4$ and $R^5$ represent a hydrogen atom or a substituent, and two adjacent $R^4$ and adjacent $R^4$ and $R^5$ may be bonded to each other to form a ring structure. A plurality of $R^4$ may be the same as or different from each other. * represents a bond.

<5> The ethylene production method according to <3>, in which the carbon dioxide reduction catalyst is a compound represented by the following Formula (2).

[Chem. 3]

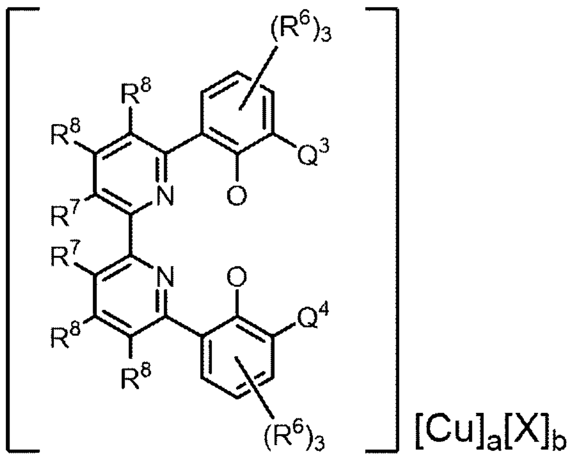

(2)

In Formula (2), $R^6$ to $R^8$ each independently represent a hydrogen atom or a substituent, and two adjacent $R^6$, two adjacent $R^7$, and two adjacent $R^8$ may be bonded to each other to form a ring structure. A plurality of $R^6$ to $R^8$ may be the same as or different from each other. $Q^3$ and $Q^4$ represent a monovalent group containing one or more aromatic rings, and $Q^3$ and $Q^4$ may be bonded to each other to form a ring structure. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

<6> The ethylene production method according to <3>, in which the carbon dioxide reduction catalyst is a compound represented by the following Formula (3).

[Chem. 4]

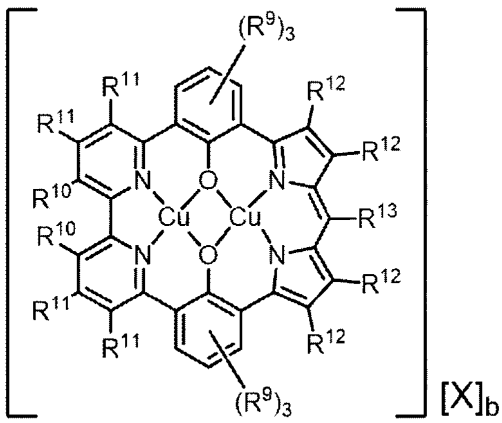

(3)

In Formula (3), $R^9$ to $R^{13}$ each independently represent a hydrogen atom, a substituent, or a divalent group, and two adjacent $R^9$, two adjacent $R^{10}$, two adjacent $R^{11}$, two adjacent $R^{12}$, and adjacent $R^{12}$ and $R^{13}$ may be bonded to each other to form a ring structure. A plurality of $R^9$ to $R^{12}$ may be the same as or different from each other. When $R^{13}$ is a divalent group, the divalent group may form a bond with another compound represented by Formula (3) to form a dimer. X is a counter ion or a neutral molecule, b is an integer of 0 or more, and when a plurality of X are present, the plurality of X may be the same as or different from each other.

<7> The ethylene production method according to <3>, in which the carbon dioxide reduction catalyst is a compound represented by the following Formula (4).

[Chem. 5]

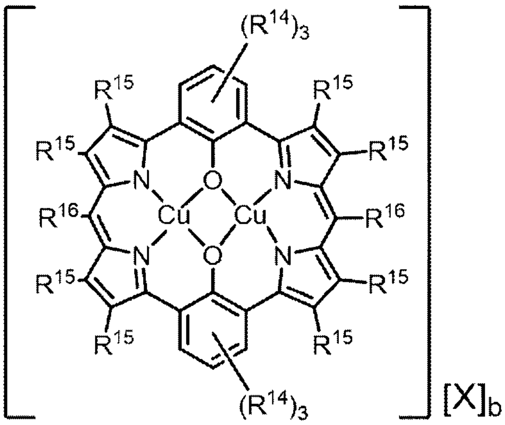

(4)

In Formula (4), $R^{14}$ to $R^{16}$ each independently represent a hydrogen atom or a substituent, and two adjacent $R^{14}$, two adjacent $R^{15}$, and adjacent $R^{15}$ and $R^{16}$ may be bonded to each other to form a ring. A plurality of $R^{14}$ to $R^{16}$ may be the same as or different from each other. X is a counter ion or a neutral molecule, b is an integer of 0 or more, and when a plurality of X are present, the plurality of X may be the same as or different from each other.

<8> The ethylene production method according to <3>, in which the carbon dioxide reduction catalyst is a compound represented by the following Formula (5).

[Chem. 6]

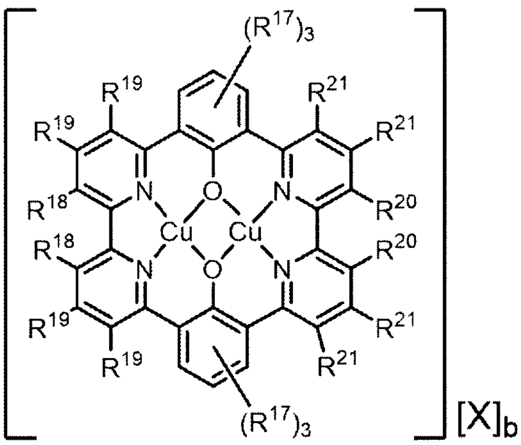

(5)

In Formula (5), $R^{17}$ to $R^{21}$ each independently represent a hydrogen atom or a substituent, and two adjacent $R^{17}$, two adjacent $R^{18}$, two adjacent $R^{19}$, two adjacent $R^{20}$, and two adjacent $R^{21}$ may be bonded to each other to form a ring. A plurality of $R^{17}$ to $R^{21}$ may be the same as or different from each other. X is a counter ion or a neutral molecule, b is an integer of 0 or more, and when a plurality of X are present, the plurality of X may be the same as or different from each other.

<9> The ethylene production method according to <3>, in which the carbon dioxide reduction catalyst is a compound represented by the following Formula (6).

[Chem. 7]

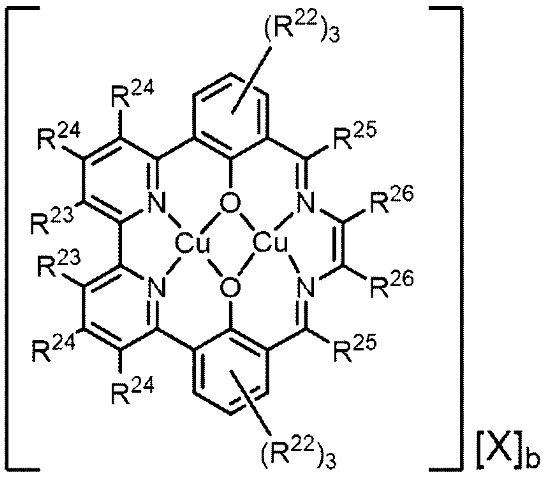

(6)

In Formula (6), $R^{22}$ to $R^{26}$ each independently represent a hydrogen atom or a substituent, and two adjacent $R^{22}$, two adjacent $R^{23}$, two adjacent $R^{24}$, two adjacent $R^{26}$, and adjacent $R^{25}$ and $R^{26}$ may be bonded to each other to form a ring structure. A plurality of $R^{22}$ to $R^{26}$ may be the same as or different from each other. X is a counter ion or a neutral molecule, b is an integer of 0 or more, and when a plurality of X are present, the plurality of X may be the same as or different from each other.

<10> The ethylene production method according to <3>, in which the carbon dioxide reduction catalyst is a compound represented by the following Formula (7).

[Chem. 8]

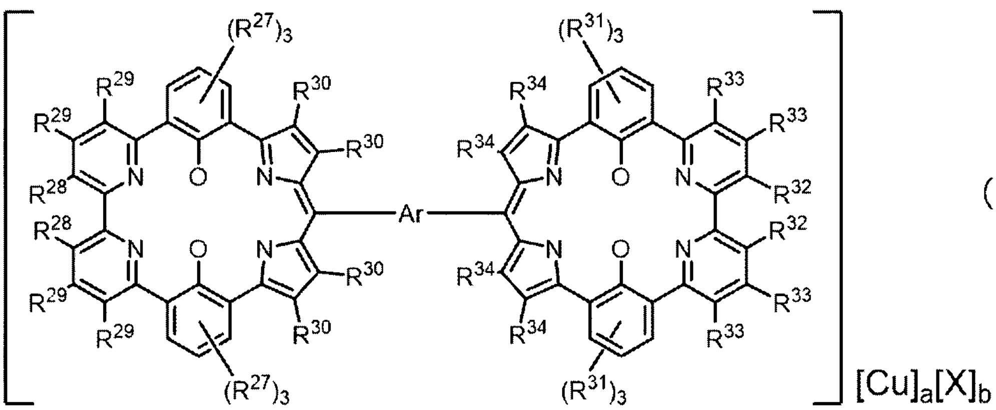

(7)

In Formula (7), $R^{27}$ to $R^{34}$ each independently represent a hydrogen atom or a substituent, and two adjacent $R^{27}$, two adjacent $R^{28}$, two adjacent $R^{29}$, two adjacent $R^{30}$, two adjacent $R^{31}$, two adjacent $R^{32}$, two adjacent $R^{33}$, and two adjacent $R^{34}$ may be bonded to each other to form a ring structure. A plurality of $R^{27}$ to $R^{34}$ may be the same as or different from each other. Ar represents a divalent aromatic group which may have a substituent. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

<11> A carbon dioxide reduction electrode including a carbon dioxide reduction catalyst that is a multinuclear copper complex and has a copper interatomic distance of 2.8 Å or less as determined by density functional theory or a conductive material on which a carbon dioxide reduction catalyst represented by the following Formula (1) is supported.

[Chem. 9]

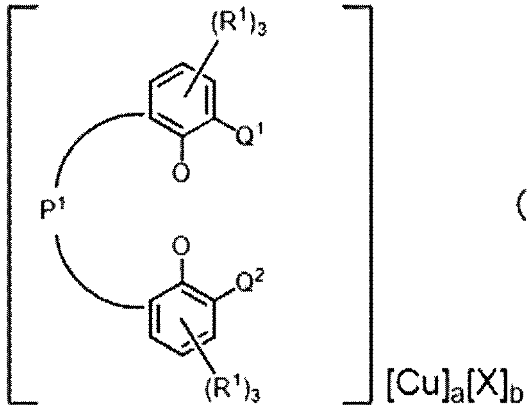

(1)

In Formula (1),

$R^1$ represents a hydrogen atom or a substituent, and a plurality of $R^1$ may be the same as or different from each other. Two adjacent $R^1$ may be bonded to each other to form a ring. $P^1$ represents a divalent group containing one or more aromatic rings. $Q^1$ and $Q^2$ represent a monovalent group containing one or more aromatic rings, and $Q^1$ and $Q^2$ may be bonded to each other to form a ring structure. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

<12> The carbon dioxide reduction electrode according to <11>, further including a support that supports the conductive material.

<13> The carbon dioxide reduction electrode according to <11> or <12>, further including an ion conductor.

<14> A carbon dioxide reduction apparatus including: an oxidation electrode; the carbon dioxide reduction electrode according to any one of <11> to <13>; a membrane separating the oxidation electrode and the carbon dioxide reduction electrode; an electrolyte solution; and a power supply connected to the oxidation electrode and the carbon dioxide reduction electrode.

EFFECT OF THE INVENTION

According to an embodiment of the present disclosure, an ethylene production method having high ethylene selectivity is provided.

According to another embodiment of the present disclosure, a carbon dioxide reduction electrode having high ethylene selectivity is provided.

According to still another embodiment of the present disclosure, a carbon dioxide reduction apparatus having high ethylene selectivity is provided.

BRIEF DESCRIPTION OF THE DRAWINGS

Fig. 1 is a schematic cross-sectional view illustrating an example of a carbon dioxide reduction electrode according to the present disclosure.

Fig. 2 is a schematic cross-sectional view illustrating an example of a carbon dioxide reduction apparatus according to the present disclosure.

MODE FOR CARRYING OUT THE INVENTION

Hereinafter, embodiments of the present disclosure will be described. These descriptions and examples illustrate embodiments and do not limit the scope of the invention.

In the present specification, a numerical range represented by "to" means a range including numerical values described before and after "to" as a lower limit value and an upper limit value.

In the numerical ranges described in stages in the present specification, an upper limit value or a lower limit value described in one numerical range may be replaced with an upper limit value or a lower limit value of the numerical range described in another stage. In addition, in the numerical range described in the present specification, the upper limit value

or the lower limit value of the numerical range may be replaced with a value shown in Examples.

In the present specification, a combination of preferred aspects is a more preferred aspect.

Each component may contain a plurality of corresponding materials.

When referring to the amount of each component in the composition, if there are a plurality of materials corresponding to each component in the composition, it means the total amount of the plurality of materials present in the composition unless otherwise specified.

The term "step" includes not only an independent step but also a step that cannot be clearly distinguished from other steps as long as the intended action of the step is achieved.

Examples of the "substituent" include a halogen atom, an alkyl group (including a cycloalkyl group), an alkenyl group, an alkynyl group, an alkoxy group, an alkylthio group, an aryl group, an aryloxy group, an arylthio group, a monovalent heterocyclic group, a substituted amino group, an acyl group, an imine residue, an amide group, an acid imide group, a substituted oxycarbonyl group, a cyano group, an alkylsulfonyl group, and a nitro group. Note that, when the number of carbon atoms is used in the present specification, the number of carbon atoms usually does not include the number of carbon atoms in the substituent A.

The "aromatic hydrocarbon ring group" means a remaining atomic group excluding one or more hydrogen atoms directly bonded to a carbon atom constituting an aromatic hydrocarbon ring which may be unsubstituted or optionally have a substituent and may have two or more condensed rings.

The "aromatic heterocyclic group" means a remaining atomic group excluding one or more hydrogen atoms directly bonded to carbon atoms or heteroatoms constituting an aromatic heterocyclic ring which may be unsubstituted or optionally have a substituent and may have two or more condensed rings.

In the compound names, "t-" means tertiary, "n-" means normal, and "p-" means para position.

<Ethylene Production Method>

An ethylene production method according to an embodiment of the present disclosure includes a step of reacting carbon dioxide with water in the presence of a carbon dioxide reduction catalyst that is a multinuclear copper complex and has a copper interatomic distance of 2.8 Å or less as determined by density functional theory.

The ethylene production method according to an embodiment of the present disclosure is an ethylene production method having high ethylene selectivity due to the above configuration. The reason is presumed as follows.

It is presumed that production of ethylene using a carbon dioxide reduction catalyst that is a multinuclear copper complex proceeds as follows: carbon dioxide is coordinated to a copper atom of the carbon dioxide reduction catalyst, two carbon dioxides coordinated to another copper atom form a bond, and a reaction with water performed, thereby producing ethylene. In order to improve the ethylene selectivity, it is considered to be effective to make it easy for two carbon dioxides coordinated to another copper atom to form a bond, and to this end, it is presumed to be effective to shorten a copper interatomic distance.

Here, the ethylene production method according to an embodiment of the present disclosure uses a carbon dioxide reduction catalyst that is a multinuclear copper complex and has a copper interatomic distance of 2.8 Å or less as determined by density functional theory. By setting the copper interatomic distance, two carbon dioxides coordinated to another copper atom easily form a bond. Therefore, it is presumed that the method is an ethylene production method having high ethylene selectivity.

The ethylene production method according to another embodiment of the present disclosure includes a step of reacting carbon dioxide with water in the presence of a carbon dioxide reduction catalyst represented by the following Formula (1).

The ethylene production method according to another embodiment of the present disclosure is an ethylene production method having high ethylene selectivity due to the above configuration. The reason is presumed as follows.

In the ethylene production method according to another embodiment of the present disclosure, a carbon dioxide reduction catalyst represented by the following Formula (1) is used. The carbon dioxide reduction catalyst has such a copper interatomic distance that makes it easy for two carbon dioxides coordinated to different copper atoms to form a bond with each other. Therefore, it is presumed that the method is an ethylene production method having high ethylene selectivity.

[Chem. 10]

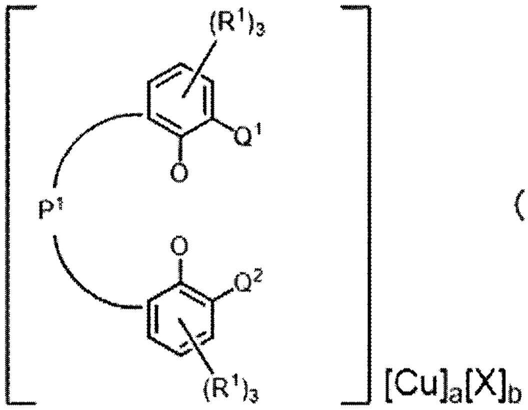

(1)

In Formula (1), $R^1$ represents a hydrogen atom or a substituent, and a plurality of $R^1$ may be the same as or different from each other. Two adjacent $R^1$ may be bonded to each other to form a ring. $P^1$ represents a divalent group containing one or more aromatic rings. $Q^1$ and $Q^2$ represent a monovalent group containing one or more aromatic rings, and $Q^1$ and $Q^2$ may be bonded to each other to form a ring structure. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

Hereinafter, an ethylene production method according to an embodiment of the present disclosure and an ethylene production method according to another embodiment of the present disclosure will be described in detail. Hereinafter, the ethylene production method according to an embodiment of the present disclosure and the ethylene production method according to another embodiment of the present disclosure are collectively referred to as "ethylene production method according to the present disclosure".

(Carbon Dioxide Reduction Catalyst)

A carbon dioxide reduction catalyst used in the ethylene production method according to the present disclosure will be described.

The carbon dioxide reduction catalyst is a multinuclear copper complex.

In an aspect, the carbon dioxide reduction catalyst has a copper interatomic distance of 2.8 Å or less, preferably 2.5 Å or more and 2.8 Å or less, more preferably 2.5 Å or more and 2.75 Å or less, and still more preferably 2.5 Å or more and 2.7 Å or less, as determined by density functional theory.

Here, the reason why the copper interatomic distance is preferably 2.5 Å or more is that it is difficult to set the copper interatomic distance to 2.5 Å or less due to the structure of the multinuclear copper complex.

. Method for Calculating Copper Interatomic Distance

The copper interatomic distance is calculated using a quantum chemistry calculation program. As the quantum chemical calculation program, for example, Gaussian16 manufactured by Gaussian, Inc. can be used.

Structure optimization calculation of the carbon dioxide reduction catalyst as a target for calculating the copper interatomic distance is performed by density functional theory (B3LYP/def2svp, sdd for Cu) to calculate the copper interatomic distance. When a self-consistent field (SCF) solution of structure optimization calculation is an unstable solution, structure optimization calculation is performed again to derive a stable solution.

From the viewpoint of ethylene selectivity, the carbon dioxide reduction catalyst is preferably a multinuclear copper complex in which at least one or more oxygen atoms are coordinate-bonded to one copper atom, and more preferably a multinuclear copper complex in which a copper atom and two oxygen atoms are coordinate-bonded.

The carbon dioxide reduction catalyst is a compound represented by the following Formula (1).

[Chem. 11]

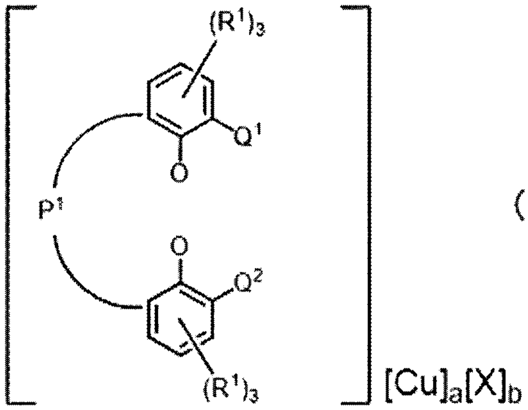

(1)

In Formula (1), $R^1$ represents a hydrogen atom or a substituent, and a plurality of $R^1$ may be the same as or different from each other. Two adjacent $R^1$ may be bonded to each other to form a ring. $P^1$ represents a divalent group containing one or more aromatic rings. $Q^1$ and $Q^2$ represent a monovalent group containing one or more aromatic rings, and $Q^1$ and $Q^2$ may be bonded to each other to form a ring structure. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

In Formula (1), $R^1$ is preferably a hydrogen atom, an alkyl group, or an alkoxy group. When $R^1$ is an alkyl group, $R^1$ is more preferably an alkyl group having 1 or more and 10 or fewer carbon atoms, and further preferably a t-butyl group. When $R^1$ is an alkoxy group, $R^1$ is more preferably an alkoxy group having 1 or more and 10 or fewer carbon atoms, and further more preferably a methoxy group.

In Formula (1), examples of the aromatic ring contained in the monovalent group represented by $Q^1$ and $Q^2$ include an aromatic hydrocarbon ring group and an aromatic heterocyclic group. The aromatic heterocyclic group as the aromatic ring contained in the monovalent group represented by $Q^1$ and $Q^2$ is preferably an aromatic heterocyclic group containing a nitrogen atom or an aromatic heterocyclic group containing a sulfur atom. When $Q^1$ and $Q^2$ are bonded to each other to form a ring structure, the ring structure is preferably, for example, a remaining atomic group obtained by removing two hydrogen atoms from phenanthroline.

a is an integer of 2 or more and 4 or less, more preferably 2 or 4, and still more preferably 2.

The counter ion represented by X is preferably an anion, and more preferably at least one anion selected from the group consisting of a fluoride ion, a chloride ion, a bromide ion, an iodide ion, a sulfide ion, an oxide ion, a hydroxide ion, a hydride ion, a sulfite ion, a phosphate ion, a cyanide ion, an acetate ion, a 2-ethylhexanoate ion, a carbonate ion, a sulfate ion, a nitrate ion, a bicarbonate ion, a trifluoroacetate ion, a thiocyanate ion, a trifluoromethanesulfonate ion, an acetylacetonate, a tetrafluoroborate ion, a hexafluorophosphate ion, and a tetraphenylborate ion. The neutral molecule represented by X is preferably at least one neutral molecule selected from the group consisting of water, methanol, ethanol, n-propanol, isopropyl alcohol, 2-methoxyethanol, 1,1-dimethylethanol, ethylene glycol, N,N'-dimethylformamide, N,N'-dimethylacetamide, N-methyl-2-pyrrolidone, dimethylsulfoxide, acetone, chloroform, acetonitrile, benzonitrile, triethylamine, pyridine, pyrazine, diazabicyclo[2,2,2]octane, 4,4'-bipyridine, tetrahydrofuran, diethyl ether, dimethoxyethane, methyl ethyl ether, 1,4-dioxane, acetic acid, propionic acid, and 2-ethylhexanoic acid.

b is an integer of 0 or more, and for example, preferably 0 or more and 4 or less.

From the viewpoint of ethylene selectivity, $P^1$ in Formula (1) is preferably a divalent group represented by the following Formula ($P^2$) or the following formula ($P^b$).

[Chem. 12]

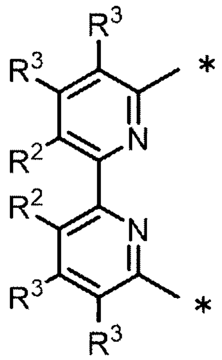

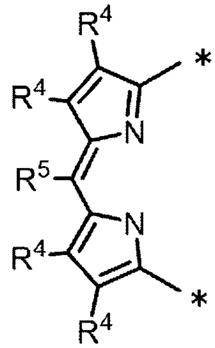

($P^a$) ($P^b$)

In Formula ($P^a$), $R^2$ and $R^3$ each independently represent a hydrogen atom or a substituent, and two adjacent $R^2$ and two adjacent $R^3$ may be bonded to each other to form a ring structure. In Formula ($P^b$), $R^4$ and $R^5$ represent a hydrogen atom or a substituent, and two adjacent $R^4$ and adjacent $R^4$ and $R^5$ may be bonded to each other to form a ring structure. A plurality of $R^4$ may be the same as or different from each other. * represents a bond.

In Formula ($P^a$), $R^2$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^2$ are preferably bonded to each other to form a ring structure, and the ring structure formed by bonding two adjacent $R^2$ to each other is preferably a benzene ring.

In Formula ($P^a$), $R^3$ is preferably a hydrogen atom.

In Formula ($P^b$), $R^4$ is preferably a hydrogen atom.

In Formula ($P^b$), $R^5$ is preferably an aromatic hydrocarbon ring group, preferably an unsubstituted or substituted aromatic hydrocarbon group having 30 or fewer carbon atoms, more preferably an unsubstituted or substituted phenyl group, an unsubstituted or substituted naphthyl group, an unsubstituted or substituted anthryl group, or an unsubstituted or substituted pyrenyl group, and still more preferably an unsubstituted or substituted phenyl group. Specific examples of the substituent include a halogen atom such as a fluorine atom, a chlorine atom, a bromine atom, or an iodine atom, a hydroxy group, a carboxyl group, an ester group, a mercapto group, a sulfonic acid group, a nitro group, a phosphonic acid group, a silyl group having an alkyl group having 1 to 4 carbon atoms, a linear, branched, or cyclic monovalent saturated hydrocarbon group having a total carbon number of about 1 to 50, such as a methyl group, an ethyl group, a propyl group, an isopropyl group, a cyclopropyl group, a butyl group, an isobutyl group, a tert-butyl group, a pentyl group, a cyclopentyl group, a hexyl group, a cyclohexyl group, a norbonyl group, a nonyl group, a cyclononyl group, a decyl group, a 3,7-dimethyloctyl group, an adamantyl group, a dodecyl group, a cyclododecyl group, a pentadecyl group, an octadecyl group, or a docosyl group, an alkenyl group, an alkynyl group, and a linear, branched, or cyclic alkoxy group having a total carbon number of about 1 to 50, such as a methoxy group, an ethoxy group, a propoxy group, a butoxy group, a pentyloxy group, a cyclohexyloxy group, a norbonyloxy group, a decyloxy group, or a dodecyloxy group. The number of substituents may be any substitutable number, and is 1 to 5 for a phenyl group, 1 to 7 for a naphthyl group, and 1 to 9 for each of an anthryl group and a pyrenyl group.

From the viewpoint of ethylene selectivity, the carbon dioxide reduction catalyst is preferably a compound represented by the following Formula (2).

[Chem. 13]

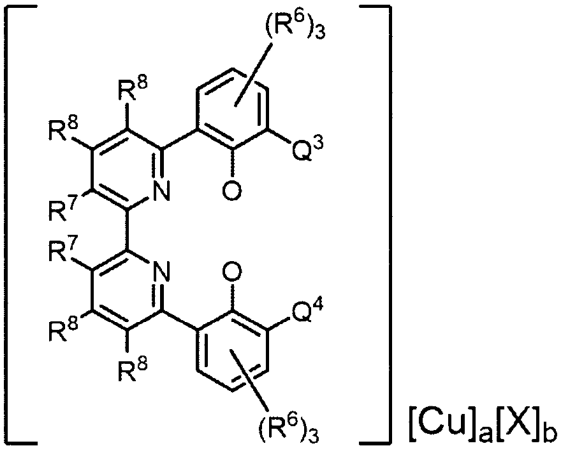

(2)

In Formula (2), $R^6$ to $R^8$ each independently represent a hydrogen atom or a substituent, and two adjacent $R^6$, two adjacent $R^7$, and two adjacent $R^8$ may be bonded to each other to form a ring structure. A plurality of $R^6$ to $R^8$ may be the same as or different from each other. $Q^3$ and $Q^4$ represent a monovalent group containing one or more aromatic rings, and $Q^3$ and $Q^4$ may be bonded to each other to form a ring structure. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

In Formula (2), $R^6$ is preferably a hydrogen atom, an alkyl group, or an alkoxy group. When $R^6$ is an alkyl group, $R^6$ is more preferably an alkyl group having 1 or more and 10 or fewer carbon atoms, and more preferably a t-butyl group. When $R^6$ is an alkoxy group, $R^6$ is more preferably an alkoxy group having 1 or more and 10 or fewer carbon atoms, and further more preferably a methoxy group.

In Formula (2), $R^7$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^7$ are preferably bonded to each other to form a ring structure, and the ring structure formed by bonding two adjacent $R^7$ to each other is preferably a benzene ring.

In Formula (2), $R^8$ is preferably a hydrogen atom.

In Formula (2), examples of the aromatic ring contained in the monovalent group represented by $Q^3$ and $Q^4$ include an aromatic hydrocarbon ring group and an aromatic heterocyclic group. The aromatic heterocyclic group as the aromatic ring contained in the monovalent group represented by $Q^3$ and $Q^4$ is preferably an aromatic heterocyclic group containing a nitrogen atom or an aromatic heterocyclic group containing a sulfur atom. When $Q^3$ and $Q^4$ are bonded to each other to form a ring structure, the ring structure is preferably, for example, a remaining atomic group obtained by removing two hydrogen atoms from phenanthroline.

Preferred aspects of a, X, and b in Formula (2) are the same as the preferred aspects of a, X, and b in Formula (1).

From the viewpoint of ethylene selectivity, the carbon dioxide reduction catalyst is preferably a compound represented by the following Formula (3).

[Chem. 14]

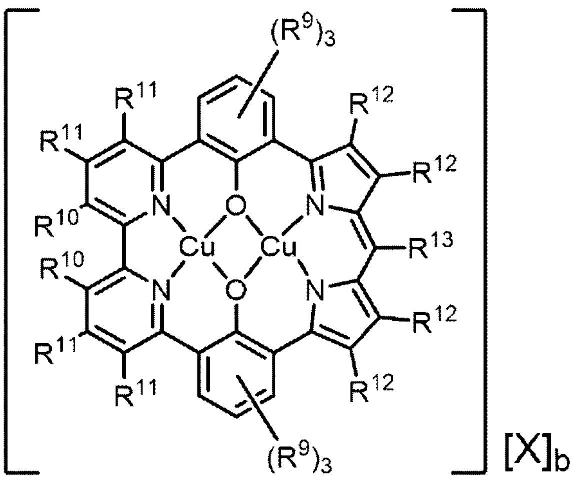

(3)

In Formula (3), $R^9$ to $R^{13}$ each independently represent a hydrogen atom, a substituent, or a divalent group, and two adjacent $R^9$, two adjacent $R^{10}$, two adjacent $R^{11}$, two adjacent $R^{12}$, and adjacent $R^{12}$ and $R^{13}$ may be bonded to each other to form a ring structure. A plurality of $R^9$ to $R^{12}$ may be the same as or different from each other. When $R^{13}$ is a divalent group, the divalent group may form a bond with another compound represented by Formula (3) to form a dimer. X is a counter ion or a neutral molecule, b is an integer of 0 or more, and when a plurality of X are present, the plurality of X may be the same as or different from each other.

In Formula (3), $R^9$ is preferably a hydrogen atom, an alkyl group, or an alkoxy group. When $R^9$ is an alkyl group, $R^9$ is more preferably an alkyl group having 1 or more and 10 or fewer carbon atoms, and more preferably a t-butyl group. When $R^9$ is an alkoxy group, $R^9$ is more preferably an alkoxy group having 1 or more and 10 or fewer carbon atoms, and further more preferably a methoxy group.

In Formula (3), $R^{10}$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^{10}$ are preferably bonded to each other to form a ring structure, and the ring structure formed by bonding two adjacent $R^{10}$ to each other is preferably a benzene ring.

In Formula (3), $R^{11}$ is preferably a hydrogen atom.

In Formula (3), $R^{12}$ is preferably a hydrogen atom.

In Formula (3), $R^{13}$ is preferably an aromatic hydrocarbon ring group, preferably an unsubstituted or substituted aromatic hydrocarbon group having 30 or fewer carbon atoms, more preferably an unsubstituted or substituted phenyl group, an unsubstituted or substituted naphthyl group, an unsubstituted or substituted anthryl group, or an unsubstituted or substituted pyrenyl group, and still more preferably an unsubstituted or substituted phenyl group. Specific examples of the substituent include a halogen atom such as a fluorine atom, a chlorine atom, a bromine atom, or an iodine atom, a hydroxy group, a carboxyl group, an ester group, a mercapto group, a sulfonic acid group, a nitro group, a phosphonic acid group, a silyl group having an alkyl group having 1 to 4 carbon atoms, a linear, branched, or cyclic monovalent saturated hydrocarbon group having a total carbon number of about 1 to 50, such as a methyl group, an ethyl group, a propyl group, an isopropyl group, a cyclopropyl group, a butyl group, an isobutyl group, a tert-butyl group, a pentyl group, a cyclopentyl group, a hexyl group, a cyclohexyl group, a norbonyl group, a nonyl group, a cyclononyl group, a decyl group, a 3,7-dimethyloctyl group, an adamantyl group, a dodecyl group, a cyclododecyl group, a pentadecyl group, an octadecyl group, or a docosyl group, an alkenyl group, an alkynyl group, and a linear, branched, or cyclic alkoxy group having a total carbon number of about 1 to 50, such as a methoxy group, an ethoxy group, a propoxy group, a butoxy group, a pentyloxy group, a cyclohexyloxy group, a norbonyloxy group, a decyloxy group, or a dodecyloxy group. The number of substituents may be any substitutable number, and is 1 to 5 for a phenyl group, 1 to 7 for a naphthyl group, and 1 to 9 for each of an anthryl group and a pyrenyl group.

Preferred aspects of X and b in Formula (3) are the same as the preferred aspects of X and b in Formula (1).

From the viewpoint of ethylene selectivity, the carbon dioxide reduction catalyst is preferably a compound represented by the following Formula (4).

[Chem. 15]

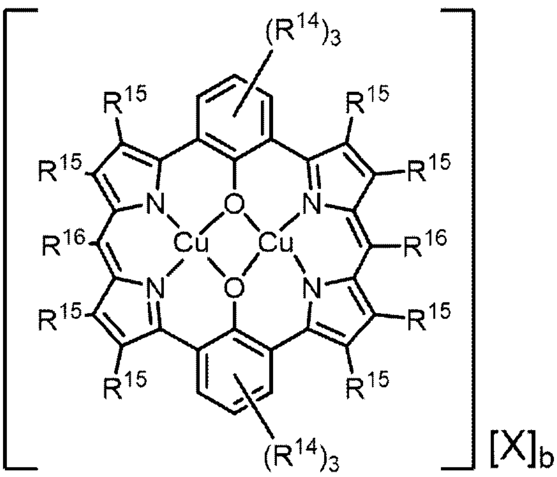

(4)

In Formula (4), $R^{14}$ to $R^{16}$ each independently represent a hydrogen atom or a substituent, and two adjacent $R^{14}$, two adjacent $R^{15}$, and adjacent $R^{15}$ and $R^{16}$ may be bonded to each other to form a ring. A plurality of $R^{14}$ to $R^{16}$ may be the same as or different from each other. X is a counter ion or a neutral molecule, b is an integer of 0 or more, and when a plurality of X are present, the plurality of X may be the same as or different from each other.

In Formula (4), $R^{14}$ is preferably a hydrogen atom, an alkyl group, or an alkoxy group. When $R^{14}$ is an alkyl group, $R^{14}$ is more preferably an alkyl group having 1 or more and 10 or fewer carbon atoms, and more preferably a t-butyl group. When $R^{14}$ is an alkoxy group, $R^{14}$ is more preferably an alkoxy group having 1 or more and 10 or fewer carbon atoms, and further more preferably a methoxy group.

In Formula (4), $R^{15}$ is preferably a hydrogen atom.

In Formula (4), $R^{16}$ is preferably an aromatic hydrocarbon ring group, preferably an unsubstituted or substituted aromatic hydrocarbon group having 30 or fewer carbon atoms, more preferably an unsubstituted or substituted phenyl group, an unsubstituted or substituted naphthyl group, an unsubstituted or substituted anthryl group, or an unsubstituted or substituted pyrenyl group, and still more preferably an unsubstituted or substituted phenyl group. Specific examples of the substituent include a halogen atom such as a fluorine atom, a chlorine atom, a bromine atom, or an iodine atom, a hydroxy group, a carboxyl group, an ester group, a mercapto group, a sulfonic acid group, a nitro group, a phosphonic acid group, a silyl group having an alkyl group having 1 to 4 carbon atoms, a linear, branched, or cyclic monovalent saturated hydrocarbon group having a total carbon number of about 1 to 50, such as a methyl group, an ethyl group, a propyl group, an isopropyl group, a cyclopropyl group, a butyl group, an isobutyl group, a tert-butyl group, a pentyl group, a cyclopentyl group, a hexyl group, a cyclohexyl group, a norbonyl group, a nonyl group, a cyclononyl group, a decyl group, a 3,7-dimethyloctyl group, an adamantyl group, a dodecyl group, a cyclododecyl group, a pentadecyl group, an octadecyl group, or a docosyl group, an alkenyl group, an alkynyl group, and a linear, branched, or cyclic alkoxy group having a total carbon number of about 1 to 50, such as a methoxy group, an ethoxy group, a propoxy group, a butoxy group, a pentyloxy group, a cyclohexyloxy group, a norbonyloxy group, a decyloxy group, or a dodecyloxy group. The number of substituents may be any substitutable number, and is 1 to 5 for a phenyl group, 1 to 7 for a naphthyl group, and 1 to 9 for each of an anthryl group and a pyrenyl group.

Preferred aspects of X and b in Formula (4) are the same as the preferred aspects of X and b in Formula (1).

From the viewpoint of ethylene selectivity, the carbon dioxide reduction catalyst is preferably a compound represented by the following Formula (5).

[Chem. 16]

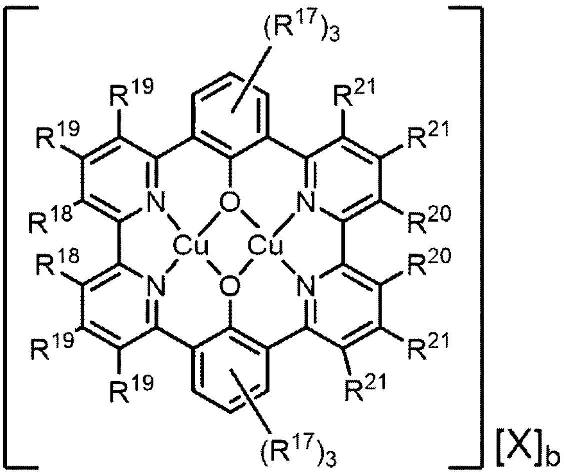

(5)

In Formula (5), $R^{17}$ to $R^{21}$ each independently represent a hydrogen atom or a substituent, and two adjacent $R^{17}$, two adjacent $R^{18}$, two adjacent $R^{19}$, two adjacent $R^{20}$, and two adjacent $R^{21}$ may be bonded to each other to form a ring. A plurality of $R^{17}$ to $R^{21}$ may be the same as or different from each other. X is a counter ion or a neutral molecule, b is an integer of 0 or more, and when a plurality of X are present, the plurality of X may be the same as or different from each other.

In Formula (5), $R^{17}$ is preferably a hydrogen atom, an alkyl group, or an alkoxy group. When $R^{17}$ is an alkyl group, $R^{17}$ is more preferably an alkyl group having 1 or more and 10 or fewer carbon atoms, and more preferably a t-butyl group. When $R^{17}$ is an alkoxy group, $R^{17}$ is more preferably an alkoxy group having 1 or more and 10 or fewer carbon atoms, and further more preferably a methoxy group.

In Formula (5), $R^{18}$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^{18}$ are preferably bonded to each other to form a ring structure, and the ring structure formed by bonding two adjacent $R^{18}$ to each other is preferably a benzene ring.

In Formula (5), $R^{19}$ is preferably a hydrogen atom.

In Formula (5), $R^{20}$ is preferably a hydrocarbon group, more preferably an alkyl group or alkenyl group having 1 or more and 6 or fewer carbon atoms, and still more preferably an alkenyl group having 1 or more and 4 or fewer carbon

atoms. Two adjacent $R^{20}$ are preferably bonded to each other to form a ring structure, and the ring structure formed by bonding two adjacent $R^{20}$ to each other is preferably a benzene ring.

In Formula (5), $R^{21}$ is preferably a hydrogen atom.

Preferred aspects of X and b in Formula (5) are the same as the preferred aspects of X and b in Formula (1).

From the viewpoint of ethylene selectivity, the carbon dioxide reduction catalyst is preferably a compound represented by the following Formula (6).

[Chem. 17]

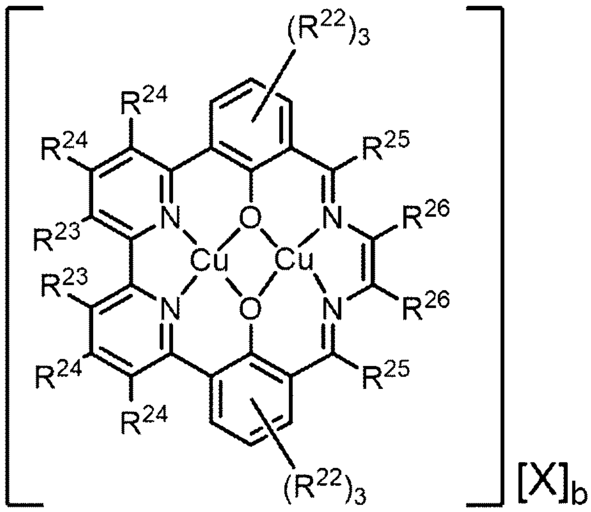

(6)

In Formula (6), $R^{22}$ to $R^{26}$ each independently represent a hydrogen atom or a substituent, and two adjacent $R^{22}$, two adjacent $R^{23}$, two adjacent $R^{24}$, two adjacent $R^{26}$, and adjacent $R^{25}$ and $R^{26}$ may be bonded to each other to form a ring structure. A plurality of $R^{22}$ to $R^{26}$ may be the same as or different from each other. X is a counter ion or a neutral molecule, b is an integer of 0 or more, and when a plurality of X are present, the plurality of X may be the same as or different from each other.

In Formula (6), $R^{22}$ is preferably a hydrogen atom, an alkyl group, or an alkoxy group. When $R^{22}$ is an alkyl group, $R^{22}$ is more preferably an alkyl group having 1 or more and 10 or fewer carbon atoms, and more preferably a t-butyl group. When $R^{22}$ is an alkoxy group, $R^{22}$ is more preferably an alkoxy group having 1 or more and 10 or fewer carbon atoms, and further more preferably a methoxy group.

In Formula (6), $R^{23}$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^{23}$ are preferably bonded to each other to form a ring structure, and the ring structure formed by bonding two adjacent $R^{23}$ to each other is preferably a benzene ring.

In Formula (6), $R^{24}$ and $R^{25}$ are preferably a hydrogen atom.

In Formula (6), $R^{26}$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^{26}$ are preferably bonded to each other to form a ring structure, and the ring structure formed by bonding two adjacent $R^{26}$ to each other is preferably a benzene ring.

Preferred aspects of X and b in Formula (6) are the same as the preferred aspects of X and b in Formula (1).

From the viewpoint of ethylene selectivity, the carbon dioxide reduction catalyst is preferably a compound represented by the following Formula (7).

[Chem. 18]

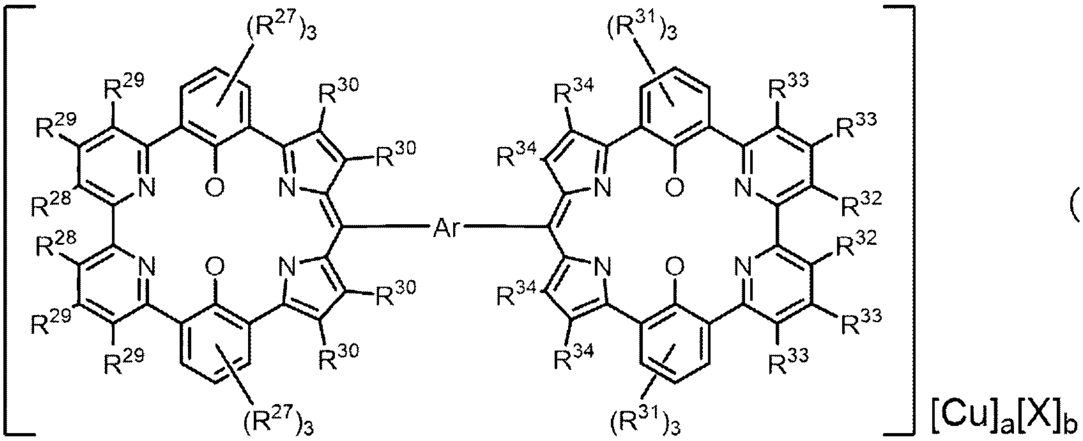

(7)

In Formula (7), $R^{27}$ to $R^{34}$ each independently represent a hydrogen atom or a substituent, and two adjacent $R^{27}$, two adjacent $R^{28}$, two adjacent $R^{29}$, two adjacent $R^{30}$, two adjacent $R^{31}$, two adjacent $R^{32}$, two adjacent $R^{33}$, and two adjacent $R^{34}$ may be bonded to each other to form a ring structure. A plurality of $R^{27}$ to $R^{34}$ may be the same as or different from each other. Ar represents a divalent aromatic group which may have a substituent. a is an integer of 2 or more and 4 or less. X is a counter ion or a neutral molecule, b is an integer of 0 or more, and when a plurality of X are present, the plurality of X may be the same as or different from each other. O is an oxygen atom and is bonded to at least one copper atom.

In Formula (7), $R^{27}$ and $R^{31}$ are preferably a hydrogen atom, an alkyl group, or an alkoxy group. When $R^{27}$ and $R^{31}$ are an alkyl group, $R^{27}$ and $R^{31}$ are more preferably an alkyl group having 1 or more and 10 or fewer carbon atoms, and more preferably a t-butyl group. When $R^{27}$ and $R^{31}$ are an alkoxy group, $R^{27}$ and $R^{31}$ are more preferably an alkoxy group having 1 or more and 10 or fewer carbon atoms, and further more preferably a methoxy group.

In Formula (7), $R^{28}$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^{28}$ are preferably bonded to each other to form a ring structure, and the ring structure formed by bonding two adjacent $R^{28}$ to each other is preferably a benzene ring.

In Formula (7), $R^{32}$ is preferably a hydrocarbon group, more preferably an alkyl group or alkenyl group having 1 or more and 6 or fewer carbon atoms, and still more preferably an alkenyl group having 1 or more and 4 or fewer carbon atoms. Two adjacent $R^{32}$ are preferably bonded to each other to form a ring structure, and the ring structure formed by bonding two adjacent $R^{32}$ to each other is preferably a benzene ring.

In Formula (7), $R^{29}$ and $R^{33}$ are preferably a hydrogen atom.

In Formula (7), $R^{30}$ and $R^{34}$ are preferably a hydrogen atom.

In Formula (7), a is preferably 4.

Preferred aspects of X and b in Formula (7) are the same as the preferred aspects of X and b in Formula (1).

Hereinafter, a specific structural formula of the carbon dioxide reduction catalyst is shown, but the carbon dioxide reduction catalyst is not limited thereto. Note that, in the following structural formula, "Me" represents a methyl group, "Et" represents an ethyl group, "t-Bu" represents a t-butyl group, "TMS" represents a trimethylsilyl group, "i-Pr" represents an isopropyl group, "$^-$OAc" represents an acetate anion, and the valence of copper is divalent ($Cu^{2+}$).

[Chem. 19]

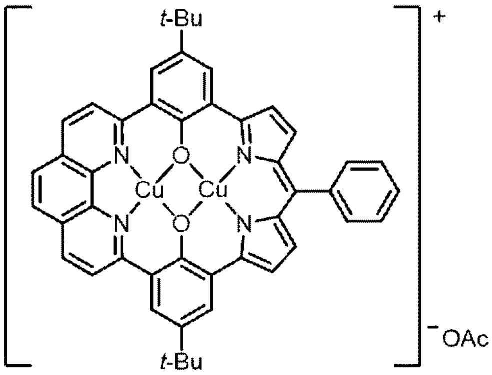

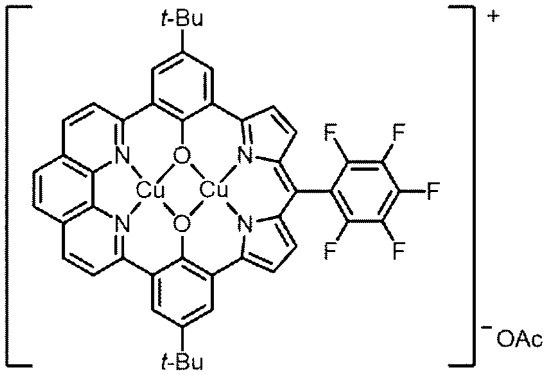

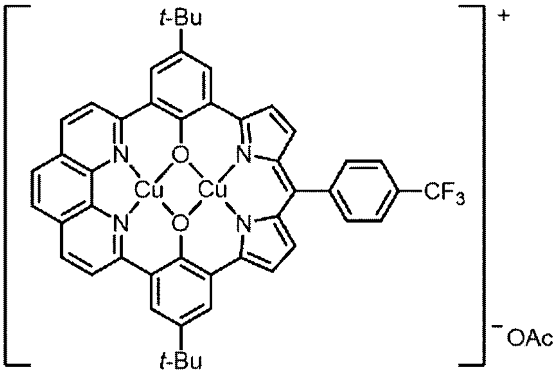

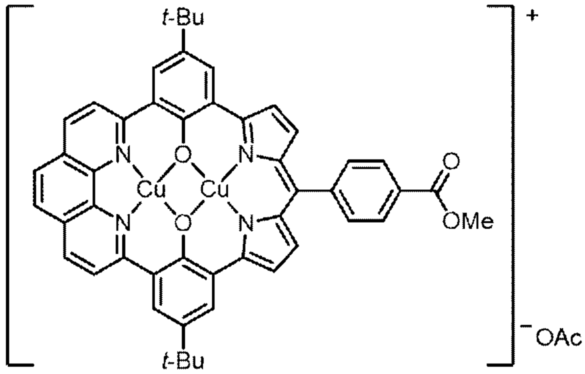

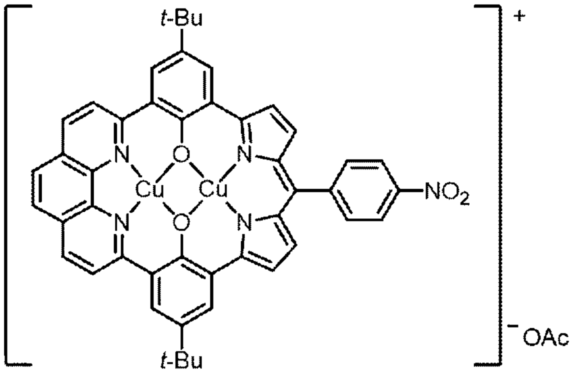

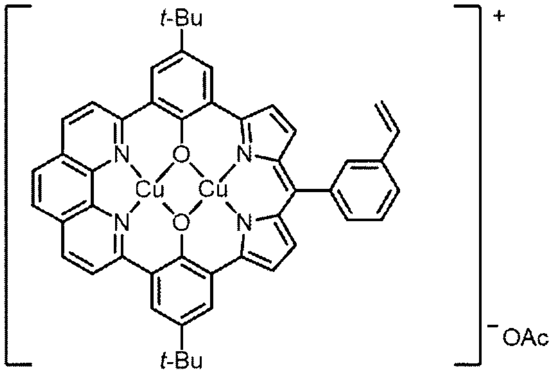

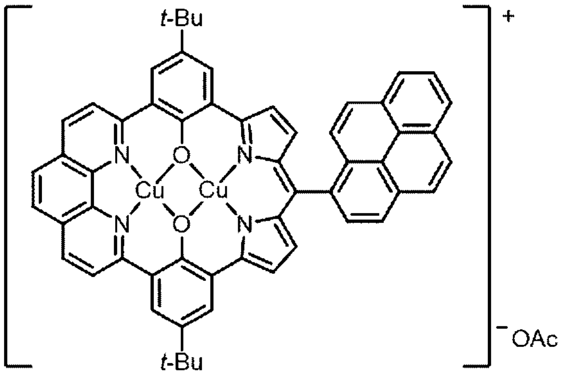

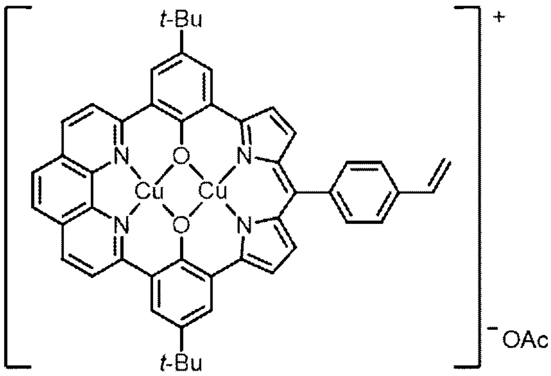

[Chem. 20]

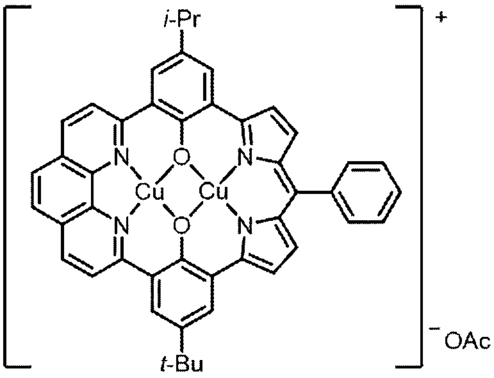

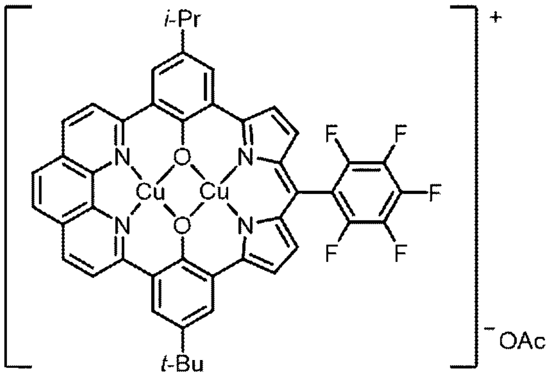

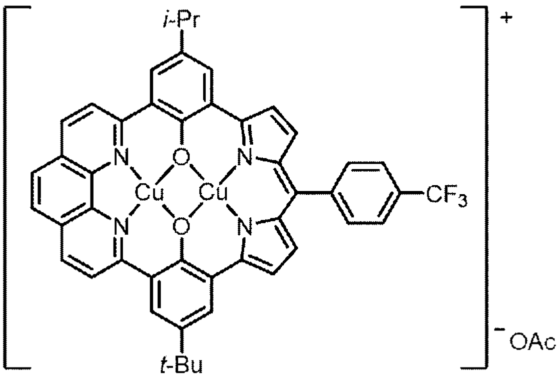

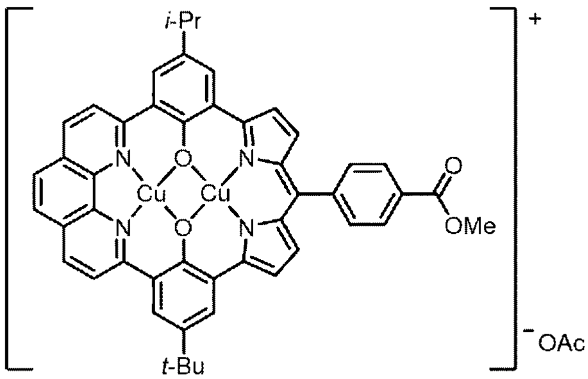

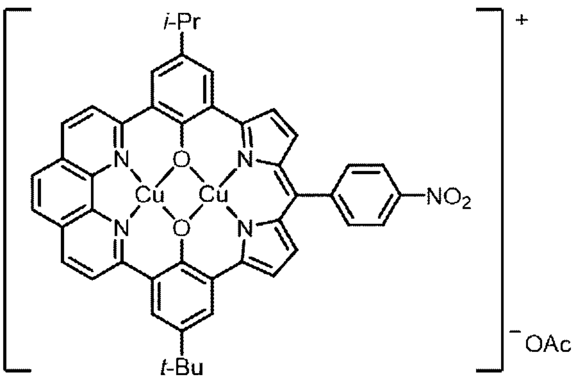

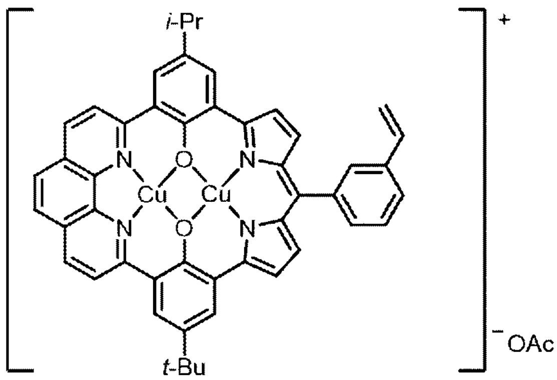

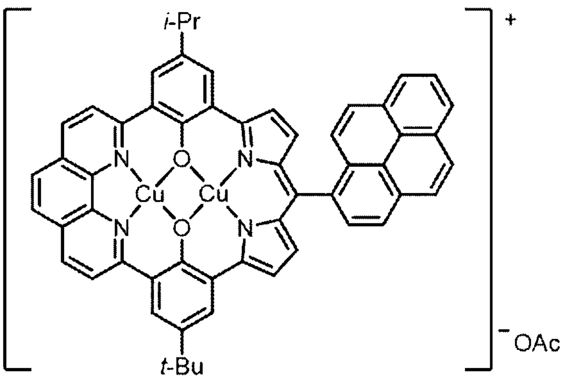

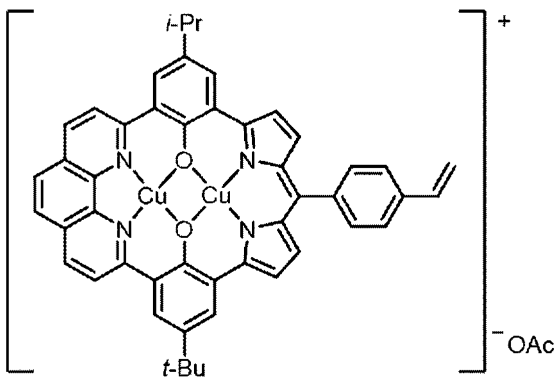

[Chem. 21]

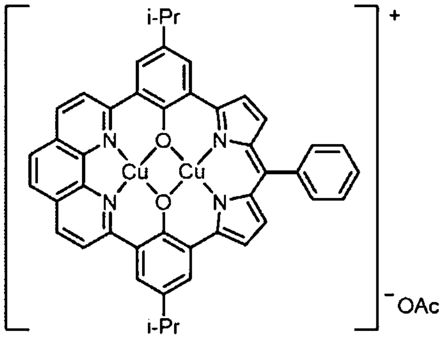

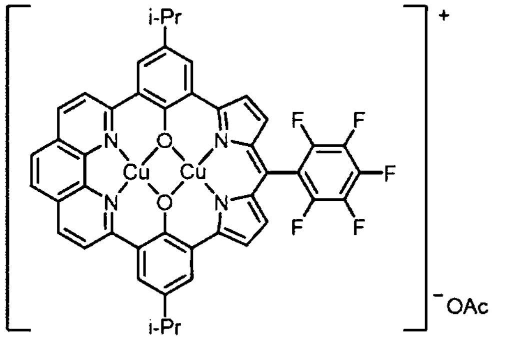

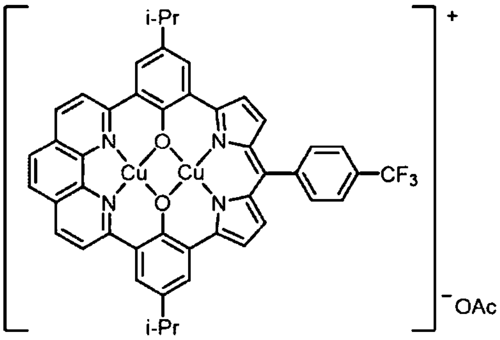

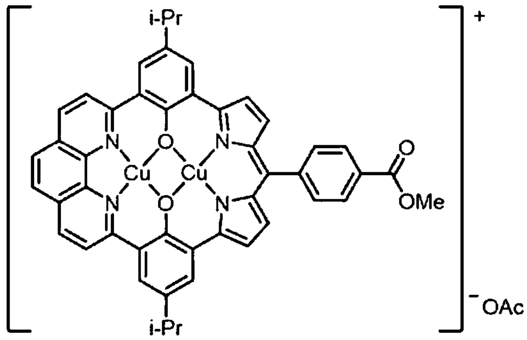

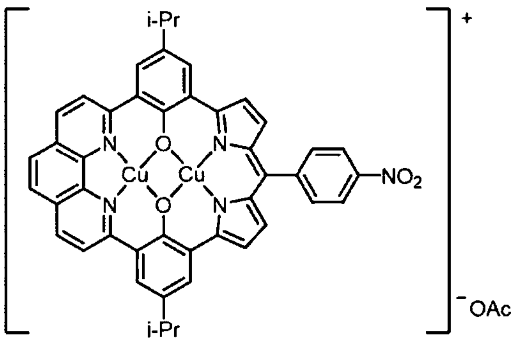

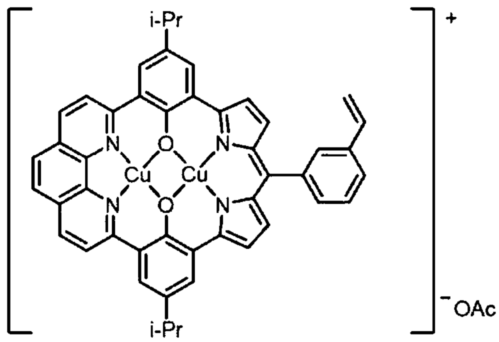

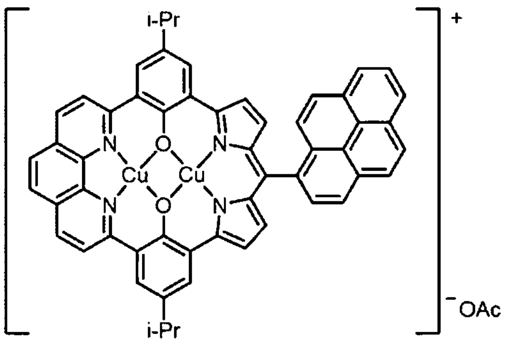

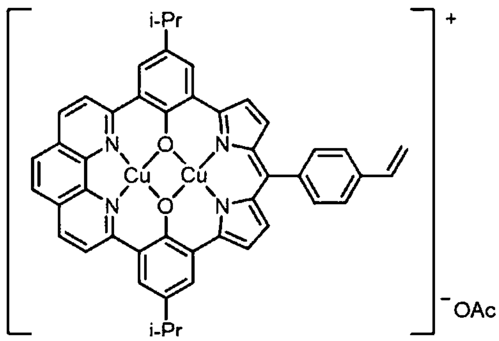

[Chem. 22]

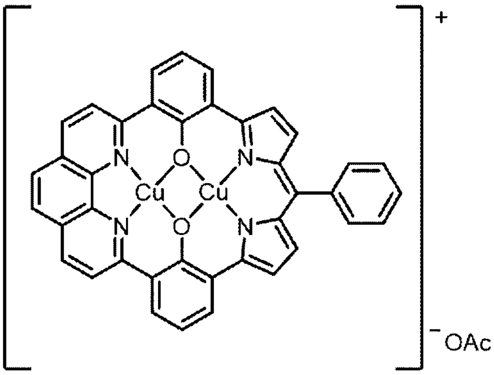

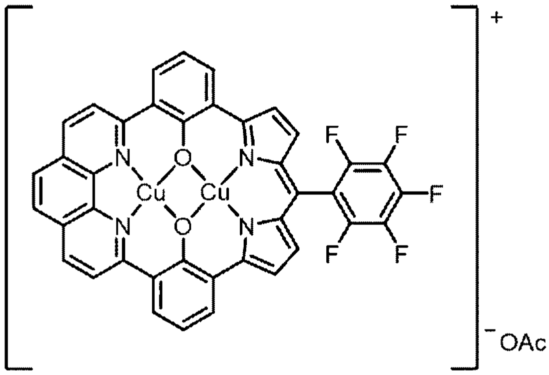

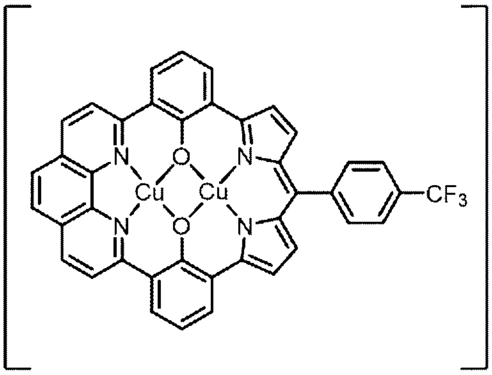

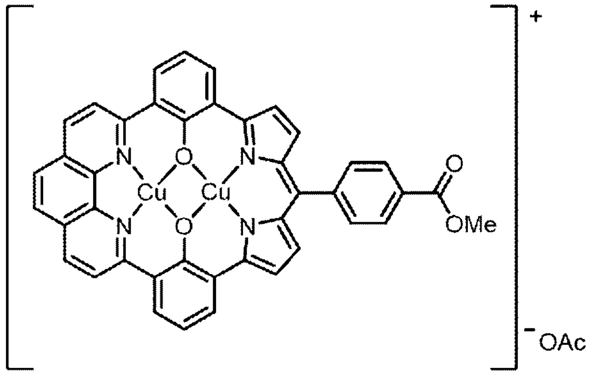

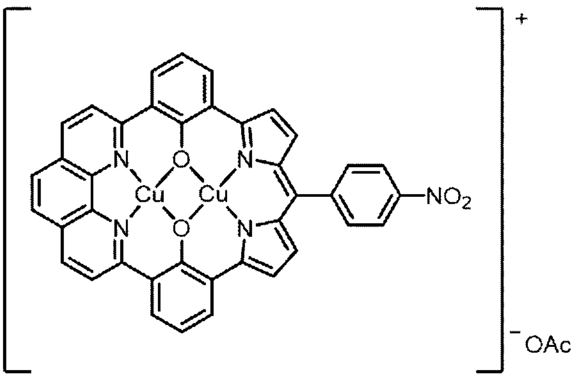

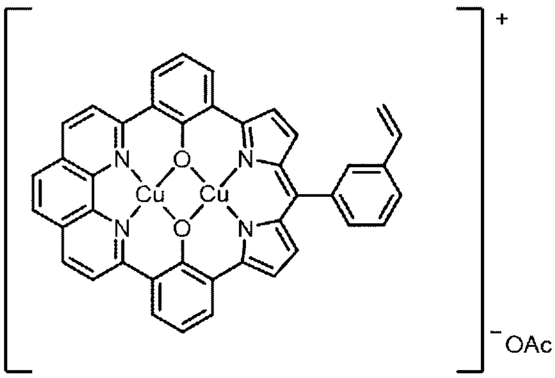

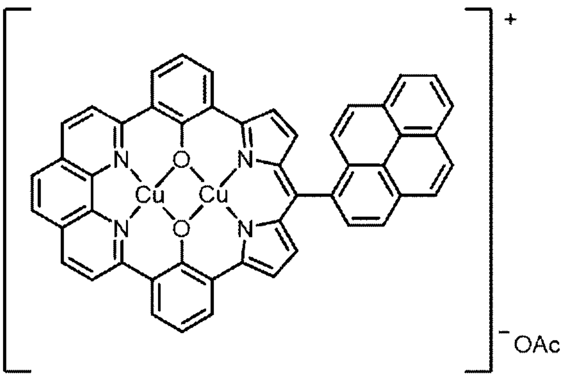

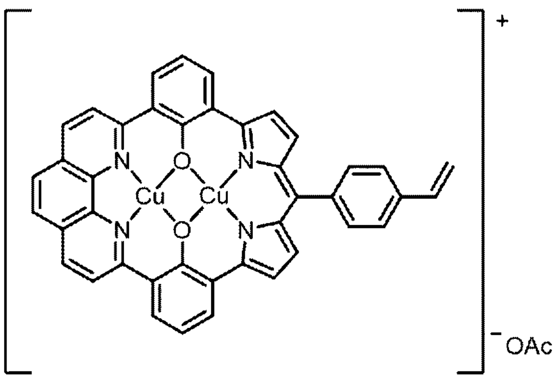

[Chem. 23]

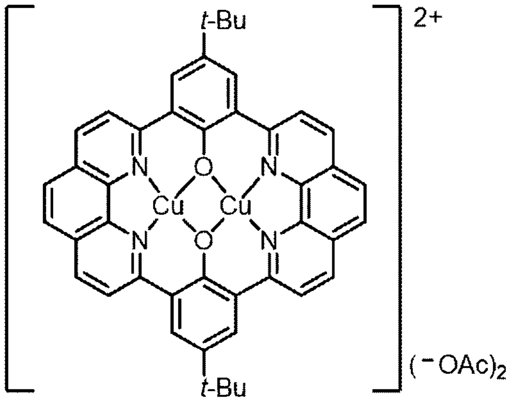

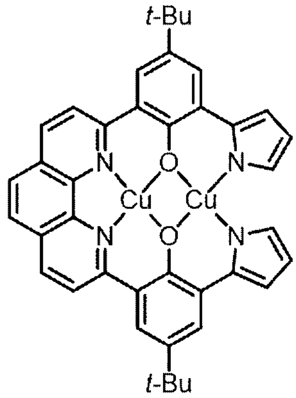

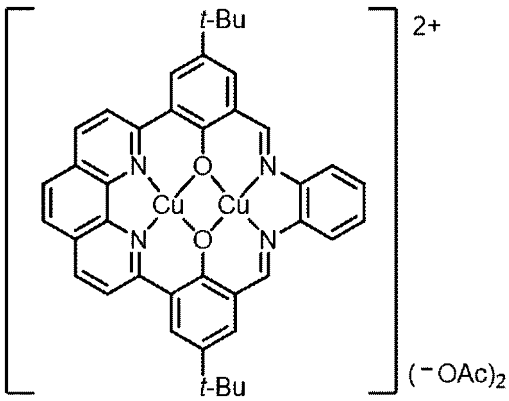

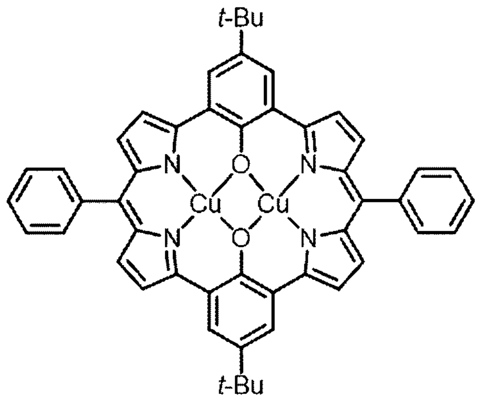

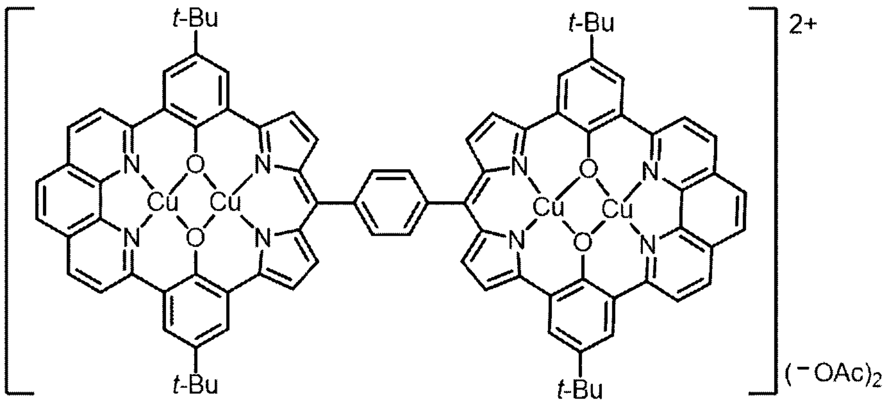

[Chem. 24]

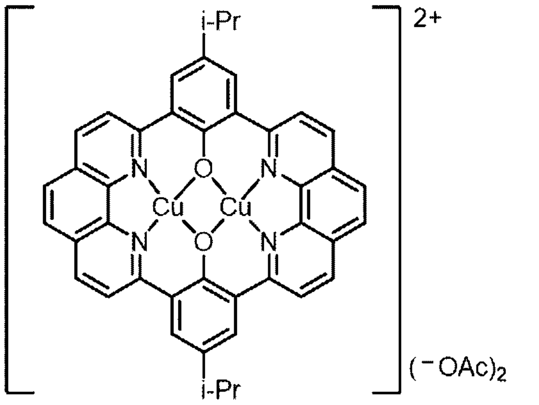

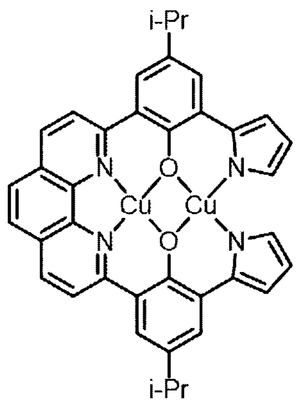

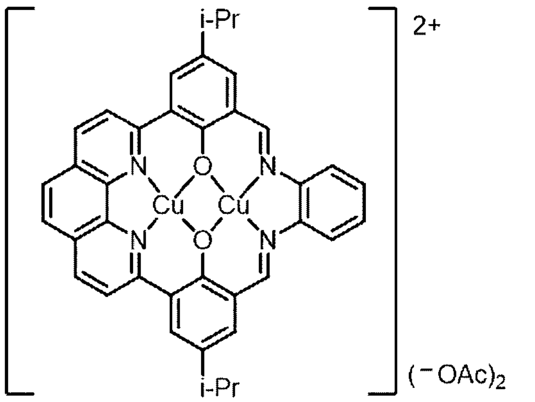

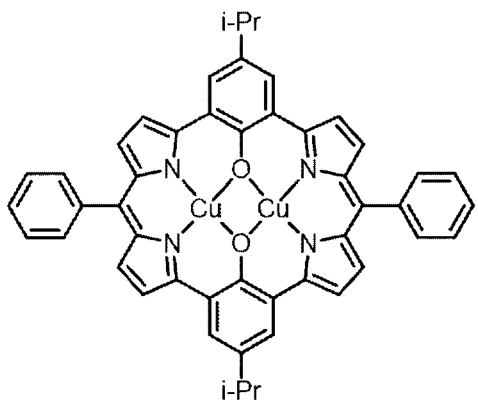

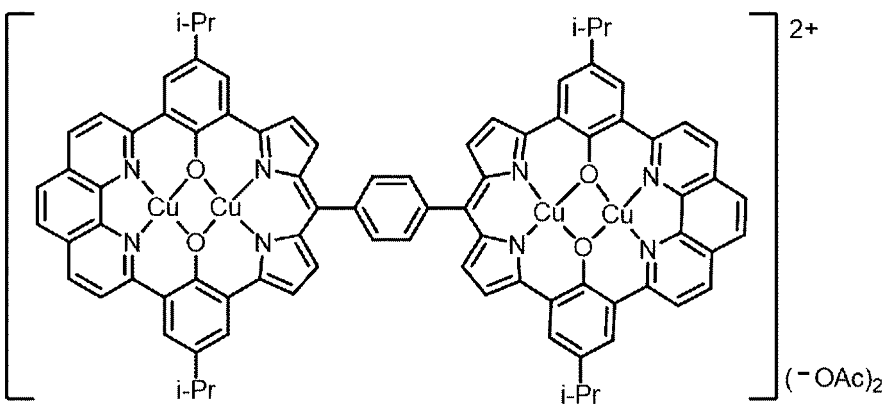

[Chem. 25]

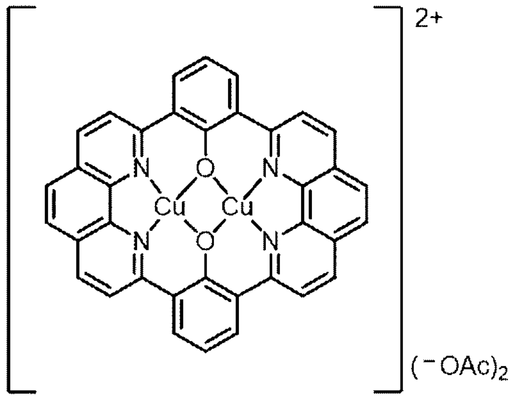

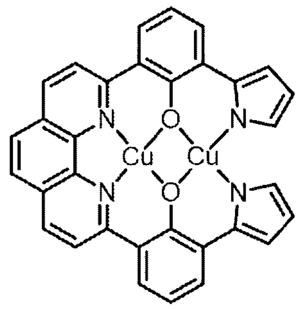

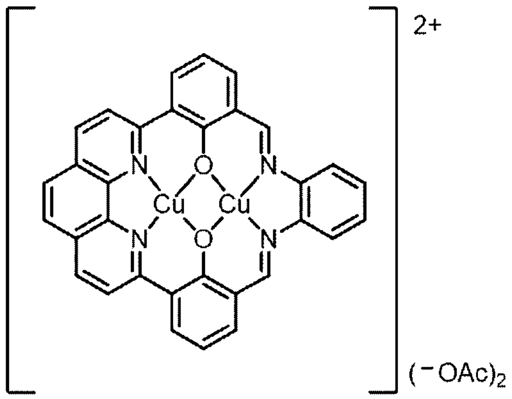

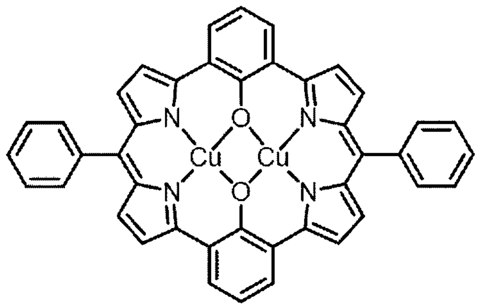

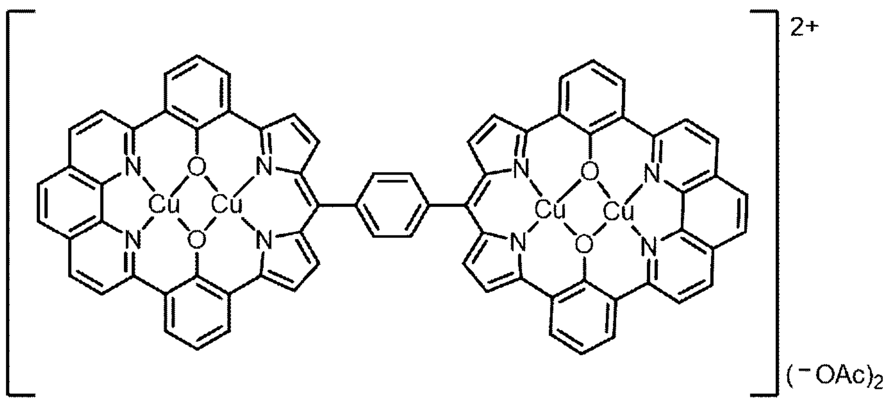

[Chem. 26]

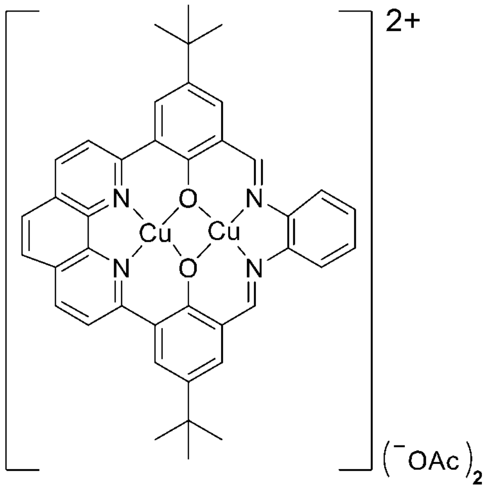

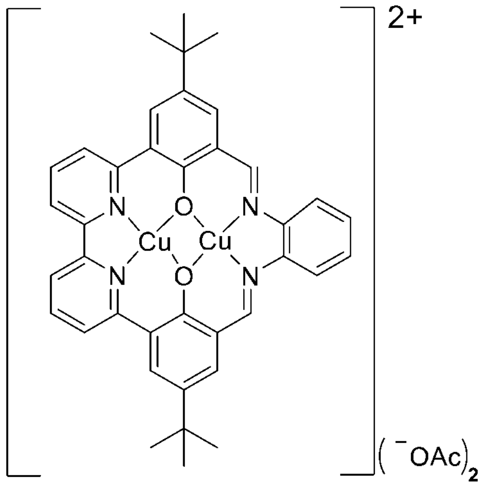

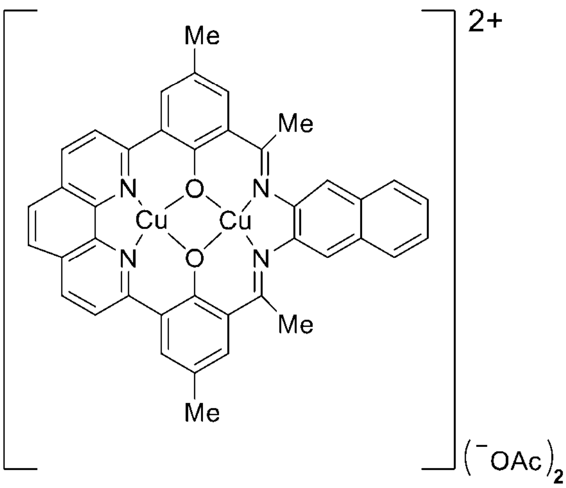

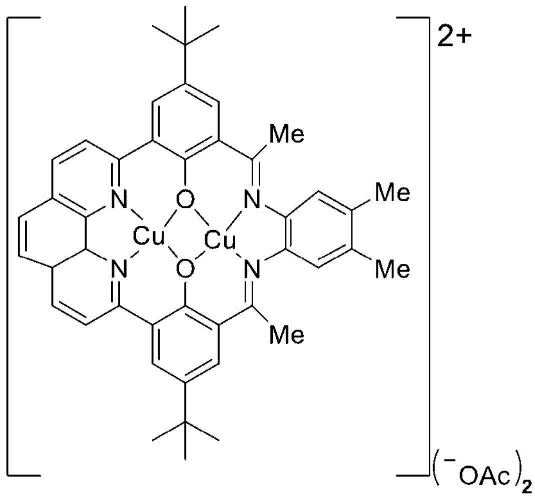

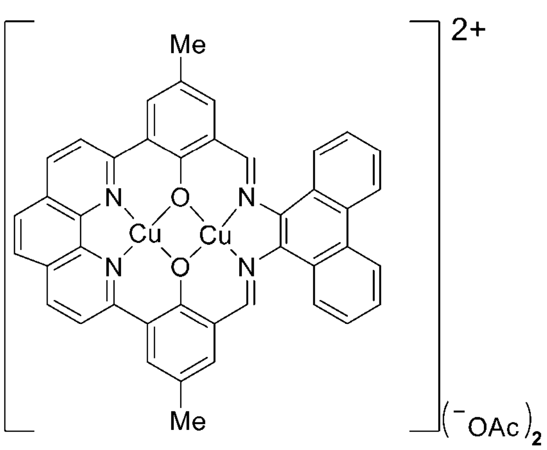

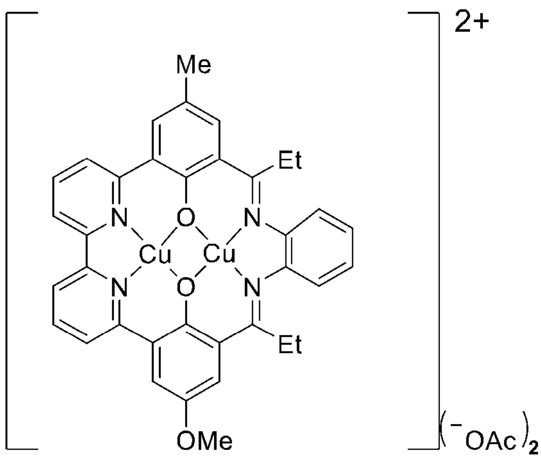

[Chem. 27]

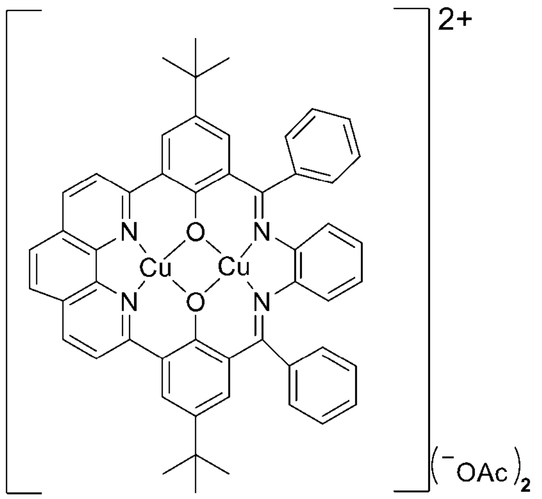

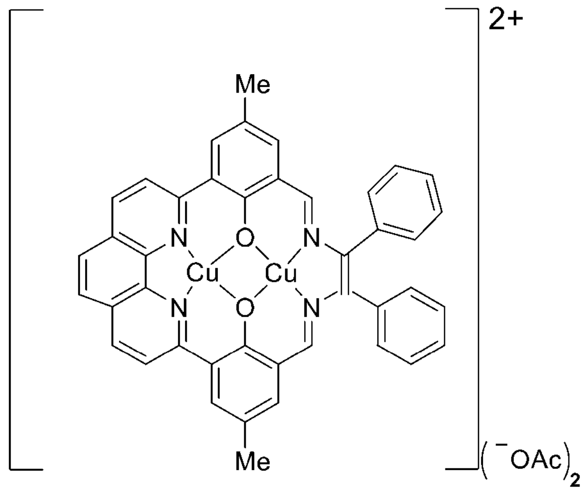

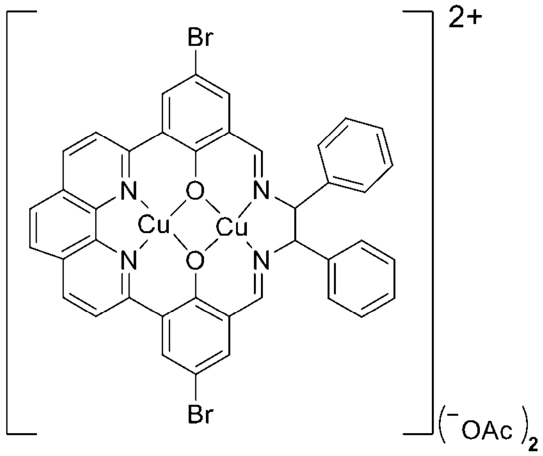

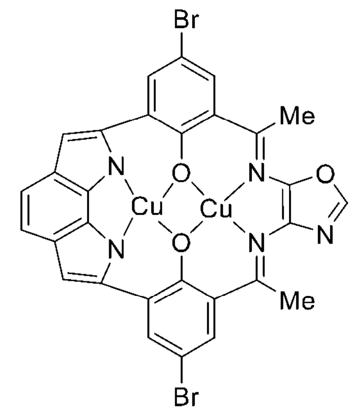

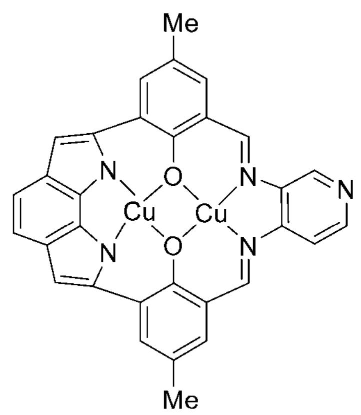

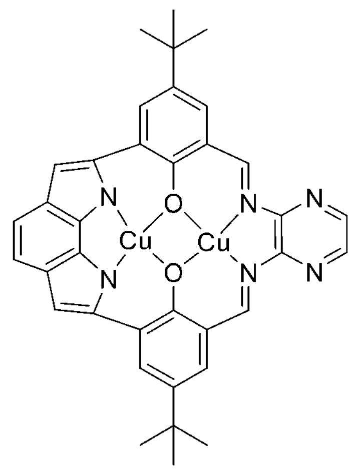

[Chem. 28]

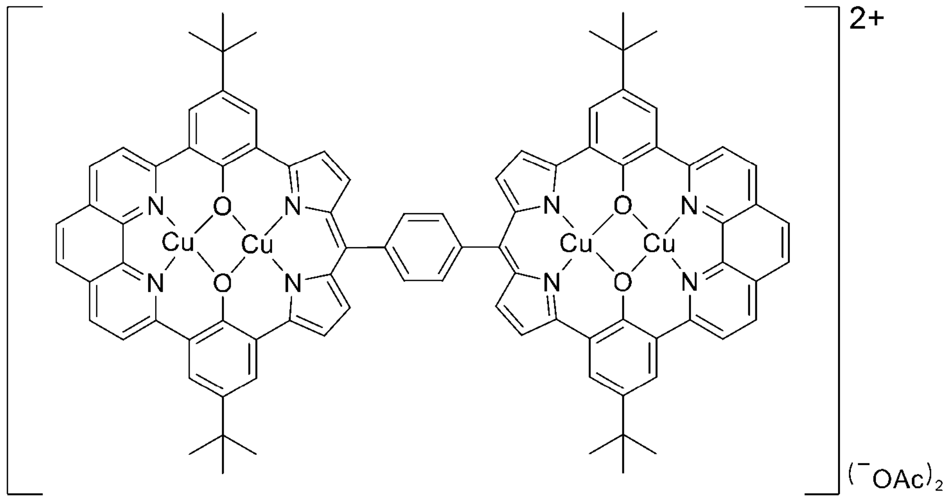

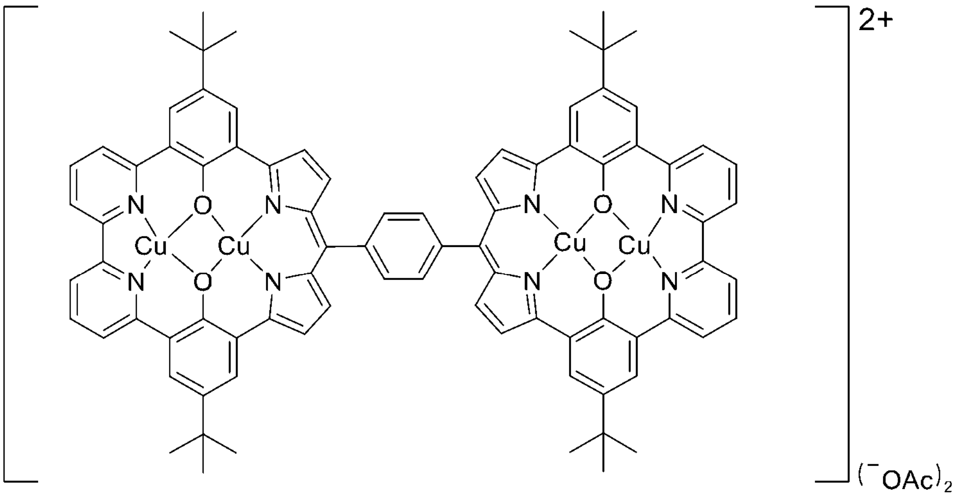

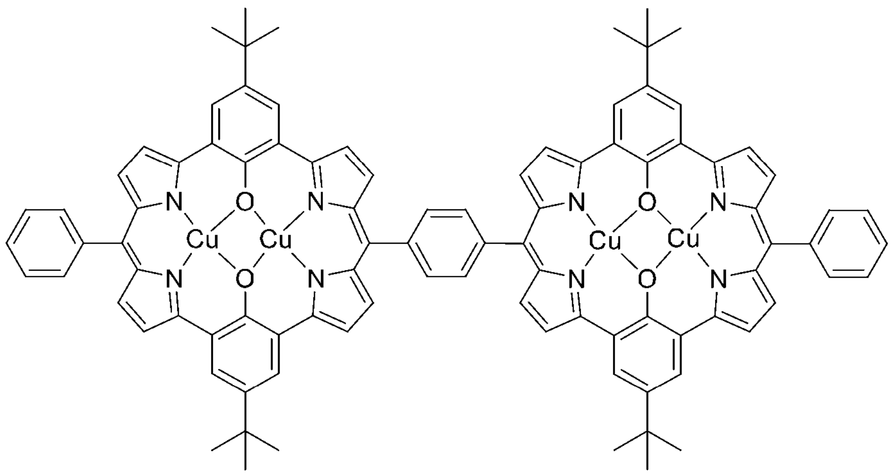

[Chem. 29]

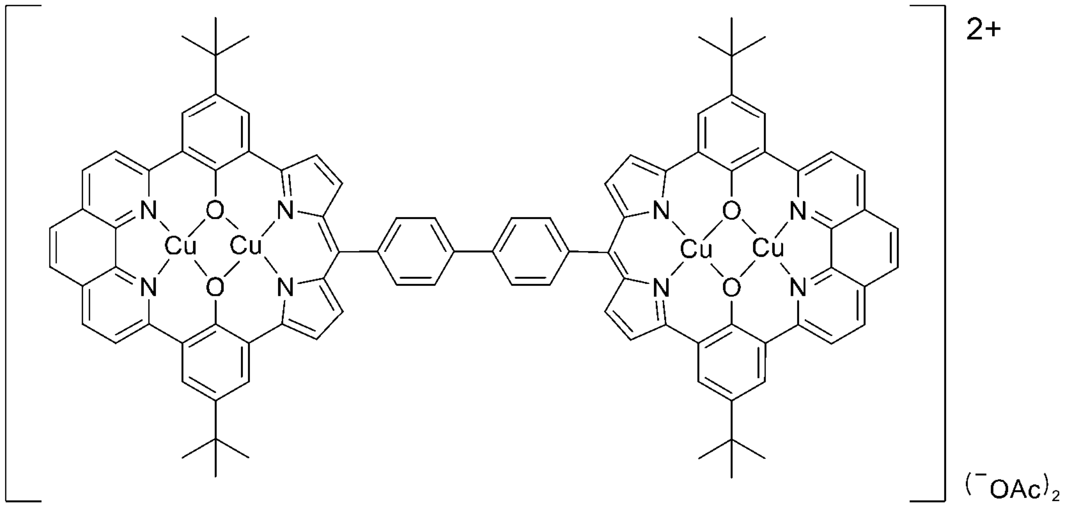

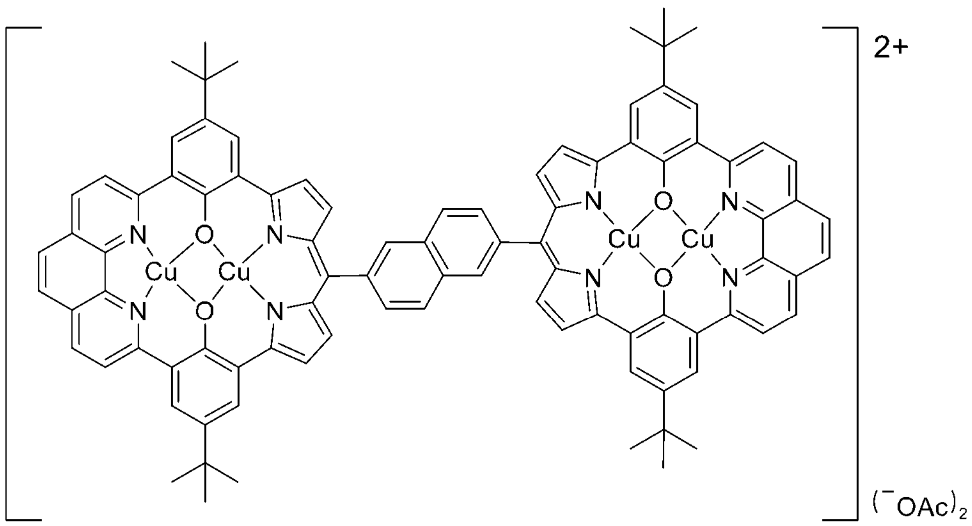

[Chem. 30]

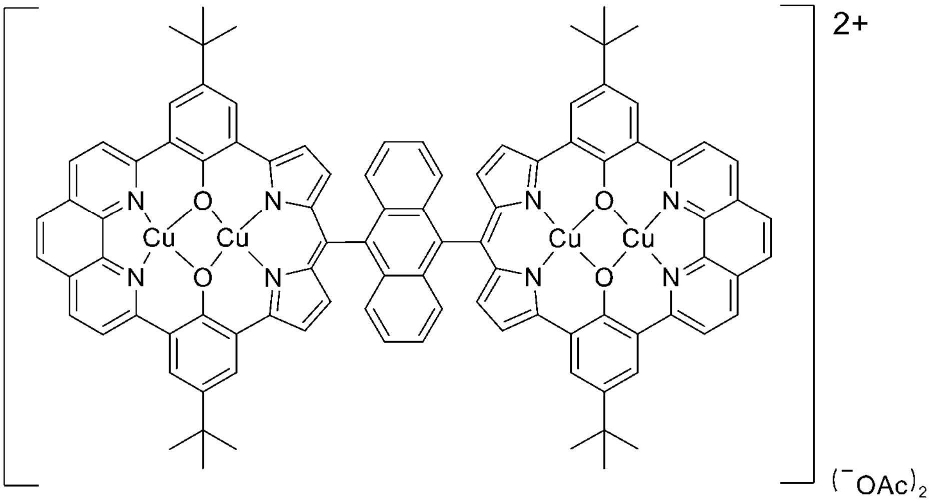

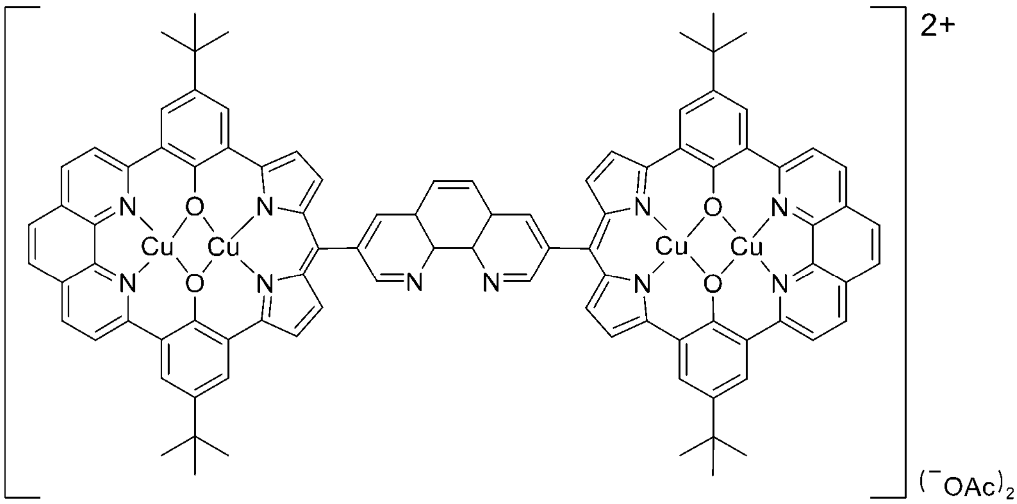

[Chem. 31]

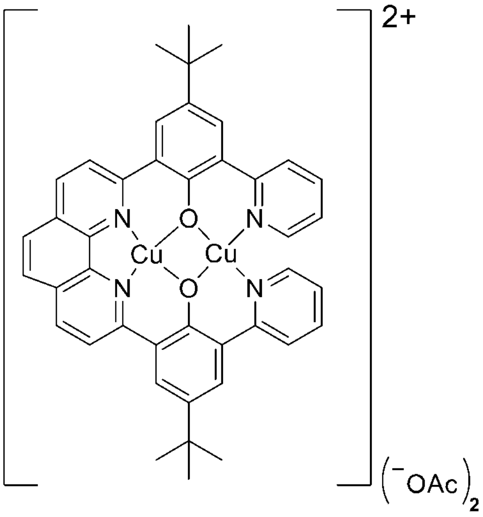

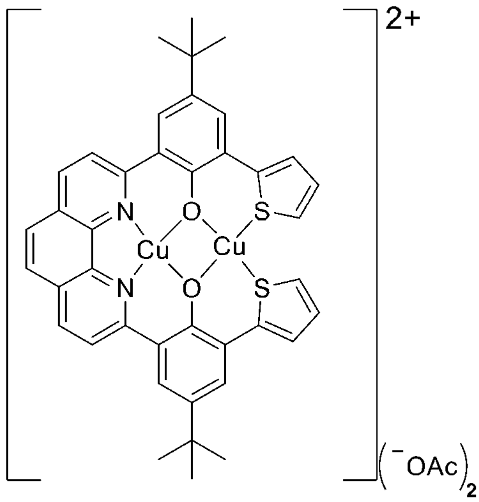

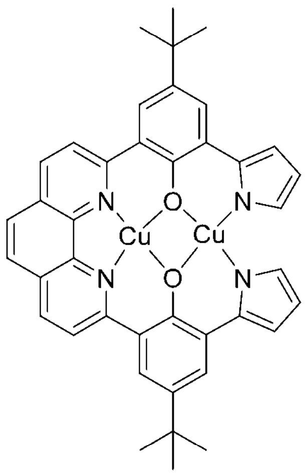

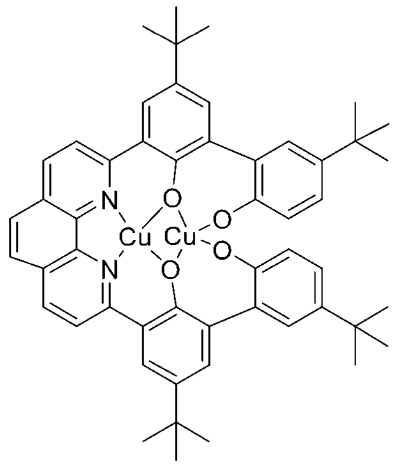

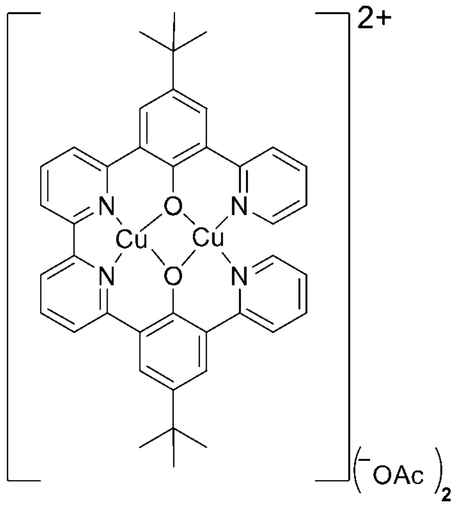

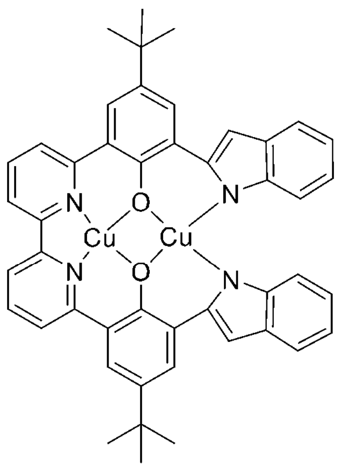

[Chem. 32]

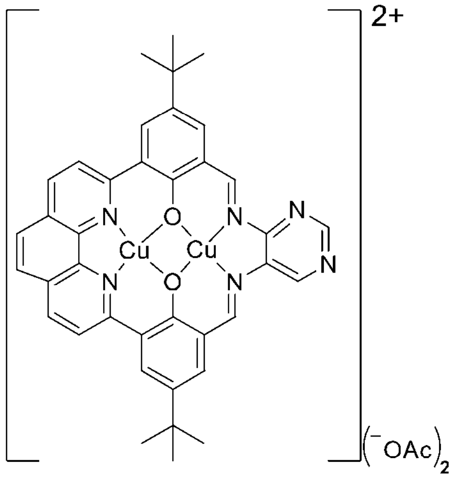

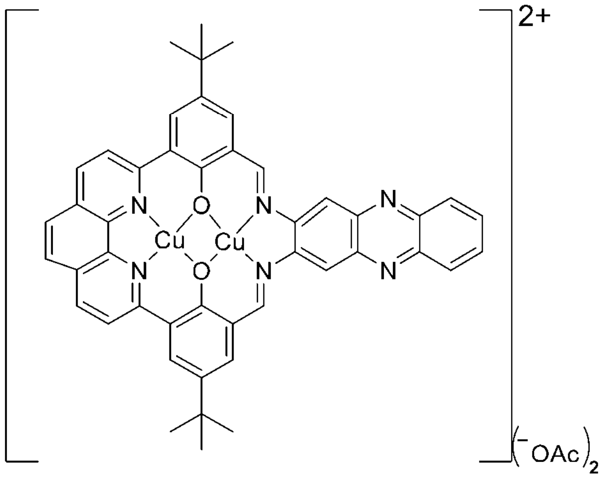

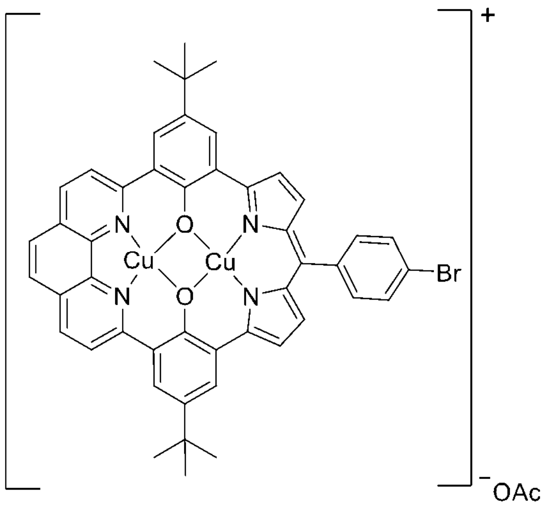

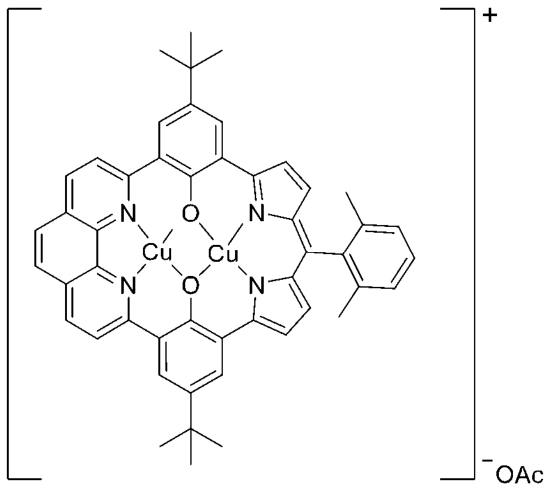

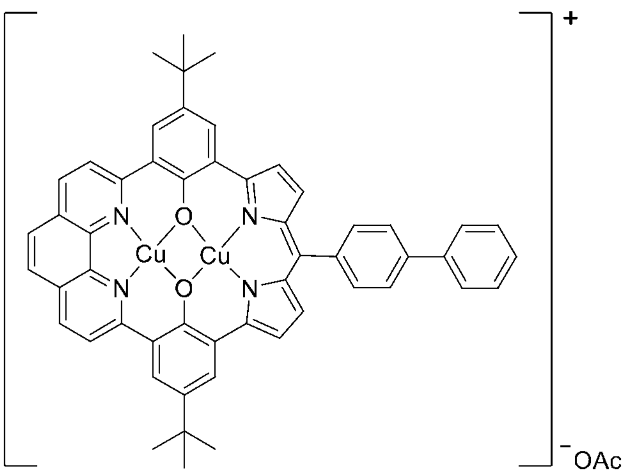

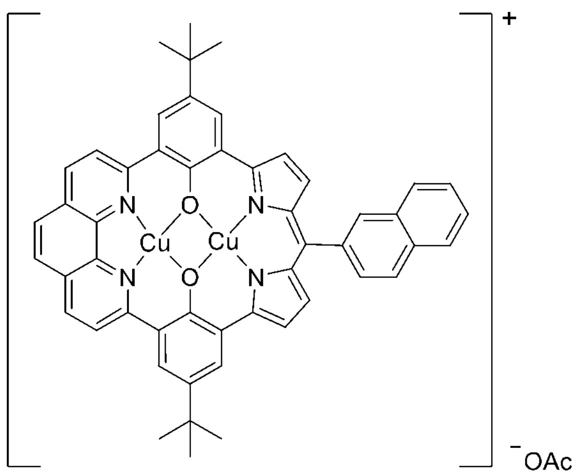

[Chem. 33]

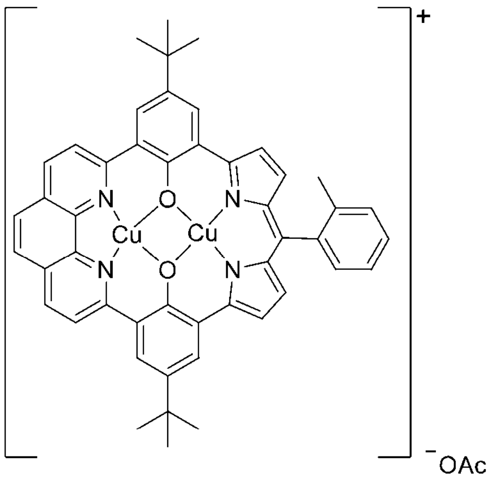

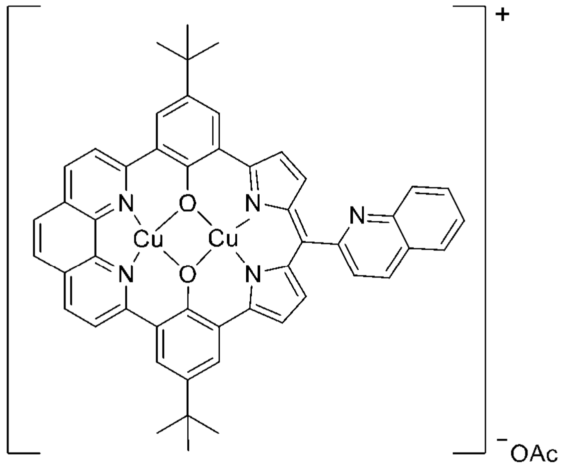

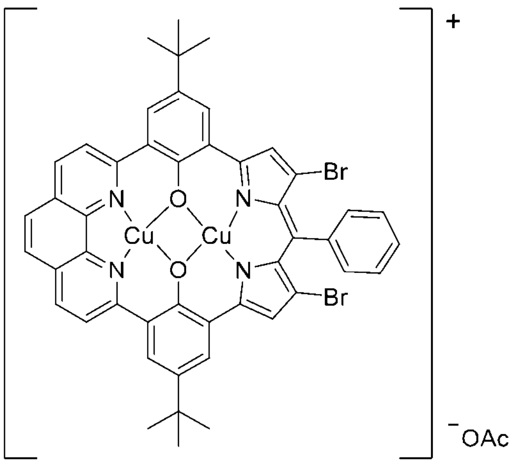

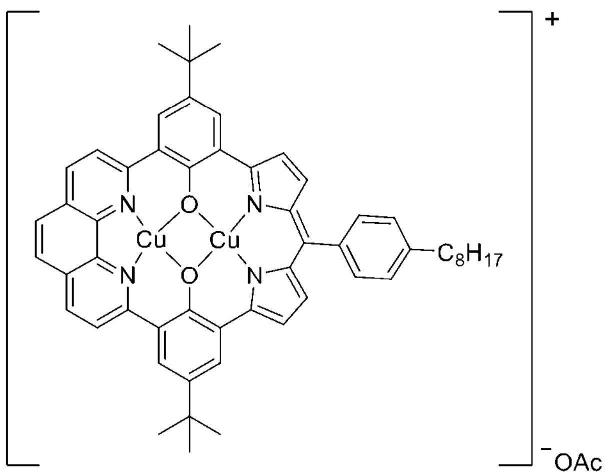

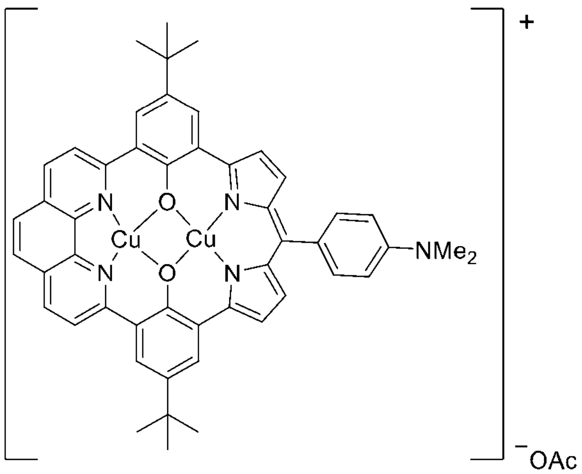

[Chem. 34]

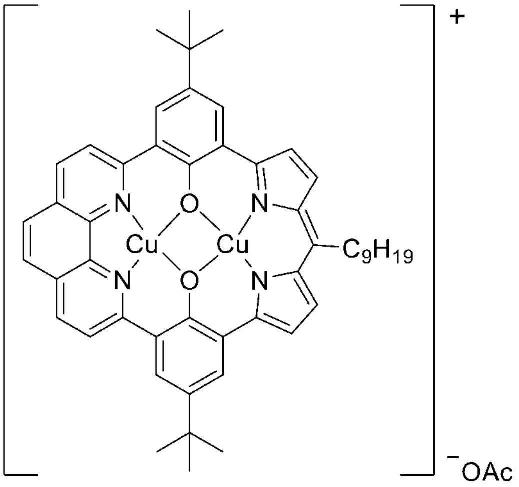

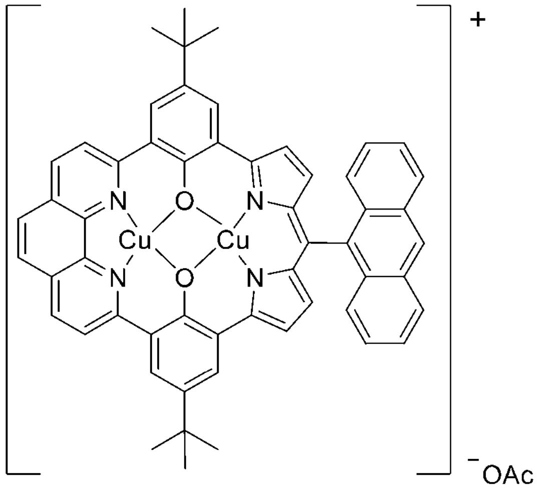

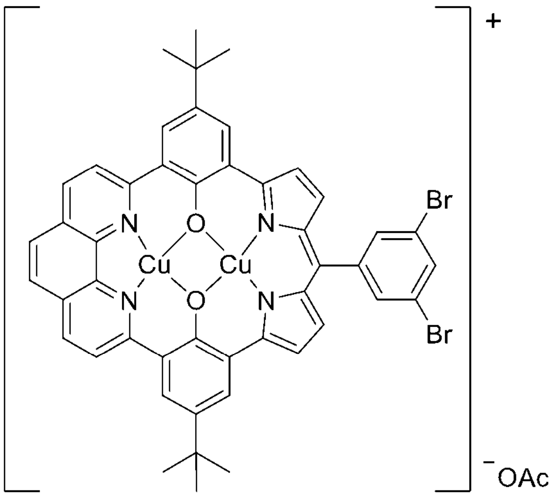

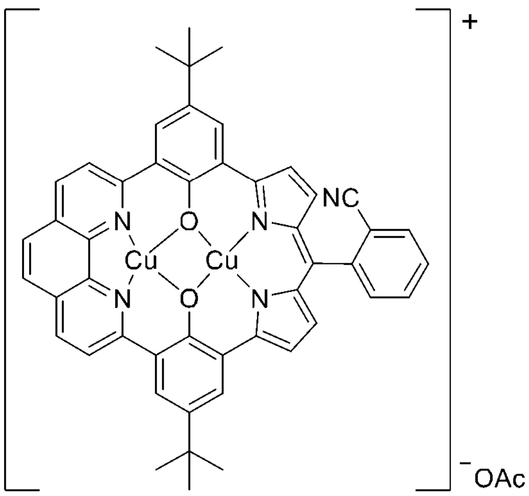

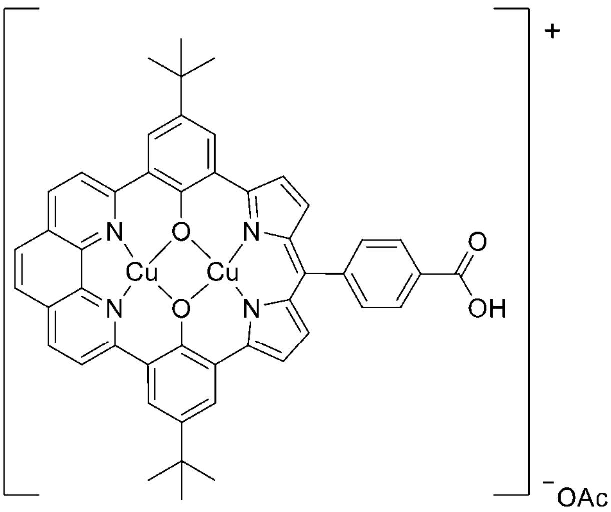

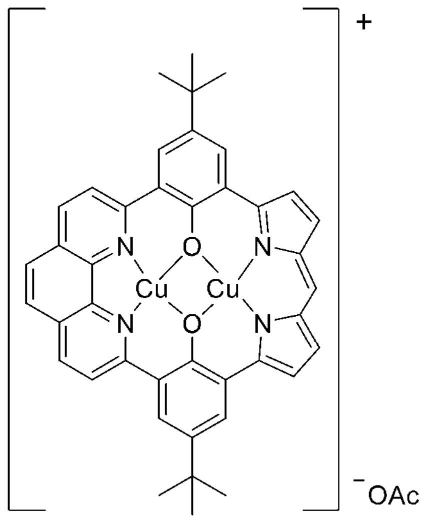

[Chem. 35]

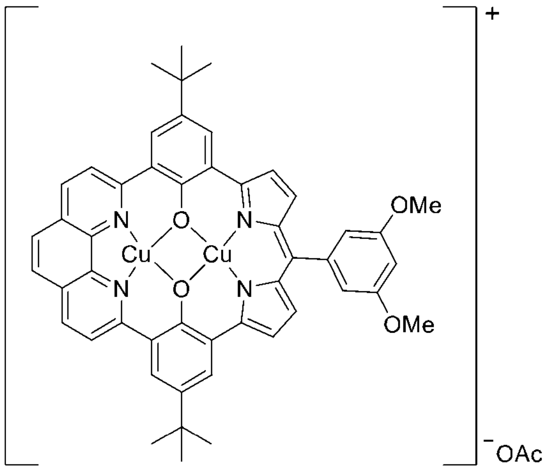

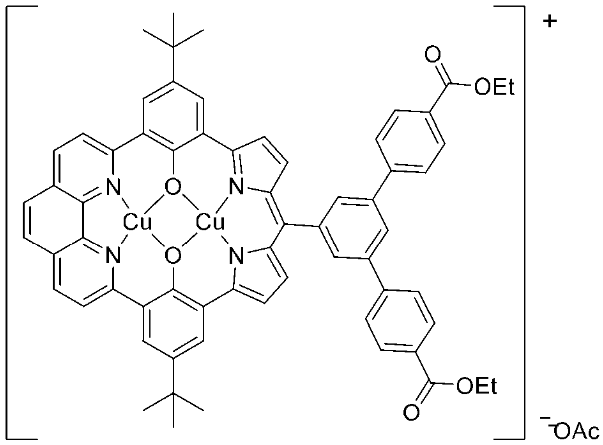

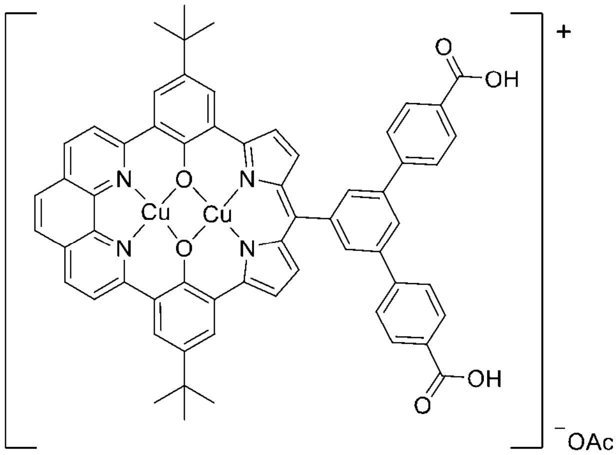

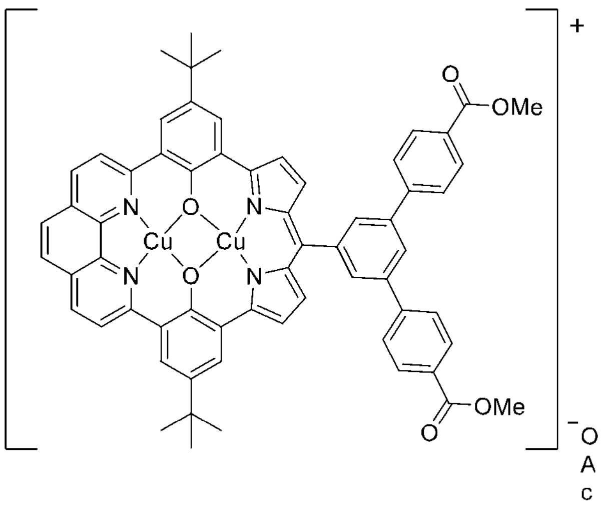

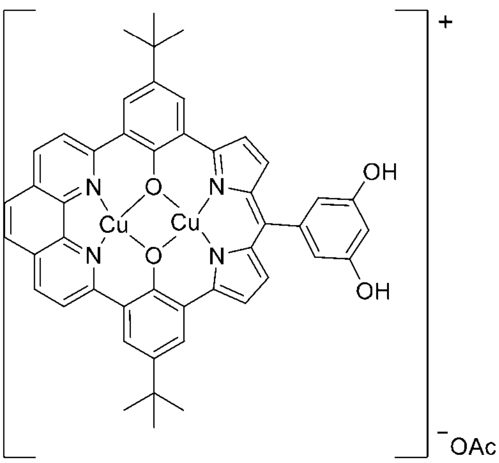

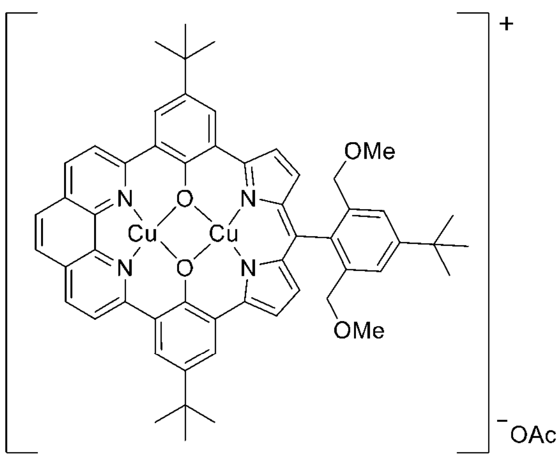

[Chem. 36]

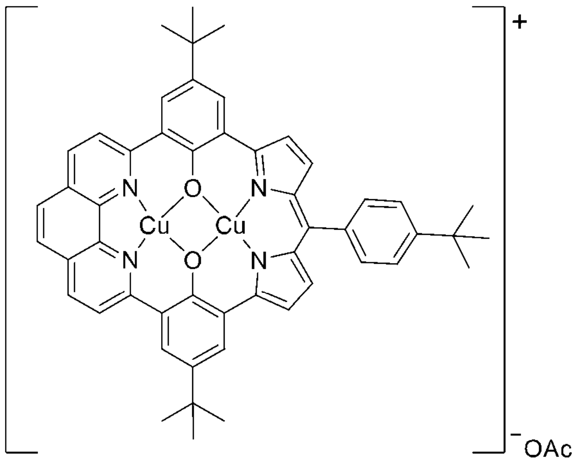

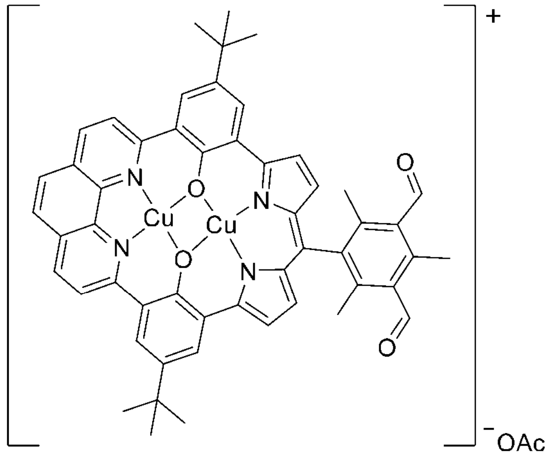

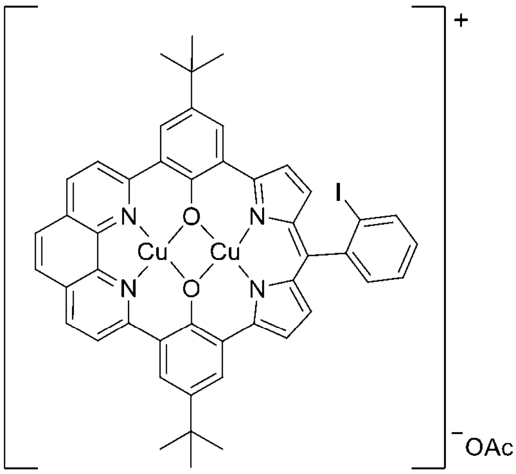

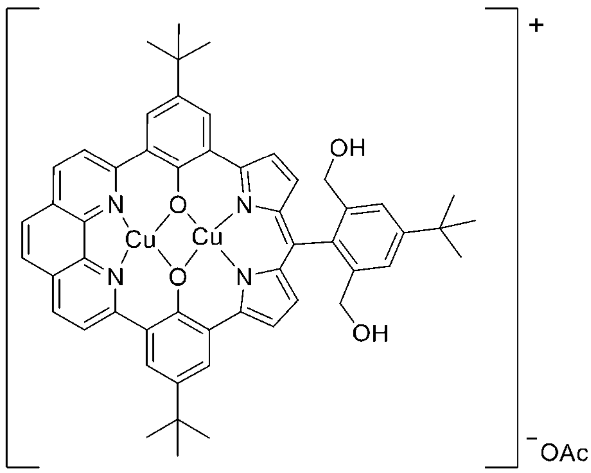

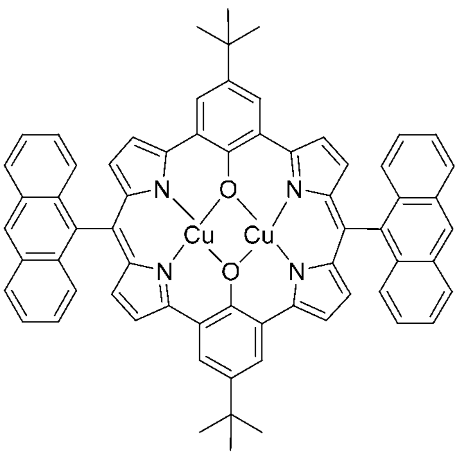

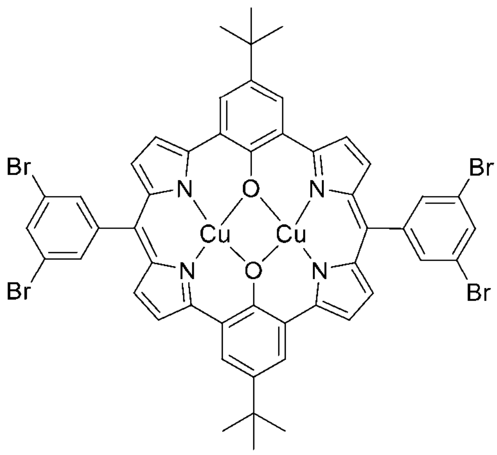

[Chem. 37]

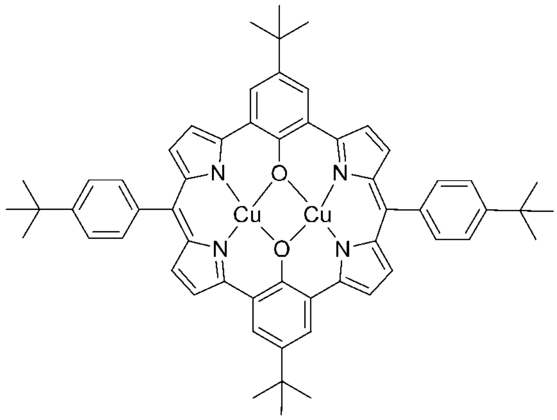

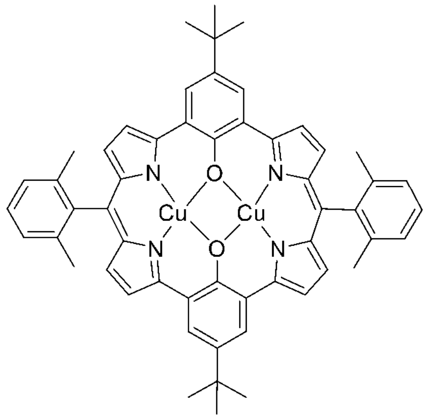

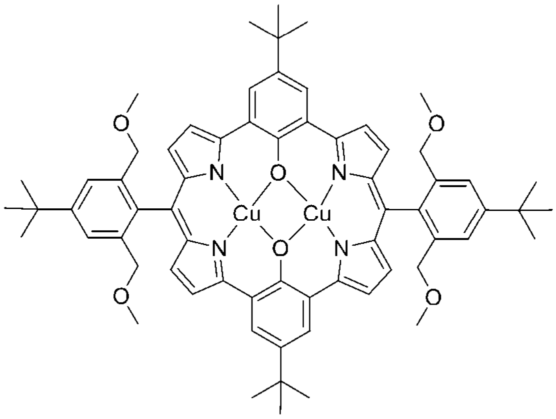

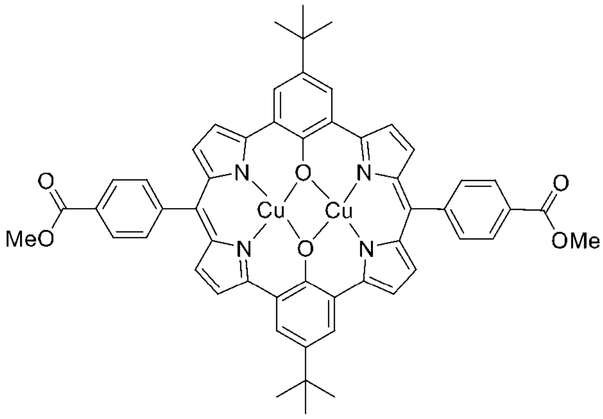

[Chem. 38]

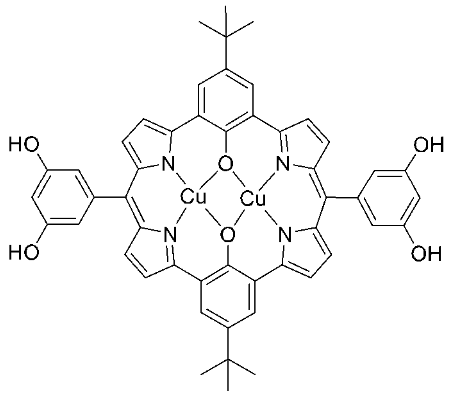

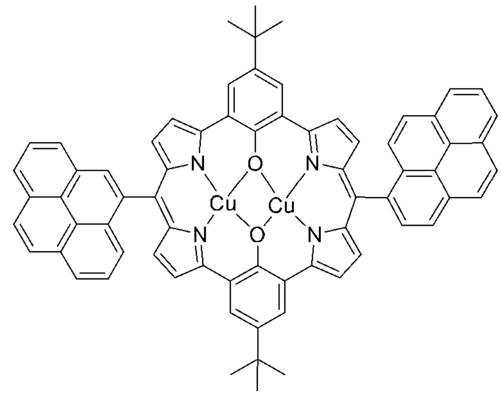

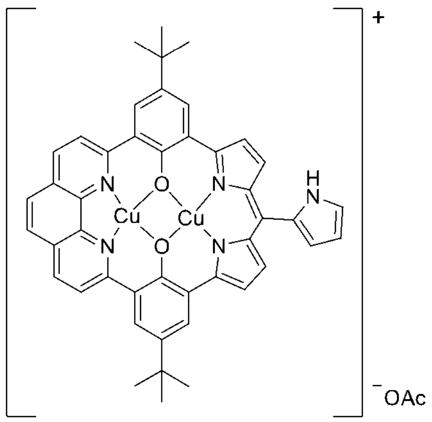

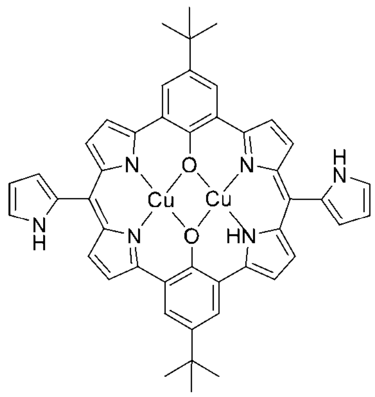

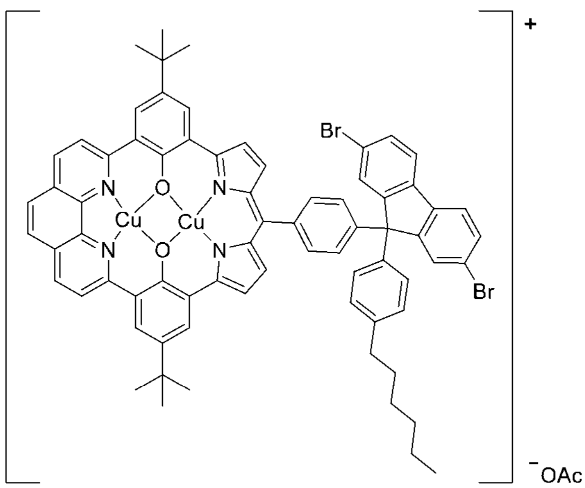

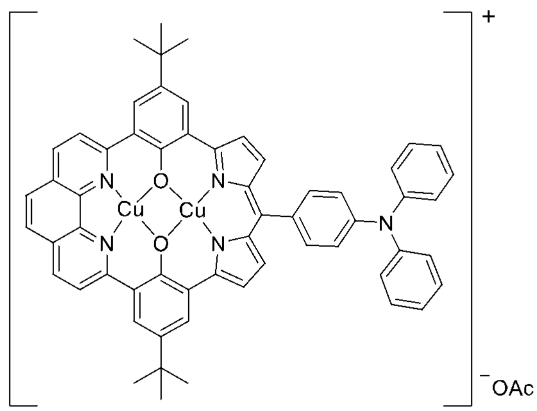

[Chem. 39]

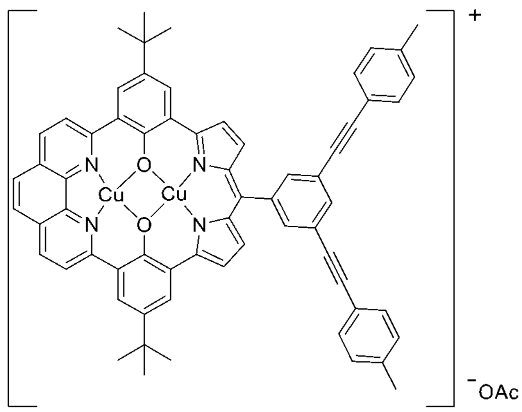

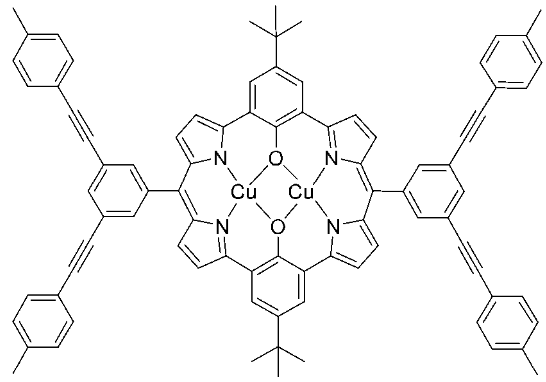

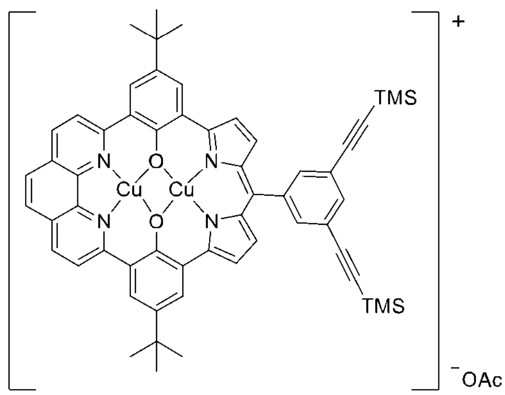

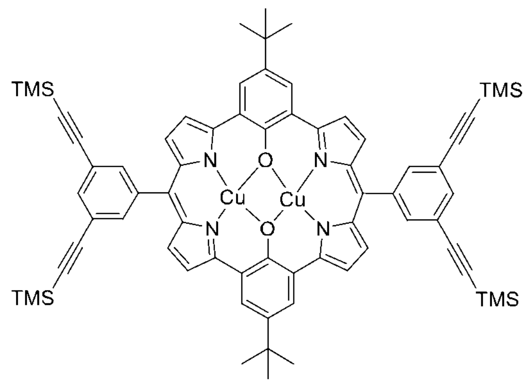

[Chem. 40]

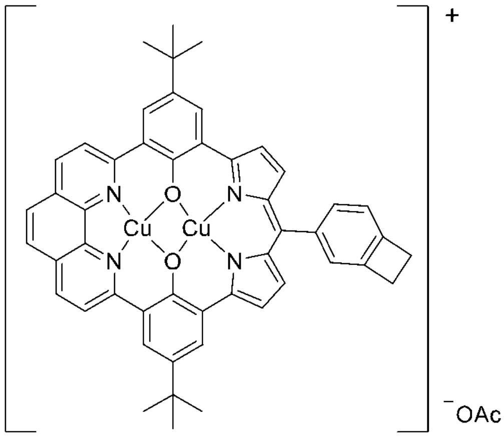

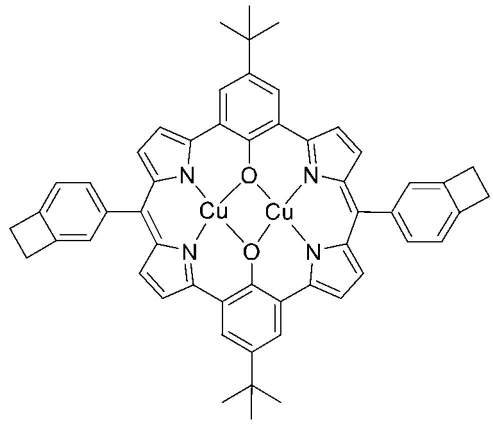

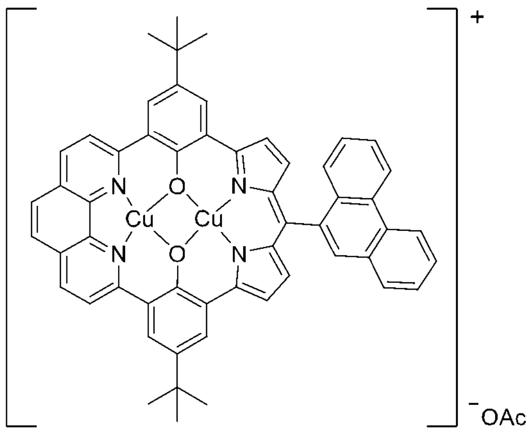

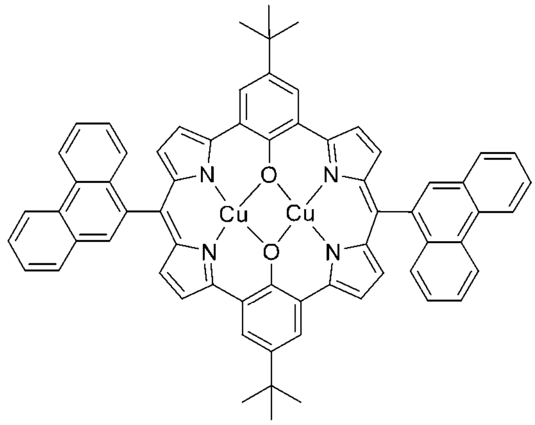

[Chem. 41]

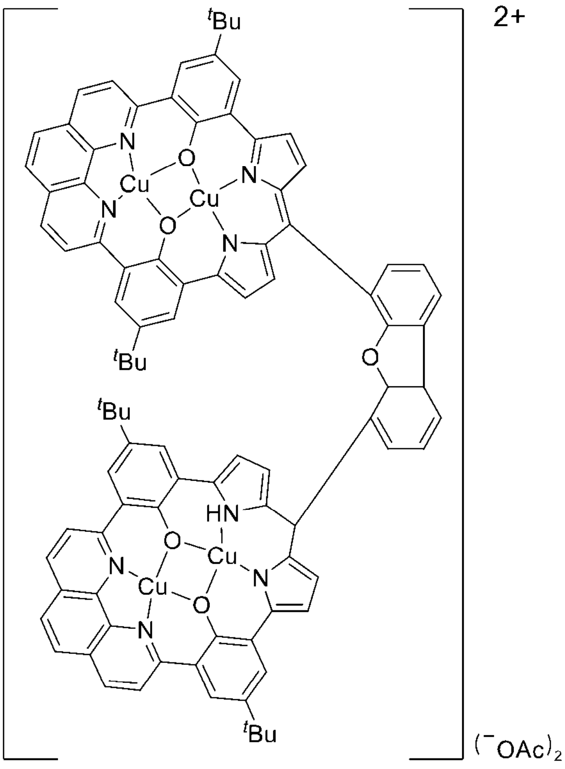

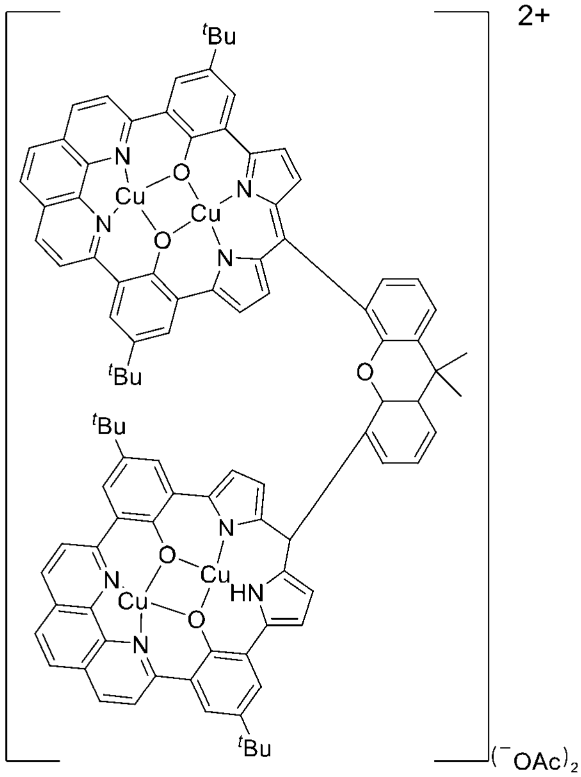

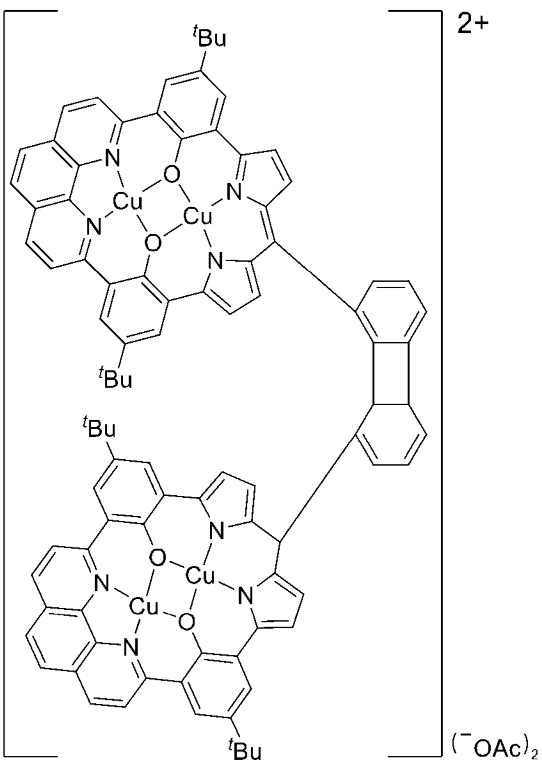

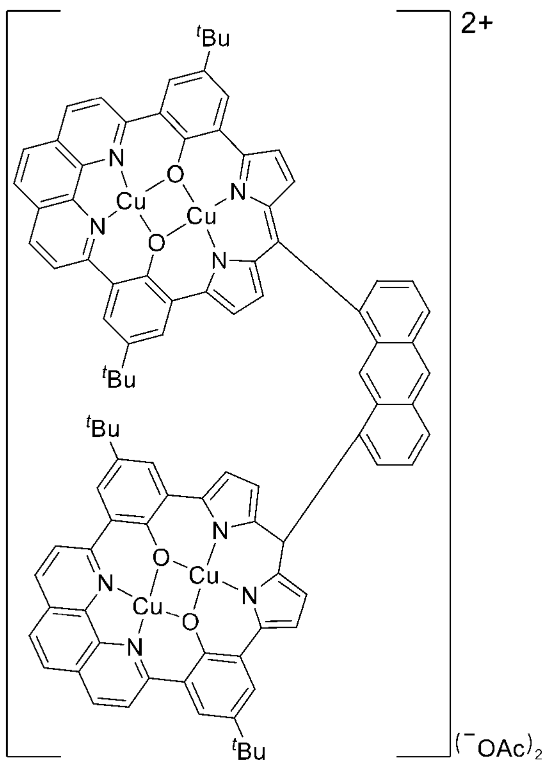

(Step of Reacting Carbon Dioxide with Water)

The ethylene production method according to the present disclosure includes a step of reacting carbon dioxide

with water in the presence of a carbon dioxide reduction catalyst.

The present step is not particularly limited as long as it is a step capable of reacting carbon dioxide with water in the presence of a carbon dioxide reduction catalyst, and from the viewpoint of ethylene selectivity, it is preferable to perform the present step using a carbon dioxide reduction apparatus including an oxidation electrode, the carbon dioxide reduction electrode according to the present disclosure, a membrane separating the oxidation electrode and the carbon dioxide reduction electrode, an electrolyte solution, and a power supply connected to the oxidation electrode and the carbon dioxide reduction electrode.

Note that details of the carbon dioxide reduction apparatus will be described below.

Examples of the method for reacting carbon dioxide with water using a carbon dioxide reduction apparatus include a method in which a current flows to an oxidation electrode and a carbon dioxide reduction electrode, carbon dioxide flows inside the apparatus so as to be in contact with the carbon dioxide reduction electrode, and water contained in an electrolyte solution of the carbon dioxide reduction apparatus and carbon dioxide are reacted on the carbon dioxide reduction electrode.

<Carbon Dioxide Reduction Electrode>

A carbon dioxide reduction electrode according to the present disclosure includes a carbon dioxide reduction catalyst that is a multinuclear copper complex and has a copper interatomic distance of 2.8 Å or less as determined by density functional theory or a conductive material on which a carbon dioxide reduction catalyst represented by Formula (1) is supported.

(Conductive Material)

The conductive material carries a carbon dioxide reduction catalyst.

As the carbon dioxide reduction catalyst included in the carbon dioxide reduction electrode according to the present disclosure, the carbon dioxide reduction catalyst used in the ethylene production method according to the present disclosure is applied, and preferred aspects are also the same.

The conductive material is preferably a porous carbon material.

Examples of the conductive material include Norit; Ketjen black; Vulcan; black pearl; carbon particles such as acetylene black; fullerenes such as C60 and C70; carbon nanotubes; carbon nanohorn; carbon fibers; graphene; graphene oxide; reduced graphene oxide; and graphene meso-sponge.

As a method for supporting the carbon dioxide reduction catalyst on the conductive material, it is preferable to perform a method by ultrasonic treatment of a dispersion obtained by adding a conductive material to a solution in which a carbon dioxide reduction catalyst is dissolved.

The amount of the carbon dioxide reduction catalyst supported on the conductive material is preferably an amount in which the mass of copper atoms with respect to the mass of the conductive material is 1 mass% or more and 50 mass% or less, and more preferably an amount in which the mass of copper atoms is 2 mass% or more and 10 mass% or less.

The amount of the carbon dioxide reduction catalyst supported on the conductive material is preferably an amount in which the mass of the carbon dioxide reduction catalyst with respect to the mass of the conductive material is 1 mass% or more and 100 mass% or less, and more preferably an amount in which the mass of the carbon dioxide reduction catalyst is 5 mass% or more and 50 mass% or less.

The mass of copper atoms with respect to the mass of the conductive material is measured using a thermogravimetric differential thermal (TG-DTA) analyzer. The measurement method is as follows.

The measurement can be performed by measuring a TG-DTA curve by heating from 25°C or lower to 900°C at a heating rate of 10°C/min in the air using a thermogravimetric differential thermal analyzer.

(Support)

The carbon dioxide reduction electrode according to the present disclosure preferably further includes a support that supports the conductive material.

The support preferably has conductivity, and examples thereof include carbon nanotubes, graphene, carbon black, carbon cloth, carbon paper, glassy carbon, graphite, and tantalum (Ta).

(Ion Conductor)

The carbon dioxide reduction electrode according to the present disclosure preferably includes an ion conductor.

Examples of the ionic conductor include an ionomer.

The ionomer is a polymer neutralized with ions.

The ionomer is preferably a polymer neutralized with a cation such as a metal (cationic ionomer) or a polymer neutralized with an anion (anionic ionomer).

Examples of the anionic ionomer include Sustanion manufactured by Dioxide Materials, AEMION manufactured by Ionomer innovations, Fumasep manufactured by FumaTech, and Orion manufactured by Orion.

The ion conductor is preferably 10 mass% or more and 200 mass% or less with respect to the mass of the conductive material on which the carbon dioxide reduction catalyst is supported.

(Other Components)

The carbon dioxide reduction electrode according to the present disclosure may include components other than the conductive material, the support, and the ion conductor.

Examples of the other components include a water repellent material. Examples of the water repellent material include a fluorine-containing resin, a silicon-containing resin, a silane coupling agent, and wax, a fluorine-containing resin is preferable from the viewpoint of a water-repellent effect, and examples of the fluorine-containing resin include polytetrafluoroethylene.

(Method for Producing Carbon Dioxide Reduction Electrode)

The carbon dioxide reduction electrode according to the present disclosure is preferably produced by preparing an electrode ink in which a conductive material on which a carbon dioxide reduction catalyst is supported, an ionic conductor, and other components as necessary are dispersed in a solvent, applying the electrode ink to a support, and drying the electrode ink.

(Example of Carbon Dioxide Reduction Electrode)

Fig. 1 is an example of a carbon dioxide reduction electrode according to the present disclosure.

Fig. 1 is a schematic cross-sectional view of the carbon dioxide reduction electrode according to the present disclosure.

In Fig. 1, a carbon dioxide reduction electrode 10 includes a layer 1 including a conductive material on which a carbon dioxide reduction catalyst is supported on a support 2.

When components other than the ion conductor are included, these components are included in the layer 1 including the conductive material on which the carbon dioxide reduction catalyst is supported.

<Carbon Dioxide Reduction Apparatus>

A carbon dioxide reduction apparatus according to the present disclosure includes: an oxidation electrode; the carbon dioxide reduction electrode according to the present disclosure; a membrane separating the oxidation electrode and the carbon dioxide reduction electrode; an electrolyte solution; and a power supply connected to the oxidation electrode and the carbon dioxide reduction electrode.

(Example of Carbon Dioxide Reduction Apparatus)

Fig. 2 is an example of a carbon dioxide reduction apparatus according to the present disclosure.

As illustrated in Fig. 2, a carbon dioxide reduction apparatus 100 includes: an oxidation electrode 11; the carbon dioxide reduction electrode 10; a membrane 12 separating the oxidation electrode 11 and the carbon dioxide reduction electrode 10; an electrolyte solution 13; and a power supply 14 connected to the oxidation electrode 11 and the carbon dioxide reduction electrode 10. The carbon dioxide reduction apparatus 100 includes an electrolytic bath 15 including these members and a reaction tank 16.

Here, the carbon dioxide reduction electrode 10 is preferably installed so that the conductive material on which the carbon dioxide reduction catalyst is supported is in contact with the electrolyte solution 13.

The carbon dioxide reduction apparatus 100 is applicable to a reaction for reducing carbon dioxide to produce ethylene. When used in the reaction, it is preferable to cause a current to flow from the carbon dioxide reduction electrode 10 toward the oxidation electrode 11 using the power supply 14. Then, it is preferable to flow carbon dioxide in the direction of arrow A into the reaction tank 16. The carbon dioxide flowing into the reaction tank 16 comes into contact with the carbon dioxide reduction catalyst in the carbon dioxide reduction electrode 10. As a result, the reaction represented by the following Reaction Formula 1 proceeds on the carbon dioxide reduction electrode 10, and the reaction represented by the following Reaction Formula 2 proceeds on the oxidation electrode 11.

Reaction Formula 1: $2CO_2+8H_2O+12e^- -C_2H_4+12OH^-$

Reaction Formula 2: $12OH^- \rightarrow 3O_2+6H_2O+12e^-$

Then, the produced ethylene flows out from the reaction tank 16 in the direction of arrow B.

Hereinafter, the carbon dioxide reduction electrode will be described in detail. Note that reference numerals are omitted.

(Oxidation Electrode)

Examples of the oxidation electrode include platinum, stainless steel, aluminum, copper, nickel, iron, titanium, and carbon, and platinum is preferable. The shape of the oxidation electrode is, for example, a foil shape or a mesh shape.

(Carbon Dioxide Reduction Electrode)

As the carbon dioxide reduction electrode, the carbon dioxide reduction electrode according to the present disclosure described above is applied.

(Membrane)

The membrane separating the oxidation electrode and the carbon dioxide reduction electrode is preferably an ion exchange membrane.

The ion exchange membrane is preferably an anion exchange membrane.

As the ion exchange membrane, for example, Sustainion RX37-50 Grade RT manufactured by Dioxide Materials can be used.

(Electrolyte solution)

Examples of the electrolyte solution include electrolyte solutions containing cations such as sodium ions or potassium ions, anions such as hydroxide ions, hydrogen carbonate ions, or carbonate ions, and water.

An ion concentration of the electrolyte solution is preferably 0.01 mol/L or more and 5.0 mol/L or less, and more preferably 0.5 mol/L or more and 2.0 mol/L or less.

(Power Supply)

The power supply is connected to the oxidation electrode and the carbon dioxide reduction electrode.

The power supply is not particularly limited as long as a current can flow between the carbon dioxide reduction electrode and the oxidation electrode.

As the power supply, for example, an electrochemical analyzer 701C manufactured by BAS can be used.

EXAMPLES

Examples will be described below, but the present invention is not limited to these Examples at all. Note that, in the following description, "part(s)" and "%" are all on a mass basis, unless otherwise specified.

Hereinafter, "TMEDA" means N,N,N',N'-tetramethylethylenediamine, "MTBE" means tert-butyl methyl ether, "THF" means tetrahydrofuran, "OAc" means an acetate anion, "DMSO" means dimethyl sulfoxide, "PhCHO" means benzaldehyde, and "$PhNH^+Me_2B(C_6F_5)_4^-$" means N,N-dimethylanilinium tetrakis(pentafluorophenyl)borate.

For NMR measurement, AV NEO 300 MHz NMR spectrometer manufactured by BRUKER was used. For ESI-MS measurement, 6130 LCMSD manufactured by Agilent was used.

<<Example 1>>

<Synthesis of Carbon Dioxide Reduction Catalyst>

A carbon dioxide reduction catalyst was synthesized by the following procedure.

(Synthesis of Compound 3)

A compound 3 was synthesized according to the following reaction formula.

[Chem. 42]

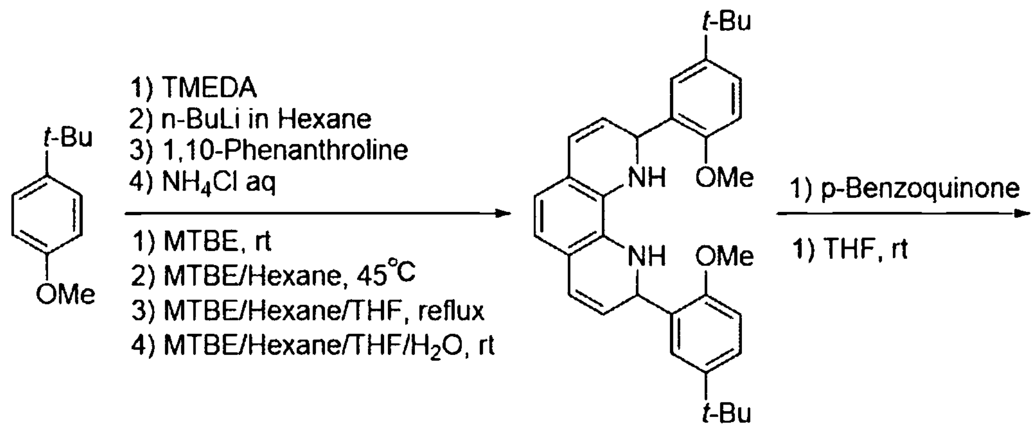


Compound 1

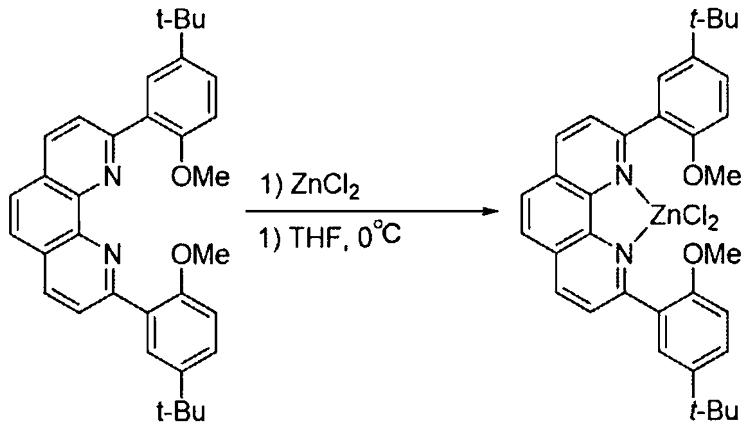


Compound 2 Compound 3

The inside of a reaction vessel was set to a nitrogen gas atmosphere, 135 mL of MTBE, 63.80 g (388 mmol) of 4-t-butylanisole, and 38.69 g (333 mmol) of TMEDA were added dropwise, and then, the resulting reactant was cooled to 0°C. 212.07 mL (1.6 mol/L, 333 mmol as n-butyllithium) of a hexane solution of n-butyllithium was added dropwise thereto, and the resulting solution was heated to 45°C and then stirred for 1.5 hours to obtain a lithiation reaction solution. The inside of another reaction vessel was set to a nitrogen gas atmosphere, and then, 10.00 g (55.5 mmol) of 1,10-phenanthroline anhydrous was suspended in 113 mL of THF at room temperature. The suspension was added dropwise to the lithiation reaction solution, and then, the resulting solution was heated to 65°C and stirred for 2 hours while being refluxed to obtain an arylation reaction solution. 100 g of a 20 mass% ammonium chloride aqueous solution was added dropwise to the arylation reaction solution cooled to room temperature. The resulting solution was stirred for 30 minutes and washed, an aqueous phase was removed, and then, an organic phase was concentrated under reduced pressure. The inside of another reaction vessel was set to a nitrogen gas atmosphere, and then, 12.00 g (111 mmol) of p-benzoquinone was dissolved in 113 mL of THF at room temperature. The solution was added dropwise to the concentrated organic phase, and the resulting solution was stirred at room temperature for 30 minutes to obtain an oxidation reaction solution containing a compound 2.

The inside of another reaction vessel was set to a nitrogen gas atmosphere, and then, 11.35 g (83.2 mmol) of zinc chloride was suspended in 113 mL of THF at room temperature. The suspension was added dropwise to the oxidation reaction solution at room temperature. The resulting suspension was cooled to 0°C and then stirred for 4 hours. Thereafter, the suspension was filtered at 0°C, washed with THF, and then dried under reduced pressure to obtain a compound 3 in a yield of 55%. The identification data of the resulting compound 3 is shown below.

$^1H$-NMR(300MHz,$CDCl_3$) :δ(ppm)=1.37(s,18H),3.76(s,6H),6.98(d,J=9.0Hz,2H),7.52(dd,J=9.0Hz,2.4Hz,2H),7.87 (d,J=2.4Hz, 2H), 8.02 (d ,J=8.4Hz,2H),8.02(s,2H),8.50(d,J=8.4Hz,2H)

(Synthesis of Compound 4)

A compound 4 was synthesized according to the following reaction formula.

[Chem. 43]

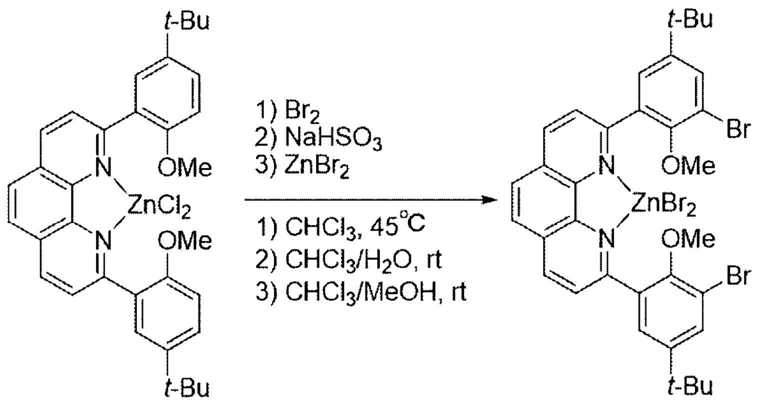


Compound 3 Compound 4

The inside of a reaction vessel was set to a nitrogen gas atmosphere, and then, 8.00 g (12.48 mmol) of the compound 3 was added to and dissolved in 108 mL of chloroform at room temperature. 15.96 g (99.85 mol) of bromine was added dropwise thereto with stirring, the temperature was raised to 45°C, and then, the resulting solution was stirred for 6 hours to obtain a bromination reaction solution. The inside of another reaction vessel was set to a nitrogen gas atmosphere, and then, 10.39 g (99.85 mmol) of sodium thiosulfate was dissolved in 160 mL of water at room temperature. The aqueous solution was added dropwise to the bromination reaction solution cooled to 0°C, the solution was stirred for 1 hour and washed, and then, an aqueous phase was removed. The inside of another reaction vessel was set to a nitrogen gas atmosphere, and then, 2.81 g (12.48 mmol) of zinc bromide was dissolved in 151 mL of methanol at room temperature. The solution was added to the washed organic phase, the temperature was raised to 75°C, and then, concentration was performed. 202 mL of methanol was added thereto, and the resulting solution was stirred for 1 hour while being refluxed at 75°C. The resulting solution was cooled to 0°C and stirred for 1 hour, filtered, washed with methanol, and then dried under reduced pressure to obtain a compound 4 in a yield of 88%. The identification data of the resulting compound 4 is shown below.

$^1H$-NMR(300MHz,$CDCl_3$):δ(ppm)=1.36(s,18H),3.65(s,6H),7.63 (d, J=2.4Hz,2H),7.87(s,2H),7.93(d,J=2.4Hz,2H),8.17(d,J=8.1Hz,2H),8. 31(d,J=8.1Hz, 2H)

(Synthesis of Compound 6)

A compound 6 was synthesized according to the following reaction formula.

[Chem. 44]

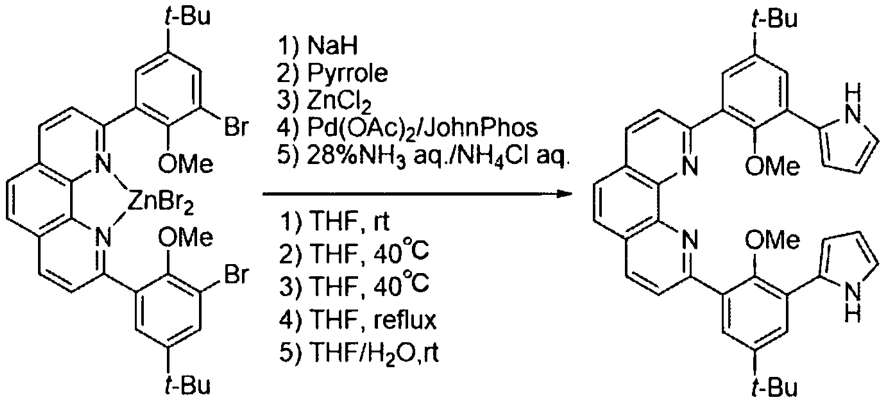


Compound 4 Compound 5

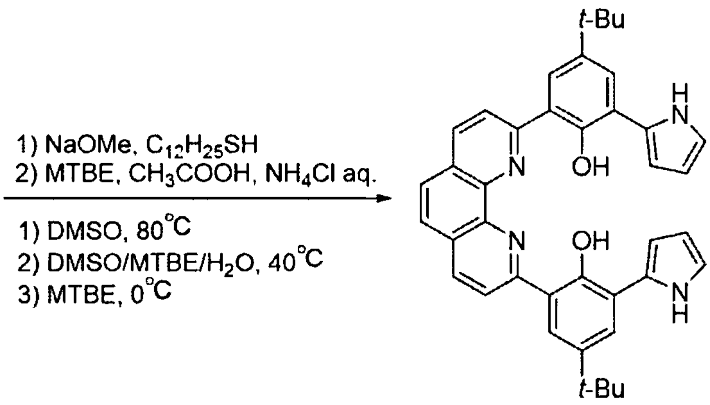


Compound 6

The inside of a reaction vessel was set to a nitrogen gas atmosphere, and then, 106 mL of THF was added to and suspended in 4.39 g (110 mmol) of sodium hydride. The resulting suspension was heated to 40°C, 32.67 g (487 mmol) of pyrrole was added dropwise thereto for 20 minutes, and the resulting solution was stirred for 30 minutes to obtain a reaction solution. The inside of another reaction vessel was set to a nitrogen gas atmosphere, and then, 19.96 g (146 mmol) of zinc chloride was suspended in 137 mL of THF at room temperature. The suspension was added dropwise to the reaction solution, stirred for 30 minutes, and then cooled to room temperature. 32.50 g (36.6 mmol) of the compound 4 was added thereto. The inside of another reaction vessel was set to a nitrogen gas atmosphere, and then, 0.082 g (0.37 mmol) of palladium acetate and 0.219 g (0.73 mmol) of 2-(di-tert-butylphosphino)biphenyl were dissolved in 6.5 mL of THF at room temperature to obtain a catalyst solution. The catalyst solution was added dropwise to the reaction solution, the temperature was raised to 75°C, and then, the resulting solution was stirred for 6 hours while being refluxed, and then cooled to room temperature.

The inside of another reaction vessel was set to a nitrogen gas atmosphere, and then, 86.23 g of ammonium chloride and 178.15 g of an ammonia aqueous solution (28%, 2,930 mmol) were dissolved in 217 mL of water at room temperature. The aqueous solution was added dropwise to the reaction solution, and the resulting solution was stirred at room temperature for 30 minutes and washed to remove an aqueous phase. 216 g of a 24.8 mass% ammonium chloride aqueous solution was added dropwise to the resulting organic phase, and the resulting solution was stirred for 15 minutes and washed to remove the aqueous phase.

79 mL of DMSO was added to the resulting organic phase, the temperature was raised to 82°C, and THF was removed by concentration under reduced pressure. 6.18 g (30.5 mmol) of 1-dodecanethiol and 7.06 g (28%, 36.6 mmol as sodium methoxide) of a methanol solution of sodium methoxide were added dropwise thereto, and the resulting solution was stirred at 82°C for 6.5 hours. The reaction solution was cooled to 40°C, and 58.6 mL of MTBE was added. The inside of another reaction vessel was set to a nitrogen gas atmosphere, and then, 23.50 g of ammonium chloride and 2.93 g (48.8 mmol) of acetic acid were dissolved in 86.7 mL of water at room temperature. The aqueous solution was added dropwise to the reaction solution, and the resulting solution was stirred at 40°C for 30 minutes and washed to remove an aqueous phase. The resulting organic phase was cooled to 0°C, stirred for 2 hours, and then filtered. The resulting crystals were washed with MTBE and methanol in this order and dried under reduced pressure to obtain a compound 6 in a yield of 76%. The identification data of the resulting compound 6 is shown below.

$^1H$-NMR (300MHz, $CDCl_3$) : $\delta$ (ppm) = 1. 40 (s, 18H), 6.25 (m, 2H), 6.44 (m, 2H), 6.74 (m, 2H), 7.84 (s, 2H), 7 .89 (s, 2H), 7.92 (s, 2H), 8.35 (d, J=8 .4Hz, 2H),8.46(d,J=8.4Hz,2H),10.61(s,2H),15.88(s,2H)

(Synthesis of Compound 8)

A compound 8 was synthesized according to the following reaction formula by the method described in WO 2019-026883 A.

[Chem. 45]

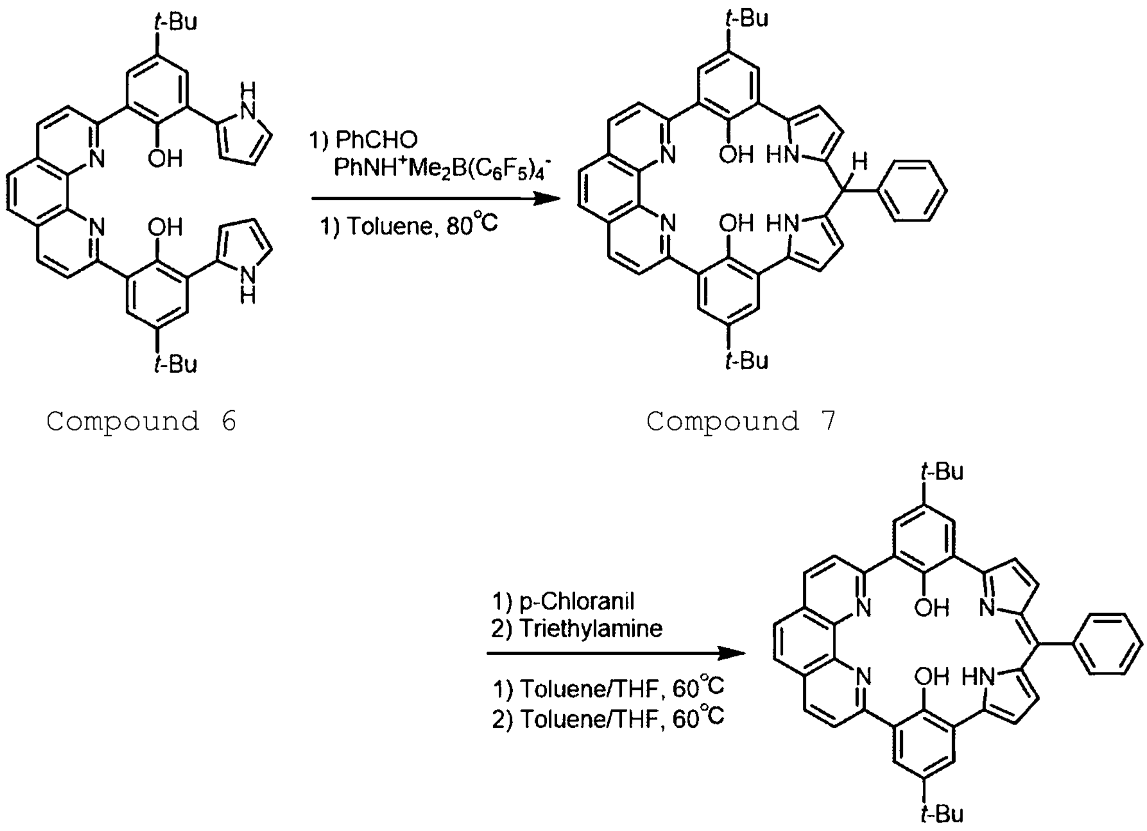


Compound 6

Compound 7

Compound 8

(Synthesis of Oxygen-Bridged Dinuclear Copper Complex 1)

An oxygen-bridged dinuclear copper complex 1 (that is, a carbon dioxide reduction catalyst 1) was synthesized according to the following reaction formula.

[Chem. 46]

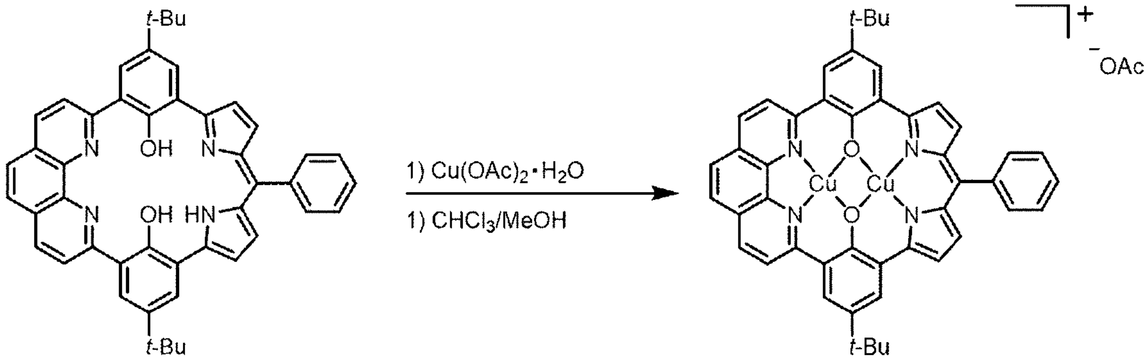


Compound 8

Oxygen-bridged dinuclear copper complex 1

The inside of a reaction vessel was set to a nitrogen gas atmosphere, and then, 29 mL of methanol which was previously degassed was added to and suspended in 1.80 g (9.02 mmol) of copper acetate monohydrate. 31 mL of chloroform was added thereto, and the temperature was raised to 50°C to obtain a copper acetate solution. The inside of another reaction vessel was set to a nitrogen gas atmosphere, and then, a suspension containing 2.50 g (3.61 mmol) of the compound 8 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 1. The reaction solution was cooled to room temperature and then filtered. The resulting crystals were washed with methanol and dried under reduced pressure to obtain an oxygen-bridged dinuclear copper complex 1 in an amount of 2.79 g and a yield of 95%. The identification data of the resulting oxygen-bridged dinuclear copper complex 1 (carbon dioxide reduction catalyst) is shown below. The results of ESI-MS measurement were confirmed as follows.

ESI-MS$[M-OAc]^+$:m/z=815.2

A copper interatomic distance of the oxygen-bridged dinuclear copper complex 1 was 2.70 (Å).

A method for calculating the copper interatomic distance will be described below. The same applies to the method for calculating the copper interatomic distance in the following Examples.

<Production of Carbon Dioxide Reduction Electrode>

(Synthesis of Conductive Material on Which Carbon Dioxide Reduction Catalyst Is Supported)

In a reaction vessel, 137.84 mg (0.157 mmol) of the oxygen-bridged dinuclear copper complex 1 was weighed, 105 mL of chloroform was added thereto, the temperature was raised to 60°C, and stirring and ultrasonic irradiation were repeated. After confirming that the oxygen-bridged dinuclear copper complex 1 was dissolved, the solution was cooled to room temperature to obtain a solution. In another reaction vessel, 400 mg of carbon black (Ketjen Black EC600JD manufactured by LION SPECIALTY CHEMICALS CO., Ltd.) as a conductive material was weighed, and the solution was added dropwise thereto to prepare a dispersion. By irradiating the dispersion with ultrasonic waves for 15 minutes, the conductive material on which the carbon dioxide reduction catalyst was supported was uniformly dispersed to obtain a suspension having a content of copper atoms of 5 mass% with respect to the mass of carbon black. The suspension was filtered using a PTFE membrane filter and then dried under reduced pressure to obtain a conductive material on which a carbon dioxide reduction catalyst was supported.

(Production of Carbon Dioxide Reduction Electrode)

In a screw tube, 25.00 mg of a powder of a conductive material on which the carbon dioxide reduction catalyst was supported and 25.00 mg of PTFE fine particles (manufactured by Sigma-Aldrich Corporation) having a particle diameter of 1 μm were weighed, and 2.50 mL of ethanol and 500 mg of a 5 mass% ethanol solution of Sustanion XA-9 (manufactured by Dioxide Materials) as an anionic ionomer which is an ion conductor were added thereto, thereby preparing a dispersion. The dispersion was irradiated with ultrasonic waves for 5 minutes to obtain an ink.

Carbon paper (diameter: 25 mm/GDL36BB manufactured by SIGRACET) was used as a support, and 250 mg of the ink obtained above was applied to the carbon paper and then dried to obtain a carbon dioxide reduction electrode of

Example 1.

<Manufacture of Carbon Dioxide Reduction Apparatus>

A carbon dioxide reduction apparatus was manufactured in the same manner as the carbon dioxide reduction apparatus 100 illustrated in Fig. 2.

A platinum mesh was used as the oxidation electrode 11. The carbon dioxide reduction electrode of Example 1 was used as the carbon dioxide reduction electrode 10. As the membrane 12, an anion exchange membrane (Sustainion RX37-50 Grade RT manufactured by Dioxide Materials) was used. A 1.0 M potassium hydroxide aqueous solution was used as the electrolyte solution 13. As the power supply 14, an electrochemical measurement apparatus (electrochemical analyzer 701C manufactured by BAS) was used. Furthermore, as a reference electrode, an Ag/AgCl reference electrode (manufactured by EC FRONTIER CO., LTD.) was placed in the electrolyte solution 13 between the membrane 12 and the carbon dioxide reduction electrode 10.

<Production of Ethylene>

Humidified carbon dioxide gas was circulated in the direction of arrow A in the reaction tank 16 through a washing air bottle containing water at 60°C. The inflow of gas was set to 5 mL/min using a mass flow controller (GF40 manufactured by BROOKS).

Ethylene was produced by applying a constant potential of -2.2 V to a reversible hydrogen electrode (Reversible Hydrogen Electrode, RHE) to the carbon dioxide reduction electrode 10 using an electrochemical measurement apparatus.

<<Example 2>>

<Production of Ethylene>

Ethylene was synthesized in the same manner as in Example 1, except that a constant potential of -3.2 V was applied to a reversible hydrogen electrode (Reversible Hydrogen Electrode, RHE).

<<Example 3>>

(Synthesis of Oxygen-Bridged Dinuclear Copper Complex 2)

An oxygen-bridged dinuclear copper complex 2 (that is, a carbon dioxide reduction catalyst 2) was synthesized according to the following reaction formula using the compound 10 synthesized by the method described in WO 2009-084283 A.

[Chem. 47]

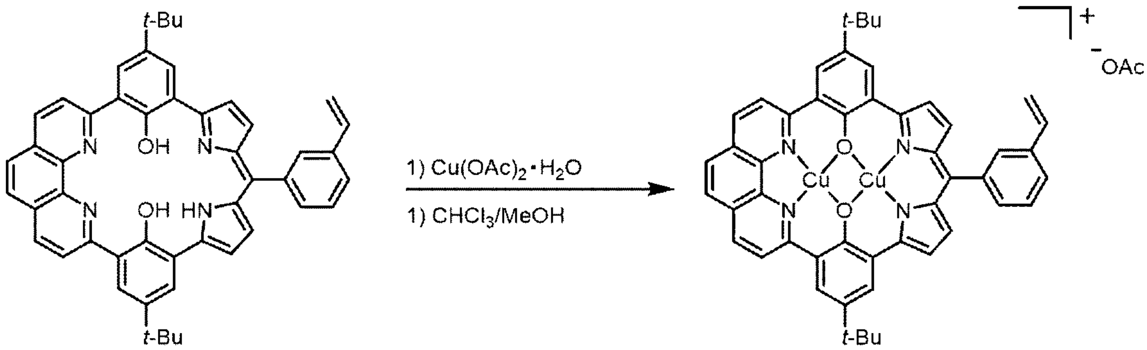


Compound 10

Oxygen-bridged dinuclear copper complex 2

The inside of a reaction vessel was set to a nitrogen gas atmosphere, and then, 29 mL of methanol which was previously degassed was added to and suspended in 0.69 g (3.48 mmol) of copper acetate monohydrate. 31 mL of chloroform was added thereto, and the temperature was raised to 50°C to obtain a copper acetate solution. The inside of

another reaction vessel was set to a nitrogen gas atmosphere, and then, a suspension containing 1.0 g (1.39 mmol) of the compound 10 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 2. The reaction solution was cooled to room temperature and then filtered. The resulting crystals were washed with methanol and dried under reduced pressure to obtain an oxygen-bridged dinuclear copper complex 2 in an amount of 1.25 g and a yield of 98%. The identification data of the resulting oxygen-bridged dinuclear copper complex 2 (carbon dioxide reduction catalyst) is shown below. The results of ESI-MS measurement were confirmed as follows.
ESI-MS[M-OAc]$^+$:m/z=841.17

A copper interatomic distance of the oxygen-bridged dinuclear copper complex 2 was 2.73 (Å).

<Production of Carbon Dioxide Reduction Electrode>

A carbon dioxide reduction electrode of Example 3 was obtained by the same procedure as in Example 1, except that an oxygen-bridged dinuclear copper complex 2 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 3 was used instead of the carbon dioxide reduction electrode of Example 1.

<Production of Ethylene>

Ethylene was synthesized in the same procedure as in Example 2, except that the carbon dioxide reduction apparatus of Example 3 was used instead of the carbon dioxide reduction apparatus of Example 1.

<<Example 4>>

(Synthesis of Oxygen-Bridged Dinuclear Copper Complex 3)

An oxygen-bridged dinuclear copper complex 3 (that is, a carbon dioxide reduction catalyst 3) was synthesized according to the following reaction formula using the compound 11 synthesized by the method described in WO 2009-084283 A.

[Chem. 48]

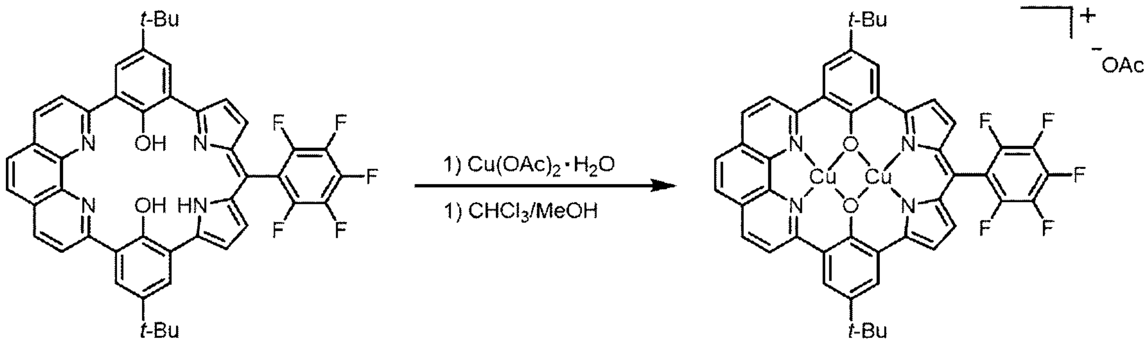


Compound 11

Oxygen-bridged dinuclear copper complex 3

The inside of a reaction vessel was set to a nitrogen gas atmosphere, and then, 29 mL of methanol which was previously degassed was added to and suspended in 0.64 g (3.19 mmol) of copper acetate monohydrate. 31 mL of chloroform was added thereto, and the temperature was raised to 50°C to obtain a copper acetate solution. The inside of another reaction vessel was set to a nitrogen gas atmosphere, and then, a suspension containing 1.0 g (1.28 mmol) of the compound 11 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 3. The reaction solution was cooled to room temperature

and then filtered. The resulting crystals were washed with methanol and dried under reduced pressure to obtain an oxygen-bridged dinuclear copper complex 3 in an amount of 1.23 g and a yield of 42%. The identification data of the resulting oxygen-bridged dinuclear copper complex 3 (carbon dioxide reduction catalyst 3) is shown below. The results of ESI-MS measurement were confirmed as follows.
ESI-MS$[M+H]^+$:m/z=906.1

A copper interatomic distance of the oxygen-bridged dinuclear copper complex 3 was 2.69 (Å).

<Production of Carbon Dioxide Reduction Electrode>

A carbon dioxide reduction electrode of Example 4 was obtained by the same procedure as in Example 1, except that an oxygen-bridged dinuclear copper complex 3 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 4 was used instead of the carbon dioxide reduction electrode of Example 1.

<Production of Ethylene>

Ethylene was synthesized in the same procedure as in Example 2, except that the carbon dioxide reduction apparatus of Example 4 was used instead of the carbon dioxide reduction apparatus of Example 1.

<<Example 5>>

(Synthesis of Oxygen-Bridged Dinuclear Copper Complex 4)

An oxygen-bridged dinuclear copper complex 4 (that is, a carbon dioxide reduction catalyst 4) was synthesized according to the following reaction formula using the compound 12 synthesized by the method described in WO 2009-084283 A.

[Chem. 49]

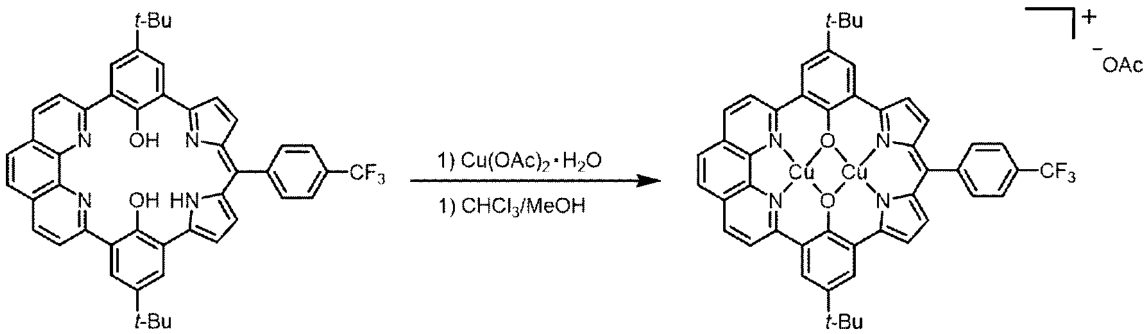


Compound 12

Oxygen-bridged dinuclear copper complex 4

The inside of a reaction vessel was set to a nitrogen gas atmosphere, and then, 29 mL of methanol which was previously degassed was added to and suspended in 0.66 g (3.29 mmol) of copper acetate monohydrate. 31 mL of chloroform was added thereto, and the temperature was raised to 50°C to obtain a copper acetate solution. The inside of another reaction vessel was set to a nitrogen gas atmosphere, and then, a suspension containing 1.0 g (1.31 mmol) of the compound 12 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 4. The reaction solution was cooled to room temperature and then filtered. The resulting crystals were washed with methanol and dried under reduced pressure to obtain an oxygen-bridged dinuclear copper complex 4 in an amount of 1.24 g and a yield of 100%. The identification data of the resulting oxygen-bridged dinuclear copper complex 4 (carbon dioxide reduction catalyst) is shown below. The results of ESI-MS measurement were confirmed as follows.
ESI-MS$[M-OAc]^+$:m/z=883.16

A copper interatomic distance of the oxygen-bridged dinuclear copper complex 4 was 2.73 (Å).

<Production of Carbon Dioxide Reduction Electrode>

A carbon dioxide reduction electrode of Example 5 was obtained by the same procedure as in Example 1, except that an oxygen-bridged dinuclear copper complex 4 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 5 was used instead of the carbon dioxide reduction electrode of Example 1.

<Production of Ethylene>

Ethylene was synthesized in the same procedure as in Example 2, except that the carbon dioxide reduction apparatus of Example 5 was used instead of the carbon dioxide reduction apparatus of Example 1.

<<Example 6>>

(Synthesis of Compound 13)

A compound 13 was synthesized according to the following reaction formula.

[Chem. 50]

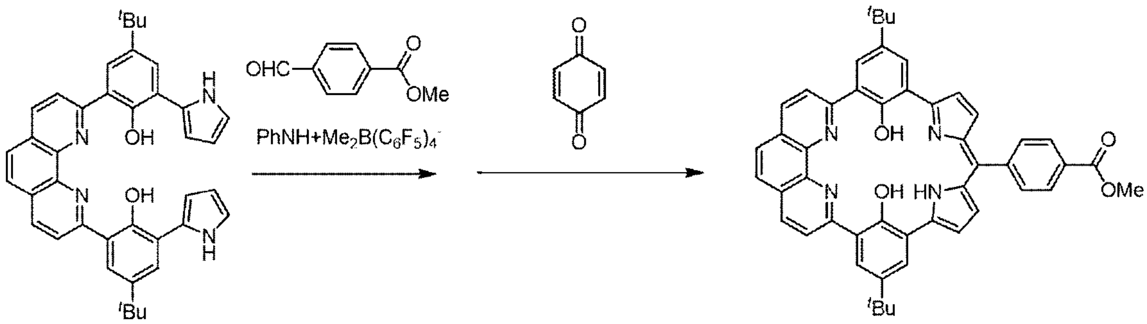


Compound 6 Compound 13

Under a nitrogen atmosphere, 92 ml of dehydrated toluene and 231 mg (0.29 mmol) of N,N-dimethylanilinium tetrakispentafluorophenyl borate were added, and the resulting solution was heated to 80°C with stirring using a rotor. A mixed solution of 1.49 g (9.06 mmol) of aldehyde, 5.0 g (8.24 mmol) of the compound 6, and 12 ml of toluene was added dropwise to the solution. After stirring for 3 hours, the resulting solution was allowed to cool, and the temperature of the reaction solution was gradually adjusted to room temperature.

A solution prepared by dissolving 0.98 g (9.06 mmol) of benzoquinone in 11 ml of THF was added dropwise to the reaction solution. After completion of the reaction was confirmed, the resulting reaction solution was filtered to obtain a target compound 13 in an amount of 5.49 g and a yield of 99%. The identification data of the resulting compound 13 is shown below. The results of ESI-MS measurement were confirmed as follows.
ESI-MS$[M+H]^+$:m/z=751.3

(Synthesis of Oxygen-Bridged Dinuclear Copper Complex 5)

An oxygen-bridged dinuclear copper complex 5 (that is, a carbon dioxide reduction catalyst 5) was synthesized according to the following reaction formula.

[Chem. 51]

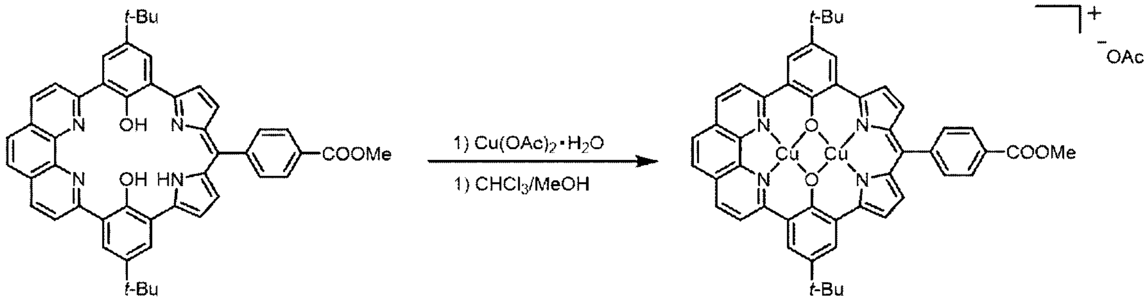


Compound 13

Oxygen-bridged dinuclear copper complex 5

The inside of a reaction vessel was set to a nitrogen gas atmosphere, and then, 29 mL of methanol which was previously degassed was added to and suspended in 0.67 g (3.33 mmol) of copper acetate monohydrate. 31 mL of chloroform was added thereto, and the temperature was raised to 50°C to obtain a copper acetate solution. The inside of another reaction vessel was set to a nitrogen gas atmosphere, and then, a suspension containing 1.0 g (1.33 mmol) of the compound 13 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 5. The reaction solution was cooled to room temperature and then filtered. The resulting crystals were washed with methanol and dried under reduced pressure to obtain an oxygen-bridged dinuclear copper complex 5 in an amount of 1.16 g and a yield of 93%. The identification data of the resulting oxygen-bridged dinuclear copper complex 5 (carbon dioxide reduction catalyst 5) is shown below. The results of ESI-MS measurement were confirmed as follows.

ESI-MS[M-OAc]$^+$:m/z=873.16

A copper interatomic distance of the oxygen-bridged dinuclear copper complex 5 was 2.73 (Å).

<Production of Carbon Dioxide Reduction Electrode>

A carbon dioxide reduction electrode of Example 6 was obtained by the same procedure as in Example 1, except that an oxygen-bridged dinuclear copper complex 5 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 6 was used instead of the carbon dioxide reduction electrode of Example 1.

<Production of Ethylene>

Ethylene was synthesized in the same procedure as in Example 2, except that the carbon dioxide reduction apparatus of Example 6 was used instead of the carbon dioxide reduction apparatus of Example 1.

<<Example 7>>

(Synthesis of Compound 14)

A compound 14 was synthesized according to the following reaction formula.

[Chem. 52]

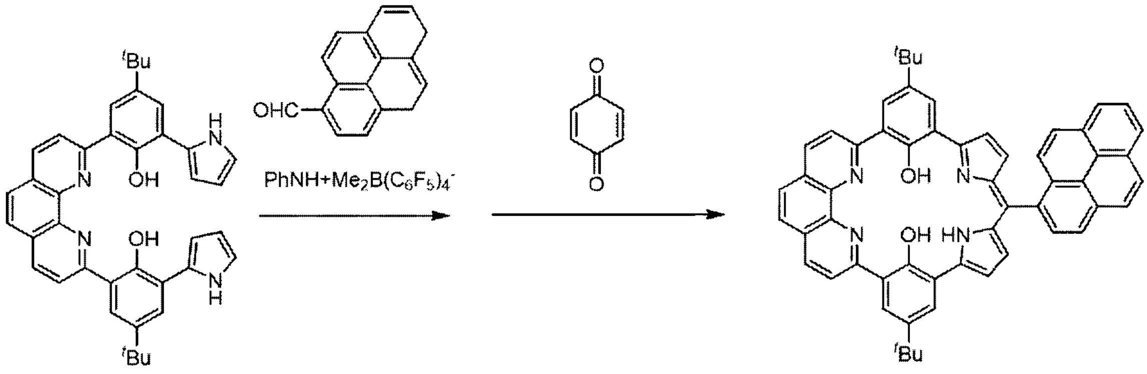


Compound 6 Compound 14

Under a nitrogen atmosphere, 92 ml of dehydrated toluene and 231 mg (0.29 mmol) of N,N-dimethylanilinium tetrakispentafluorophenyl borate were added, and the resulting solution was heated to 80°C with stirring using a rotor. A mixed solution of 2.09 g (9.06 mmol) of aldehyde, 5.0 g (8.24 mmol) of the compound 6, and 12 ml of toluene was added dropwise to the solution. After stirring for 3 hours, the resulting solution was allowed to cool, and the temperature of the reaction solution was gradually adjusted to room temperature.

A solution prepared by dissolving 0.98 g (9.06 mmol) of benzoquinone in 11 ml of THF was added dropwise to the reaction solution. After completion of the reaction was confirmed, the resulting reaction solution was filtered to obtain a target compound 14 in an amount of 5.89 g and a yield of 99%. The identification data of the resulting compound 14 is shown below. The results of ESI-MS measurement were confirmed as follows.

ESI-MS$[M+H]^+$:m/z=817.4

(Synthesis of Oxygen-Bridged Dinuclear Copper Complex 6)

An oxygen-bridged dinuclear copper complex 6 (that is, a carbon dioxide reduction catalyst 6) was synthesized according to the following reaction formula.

[Chem. 53]

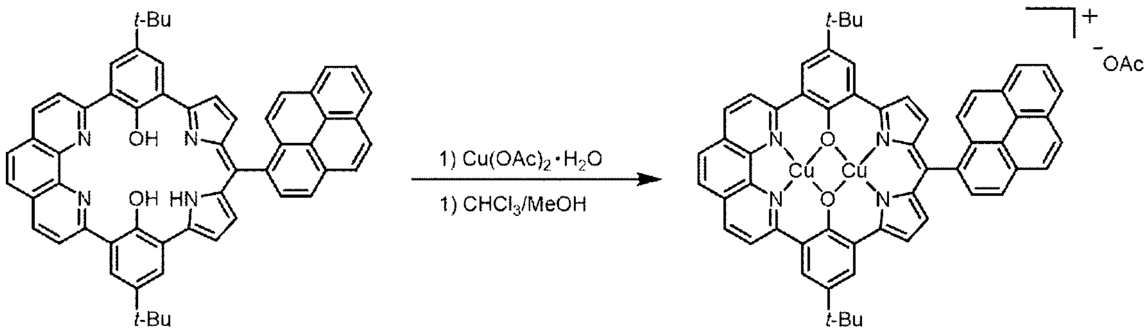


Compound 14 Oxygen-bridged dinuclear copper complex 6

The inside of a reaction vessel was set to a nitrogen gas atmosphere, and then, 29 mL of methanol which was previously degassed was added to and suspended in 0.61 g (3.06 mmol) of copper acetate monohydrate. 31 mL of chloroform was added thereto, and the temperature was raised to 50°C to obtain a copper acetate solution. The inside of another reaction vessel was set to a nitrogen gas atmosphere, and then, a suspension containing 1.0 g (1.22 mmol) of the compound 14 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 6. The reaction solution was cooled to room temperature and then filtered. The resulting crystals were washed with methanol and dried under reduced pressure to obtain an oxygen-bridged dinuclear copper complex 6 in an amount of 1.08 g and a yield of 88%. The identification data of the resulting oxygen-bridged dinuclear copper complex 6 (carbon dioxide reduction catalyst 6) is shown below. The results of ESI-MS measurement were confirmed as follows.

ESI-MS[M-OAc]$^+$:m/z=941.20

A copper interatomic distance of the oxygen-bridged dinuclear copper complex 6 was 2.7 (Å).

<Production of Carbon Dioxide Reduction Electrode>

A carbon dioxide reduction electrode of Example 7 was obtained by the same procedure as in Example 1, except that an oxygen-bridged dinuclear copper complex 6 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 7 was used instead of the carbon dioxide reduction electrode of Example 1.

<Production of Ethylene>

Ethylene was synthesized in the same procedure as in Example 2, except that the carbon dioxide reduction apparatus of Example 7 was used instead of the carbon dioxide reduction apparatus of Example 1.

<<Example 8>>

(Synthesis of Compound 15)

A compound 15 was synthesized according to the following reaction formula.

[Chem. 54]

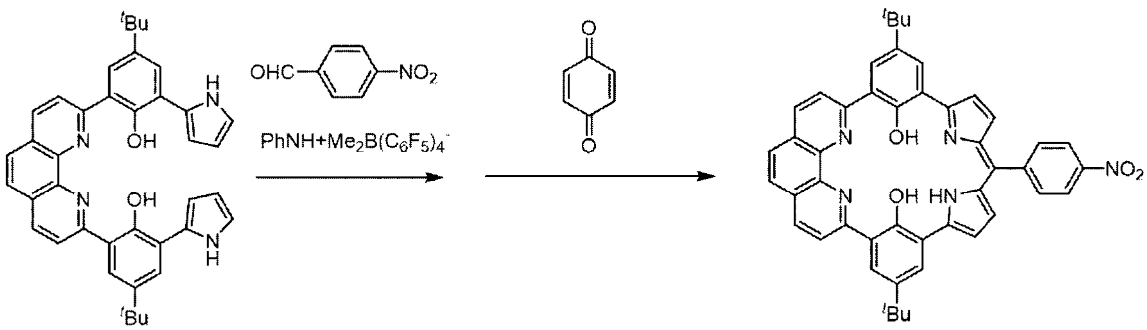


Compound 6 Compound 15

Under a nitrogen atmosphere, 92 ml of dehydrated toluene and 231 mg (0.29 mmol) of N,N-dimethylanilinium tetrakispentafluorophenyl borate were added, and the resulting solution was heated to 80°C with stirring using a rotor. A mixed solution of 1.37 g (9.36 mmol) of aldehyde, 5.0 g (8.24 mmol) of the compound 6, and 12 ml of toluene was added dropwise to the solution. After stirring for 3 hours, the resulting solution was allowed to cool, and the temperature of the reaction solution was gradually adjusted to room temperature.

A solution prepared by dissolving 0.98 g (9.06 mmol) of benzoquinone in 11 ml of THF was added dropwise to the reaction solution. After completion of the reaction was confirmed, the resulting reaction solution was filtered to obtain a target compound 15 in an amount of 5.59 g and a yield of 92%. The identification data of the resulting compound 15 is shown below. The results of ESI-MS measurement were confirmed as follows.

ESI-MS[M+H]$^+$:m/z=738.3

(Synthesis of Oxygen-Bridged Dinuclear Copper Complex 7)

An oxygen-bridged dinuclear copper complex 7 (that is, a carbon dioxide reduction catalyst 7) was synthesized according to the following reaction formula.

[Chem. 55]

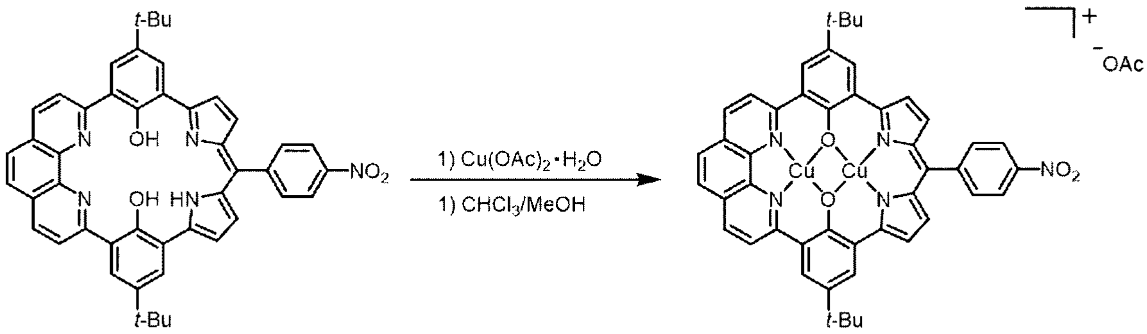


Compound 15

Oxygen-bridged dinuclear copper complex 7

The inside of a reaction vessel was set to a nitrogen gas atmosphere, and then, 29 mL of methanol which was previously degassed was added to and suspended in 0.68 g (3.39 mmol) of copper acetate monohydrate. 31 mL of chloroform was added thereto, and the temperature was raised to 50°C to obtain a copper acetate solution. The inside of another reaction vessel was set to a nitrogen gas atmosphere, and then, a suspension containing 1.0 g (1.36 mmol) of the compound 15 and 31 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 7. The reaction solution was cooled to room temperature and then filtered. The resulting crystals were washed with methanol and dried under reduced pressure to obtain an oxygen-bridged dinuclear copper complex 7 in an amount of 1.08 g and a yield of 88%. The identification data of the resulting oxygen-bridged dinuclear copper complex 7 (carbon dioxide reduction catalyst 7) is shown below. The results of ESI-MS measurement were confirmed as follows.
ESI-MS$[M-OAc]^+$:m/z=860.1

A copper interatomic distance of the oxygen-bridged dinuclear copper complex 7 was 2.76 (Å).

<Production of Carbon Dioxide Reduction Electrode>

A carbon dioxide reduction electrode of Example 8 was obtained by the same procedure as in Example 1, except that an oxygen-bridged dinuclear copper complex 7 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 8 was used instead of the carbon dioxide reduction electrode of Example 1.

<Production of Ethylene>

Ethylene was synthesized in the same procedure as in Example 2, except that the carbon dioxide reduction apparatus of Example 8 was used instead of the carbon dioxide reduction apparatus of Example 1.

<<Example 9>>

(Synthesis of Oxygen-Bridged Dinuclear Copper Complex 8)

An oxygen-bridged dinuclear copper complex 8 (that is, a carbon dioxide reduction catalyst 8) was synthesized according to the following reaction formula using the compound 17 synthesized by the method described in Tetrahedron, 1999, 55, 8377.

[Chem. 56]

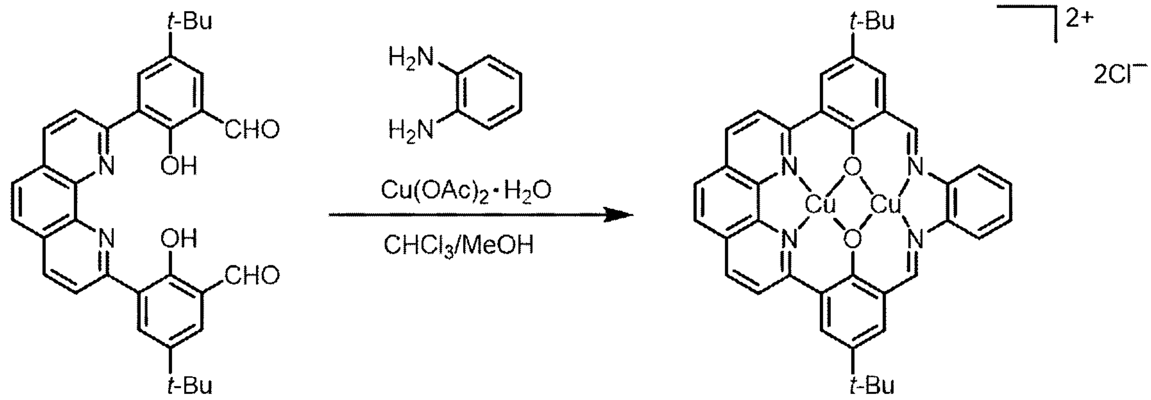


Compound 17

Oxygen-bridged dinuclear copper complex 8

The inside of a reaction vessel was set to a nitrogen gas atmosphere, and then, 2.9 mL of methanol which was previously degassed was added to and suspended in 0.21 g (1.03 mmol) of copper acetate monohydrate. 3.1 mL of chloroform was added thereto, and the temperature was raised to 50°C to obtain a copper acetate solution. The inside of another reaction vessel was set to a nitrogen gas atmosphere, and then, a suspension containing 0.25 g (0.41 mmol) of the compound 17 and 10 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 8. The reaction solution was cooled to room temperature and then filtered. The resulting crystals were washed with methanol and dried under reduced pressure to obtain an oxygen-bridged dinuclear copper complex 8 in an amount of 0.11 g and a yield of 33%. The identification data of the resulting oxygen-bridged dinuclear copper complex 8 (carbon dioxide reduction catalyst 8) is shown below. The results of ESI-MS measurement were confirmed as follows.

ESI-MS$[M-Cl]^+$:m/z=763.1

A copper interatomic distance of the oxygen-bridged dinuclear copper complex 8 was 2.75 (Å).

<Production of Carbon Dioxide Reduction Electrode>

A carbon dioxide reduction electrode of Example 9 was obtained by the same procedure as in Example 1, except that an oxygen-bridged dinuclear copper complex 8 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 9 was used instead of the carbon dioxide reduction electrode of Example 1.

<Production of Ethylene>

Ethylene was synthesized in the same procedure as in Example 2, except that the carbon dioxide reduction apparatus of Example 9 was used instead of the carbon dioxide reduction apparatus of Example 1.

<<Example 10>>

(Synthesis of Oxygen-Bridged Dinuclear Copper Complex 9)

An oxygen-bridged dinuclear copper complex 9 (that is, a carbon dioxide reduction catalyst 9) was synthesized according to the following reaction formula using the compound 18 synthesized by the method described in JP-A-2009-173627.

[Chem. 57]

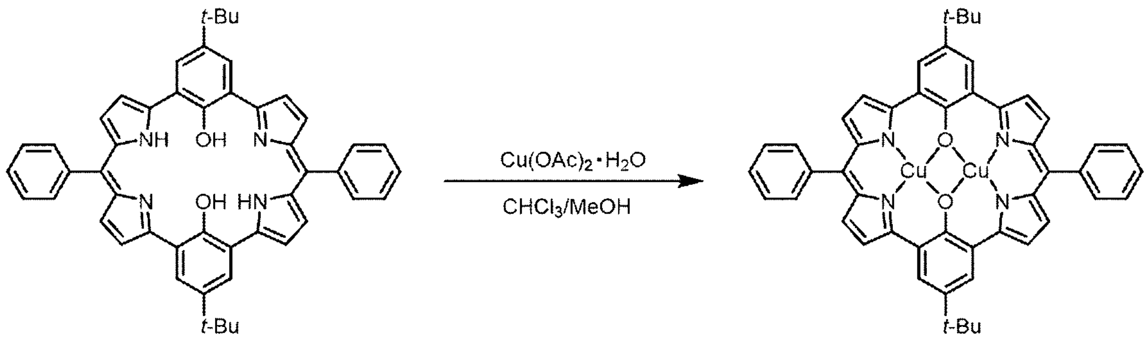


Compound 18

Oxygen-bridged dinuclear copper complex 9

The inside of a reaction vessel was set to a nitrogen gas atmosphere, and then, 12 mL of methanol which was previously degassed was added to and suspended in 0.68 g (3.41 mmol) of copper acetate monohydrate. 11 mL of chloroform was added thereto, and the temperature was raised to 50°C to obtain a copper acetate solution. The inside of another reaction vessel was set to a nitrogen gas atmosphere, and then, a suspension containing 1.0 g (1.36 mmol) of the compound 18 and 10 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 9. The reaction solution was cooled to room temperature and then filtered. The resulting crystals were washed with methanol and dried under reduced pressure to obtain an oxygen-bridged dinuclear copper complex 9 in an amount of 0.93 g and a yield of 80%. The identification data of the resulting oxygen-bridged dinuclear copper complex 9 (carbon dioxide reduction catalyst 10) is shown below. The results of ESI-MS measurement were confirmed as follows.
ESI-MS$[M+H]^+$:m/z=855.2

<Production of Carbon Dioxide Reduction Electrode>

A carbon dioxide reduction electrode of Example 10 was obtained by the same procedure as in Example 1, except that an oxygen-bridged dinuclear copper complex 9 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 10 was used instead of the carbon dioxide reduction electrode of Example 1.

<Production of Ethylene>

Ethylene was synthesized in the same procedure as in Example 2, except that the carbon dioxide reduction apparatus of Example 10 was used instead of the carbon dioxide reduction apparatus of Example 1.

<<Example 11>>

(Synthesis of Oxygen-Bridged Dinuclear Copper Complex 10)

An oxygen-bridged dinuclear copper complex 10 (that is, a carbon dioxide reduction catalyst 10) was synthesized according to the following reaction formula using the compound 17 synthesized by the method described in Tetrahedron, 1999, 55, 8377.

[Chem. 58]

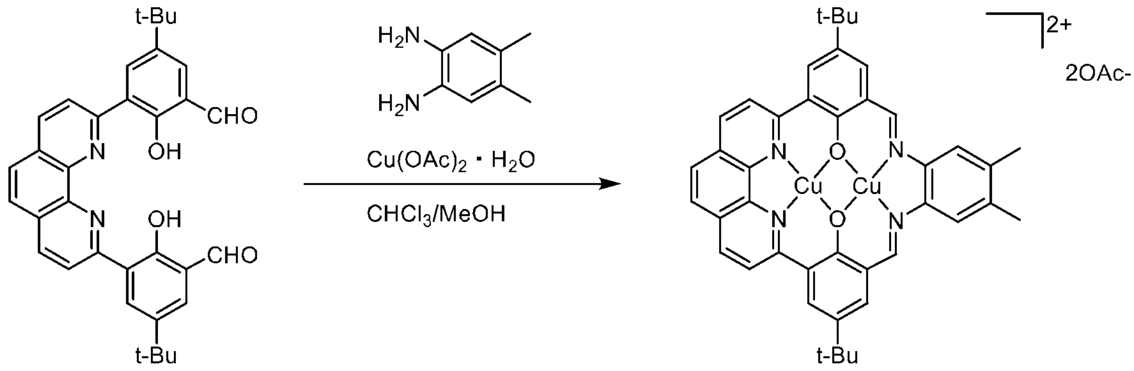


Compound 17

Oxygen-bridged dinuclear copper complex 10

The inside of a reaction vessel was set to a nitrogen gas atmosphere, and then, 5.0 mL of methanol which was previously degassed was added to and suspended in 0.08 g (0.38 mmol) of copper acetate monohydrate. 5.0 mL of chloroform was added thereto, and the temperature was raised to 50°C to obtain a copper acetate solution. The inside of another reaction vessel was set to a nitrogen gas atmosphere, and then, a suspension containing 0.1 g (0.19 mmol) of the compound 17, 0.03 g (0.19 mmol) of 4,5-dimethyl-1,2-phenylenediamine, and 10 mL of chloroform was prepared. The suspension was added dropwise to the copper acetate solution, and then, the resulting suspension was heated to 65°C and stirred for 3 hours while being refluxed to obtain a reaction solution containing an oxygen-bridged dinuclear copper complex 9. The reaction solution was cooled to room temperature, and then, the solvent was distilled off. The resulting solid was obtained by filtration while being washed with acetone, and dried under reduced pressure to obtain an oxygen-bridged dinuclear copper complex 10 in an amount of 0.13 g and a yield of 85%. The identification data of the resulting oxygen-bridged dinuclear copper complex 10 (carbon dioxide reduction catalyst 10) is shown below. The results of ESI-MS measurement were confirmed as follows.
ESI-MS$[M-OAc]^+$:m/z=815.2

A copper interatomic distance of the oxygen-bridged dinuclear copper complex 10 was 2.71 (Å).

<Production of Carbon Dioxide Reduction Electrode>

A carbon dioxide reduction electrode of Example 11 was obtained by the same procedure as in Example 1, except that an oxygen-bridged dinuclear copper complex 10 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 11 was used instead of the carbon dioxide reduction electrode of Example 1.

<Production of Ethylene>

Ethylene was synthesized in the same procedure as in Example 2, except that the carbon dioxide reduction apparatus of Example 11 was used instead of the carbon dioxide reduction apparatus of Example 1.

<<Example 12>>

(Synthesis of Oxygen-Bridged Dinuclear Copper Complex 11)

An oxygen-bridged dinuclear copper complex 11 (that is, a carbon dioxide reduction catalyst 11) was synthesized according to the following reaction formula using the compound 17 synthesized by the method described in Tetrahedron, 1999, 55, 8377.

[Chem. 59]

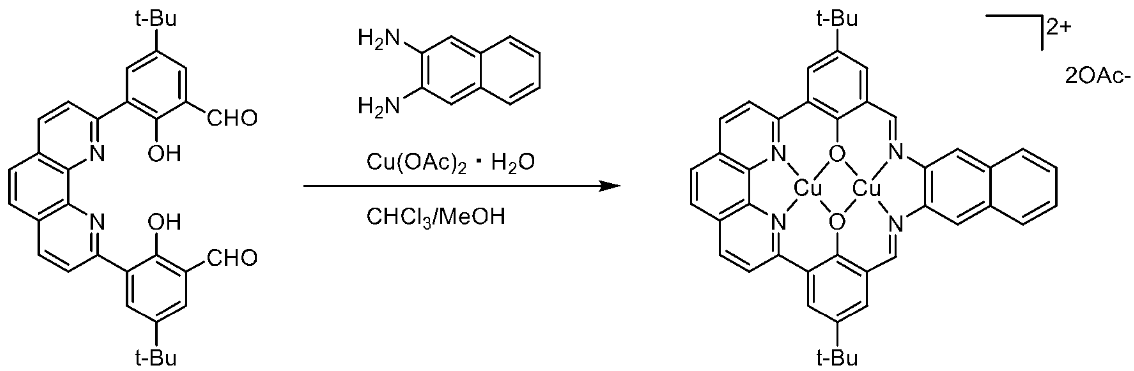


Compound 17

Oxygen-bridged dinuclear copper complex 11

The inside of a reaction vessel was set to a nitrogen gas atmosphere, and then, 5.0 mL of methanol which was previously degassed was added to and suspended in 0.08 g (0.38 mmol) of copper acetate monohydrate. 5.0 mL of chloroform was added thereto, and the temperature was raised to 50°C to obtain a copper acetate solution. The inside of another reaction vessel was set to a nitrogen gas atmosphere, and then, a suspension containing 0.1 g (0.19 mmol) of the compound 17, 0.03 g (0.19 mmol) of 2,3-naphthalenediamine, and 10 mL of chloroform was prepared. The suspension was added dropwise to the copper acetate solution, and then, the resulting suspension was heated to 65°C and stirred for 3 hours while being refluxed to obtain a reaction solution containing an oxygen-bridged dinuclear copper complex 10. The reaction solution was cooled to room temperature, and then, the solvent was distilled off. The resulting solid was obtained by filtration while being washed with acetone, and dried under reduced pressure to obtain an oxygen-bridged dinuclear copper complex 11 in an amount of 0.15 g and a yield of 95%. The identification data of the resulting oxygen-bridged dinuclear copper complex 11 (carbon dioxide reduction catalyst 11) is shown below. The results of ESI-MS measurement were confirmed as follows.

ESI-MS$[M-OAc]^+$:m/z=837.2

A copper interatomic distance of the oxygen-bridged dinuclear copper complex 11 was 2.71 (Å).

<Production of Carbon Dioxide Reduction Electrode>

A carbon dioxide reduction electrode of Example 12 was obtained by the same procedure as in Example 1, except that an oxygen-bridged dinuclear copper complex 11 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 12 was used instead of the carbon dioxide reduction electrode of Example 1.

<Production of Ethylene>

Ethylene was synthesized in the same procedure as in Example 2, except that the carbon dioxide reduction apparatus of Example 12 was used instead of the carbon dioxide reduction apparatus of Example 1.

<<Example 13>>

(Synthesis of Oxygen-Bridged Dinuclear Copper Complex 12)

An oxygen-bridged dinuclear copper complex 12 (that is, a carbon dioxide reduction catalyst 12) was synthesized according to the following reaction formula using the compound 17 synthesized by the method described in Tetrahedron, 1999, 55, 8377.

[Chem. 60]

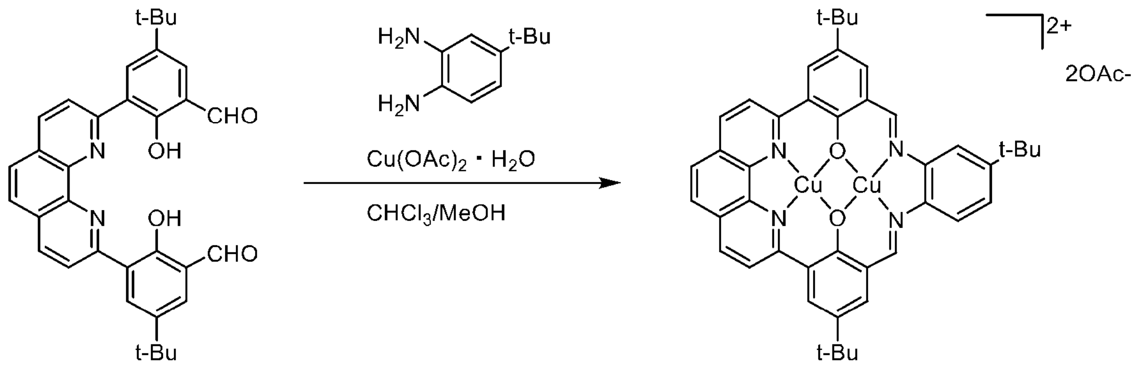


Compound 17

Oxygen-bridged dinuclear copper complex 12

The inside of a reaction vessel was set to a nitrogen gas atmosphere, and then, 5.0 mL of methanol which was previously degassed was added to and suspended in 0.08 g (0.38 mmol) of copper acetate monohydrate. 5.0 mL of chloroform was added thereto, and the temperature was raised to 50°C to obtain a copper acetate solution. The inside of another reaction vessel was set to a nitrogen gas atmosphere, and then, a suspension containing 0.1 g (0.19 mmol) of the compound 17, 0.03 g (0.19 mmol) of 4-tert-butylbenzene-1,2-diamine, and 10 mL of chloroform was prepared. The suspension was added dropwise to the copper acetate solution, and then, the resulting suspension was heated to 65°C and stirred for 3 hours while being refluxed to obtain a reaction solution containing an oxygen-bridged dinuclear copper complex 11. The reaction solution was cooled to room temperature, and then, the solvent was distilled off. The resulting solid was obtained by filtration while being washed with acetone, and dried under reduced pressure to obtain an oxygen-bridged dinuclear copper complex 12 in an amount of 0.15 g and a yield of 89%. The identification data of the resulting oxygen-bridged dinuclear copper complex 12 (carbon dioxide reduction catalyst 12) is shown below. The results of ESI-MS measurement were confirmed as follows.
ESI-MS$[M+H]^+$:m/z=903.3

A copper interatomic distance of the oxygen-bridged dinuclear copper complex 12 was 2.71 (Å).

<Production of Carbon Dioxide Reduction Electrode>

A carbon dioxide reduction electrode of Example 12 was obtained by the same procedure as in Example 1, except that an oxygen-bridged dinuclear copper complex 12 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 12 was used instead of the carbon dioxide reduction electrode of Example 1.

<Production of Ethylene>

Ethylene was synthesized in the same procedure as in Example 2, except that the carbon dioxide reduction apparatus of Example 12 was used instead of the carbon dioxide reduction apparatus of Example 1.

<<Example 14>>

(Synthesis of Compound 20)

A compound 20 was synthesized according to the following reaction formula using the compound 19 synthesized by the method described in WO 2017-073467 A.

[Chem. 61]

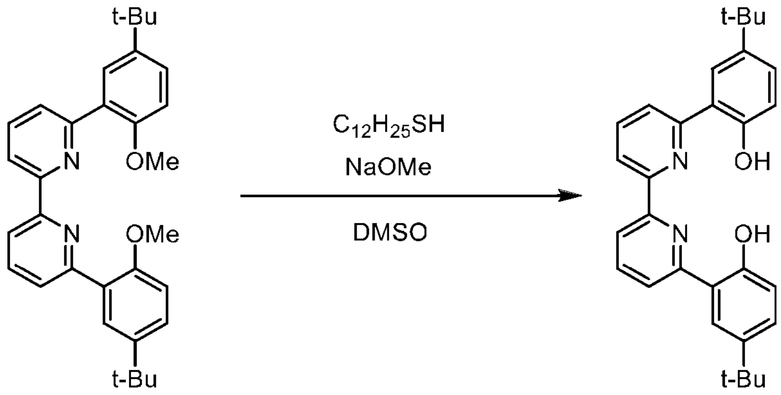


Compound 19 Compound 20

The inside of a reaction vessel was set to a nitrogen gas atmosphere, and then, 28 mL of DMSO which was previously degassed was added to 2.5 g (5.2 mmol) of the compound 19 to dissolve the compound. 5.2 g (26.0 mmol) of dodecanethiol and 6.0 g (31.2 mmol) of a sodium methoxidemethanol solution were added thereto, and the resulting solution was heated to 80°C and stirred for 8 hours. The reaction solution was allowed to cool to room temperature, neutralized with 1 M hydrochloric acid, and then, extracted with chloroform. An organic layer was dehydrated with magnesium sulfate, and concentrated with a rotary evaporator to distill off the solvent. Normal heptane was added to the resulting crude product, and a precipitated solid was obtained by filtration while being washed with normal heptane and dried under reduced pressure to obtain a compound 20 in an amount of 1.0 g and a yield of 42%. The identification data of the resulting compound 20 is shown below. The results of 1H NMR measurement were confirmed as follows.
1H NMR($CDCl_3$,400MHz) :13.86(s, 2H), 8.09(m,6H), 7.90(d,2H), 7.42(dd, 2H), 6.99(d, 2H), 1,39(s, 18H)

(Synthesis of Compound 21)

A compound 21 was synthesized according to the following reaction formula.

[Chem. 62]

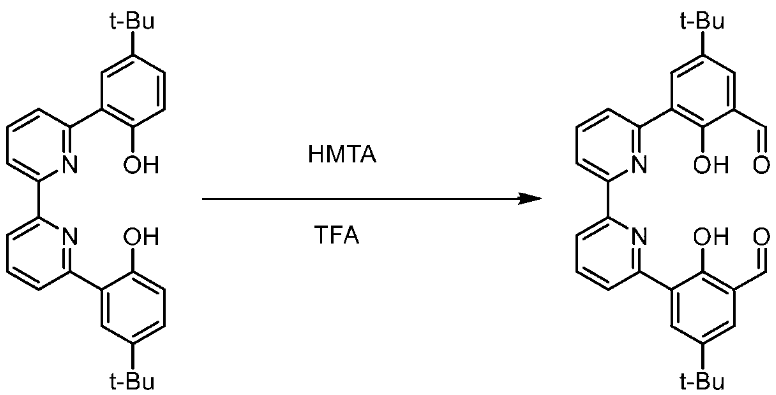


Compound 20 Compound 21

The inside of a reaction vessel was set to a nitrogen gas atmosphere, 0.6 g (1.3 mmol) of the compound 20, 0.45 g (3.18 mmol) of hexamethylenetetramine (HMTA) and 6.6 mL of trifluoroacetic acid (TFA) were added thereto, the temperature was raised to 100°C while stirring, and the resulting solution was kept warm for 8 hours. The reaction solution was cooled to 60°C, 20 mL of a 30% sulfuric acid aqueous solution and 20 mL of chloroform were added, and the resulting solution was stirred for 1 hour while being kept warm. An organic layer was extracted by liquid separation, dried with magnesium sulfate, and concentrated by a rotary evaporator to distill off the solvent. Methanol was added to the resulting crude product, and a precipitated solid was obtained by filtration while being washed with methanol and dried under reduced pressure to obtain a compound 21 in an amount of 0.32 g and a yield of 47%. The identification data of the resulting compound 21 is shown below. The results of 1H NMR measurement were confirmed as follows.
1H NMR($CDCl_3$, 400MHz) :10.46(s, 2H) , 8.13 (m, 6H) , 8.02(m,2H), 7.83(d, 2H), 1,31(s, 18H)

(Synthesis of Oxygen-Bridged Dinuclear Copper Complex 13)

An oxygen-bridged dinuclear copper complex 13 (that is, a carbon dioxide reduction catalyst 13) was synthesized according to the following reaction formula using the compound 21.

[Chem. 63]

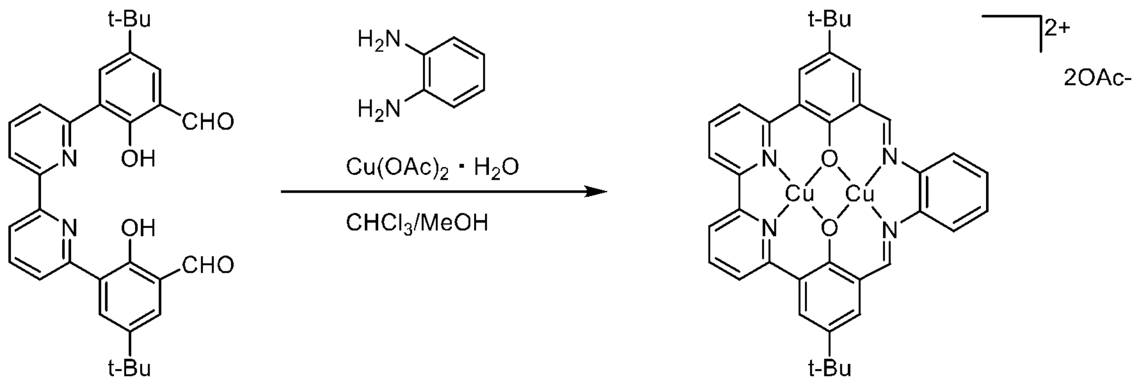


Compound 21

Oxygen-bridged dinuclear copper complex 13

The inside of a reaction vessel was set to a nitrogen gas atmosphere, and then, 5.0 mL of methanol which was previously degassed was added to and suspended in 0.08 g (0.39 mmol) of copper acetate monohydrate. 5.0 mL of chloroform was added thereto, and the temperature was raised to 50°C to obtain a copper acetate solution. The inside of another reaction vessel was set to a nitrogen gas atmosphere, and then, a suspension containing 0.1 g (0.197 mmol) of the compound 21, 0.02 g (0.197 mmol) of 1,2-phenylenediamine, and 10 mL of chloroform was prepared. The suspension was added dropwise to the copper acetate solution, and then, the resulting suspension was heated to 65°C and stirred for 3 hours while being refluxed to obtain a reaction solution containing an oxygen-bridged dinuclear copper complex 12. The reaction solution was cooled to room temperature, and then, the solvent was distilled off. The resulting solid was obtained by filtration while being washed with acetone, and dried under reduced pressure to obtain an oxygen-bridged dinuclear copper complex 13 in an amount of 0.14 g and a yield of 84%. The identification data of the resulting oxygen-bridged dinuclear copper complex 13 (carbon dioxide reduction catalyst 13) is shown below. The results of ESI-MS measurement were confirmed as follows.

ESI-MS$[M-OAc]^+$:m/z=765.2

A copper interatomic distance of the oxygen-bridged dinuclear copper complex 13 was 2.75 (Å).

<Production of Carbon Dioxide Reduction Electrode>

A carbon dioxide reduction electrode of Example 14 was obtained by the same procedure as in Example 1, except that an oxygen-bridged dinuclear copper complex 13 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 14 was used instead of the carbon dioxide reduction electrode of Example 1.

<Production of Ethylene>

Ethylene was synthesized in the same procedure as in Example 2, except that the carbon dioxide reduction apparatus of Example 14 was used instead of the carbon dioxide reduction apparatus of Example 1.

<Production of Carbon Dioxide Reduction Electrode>

A carbon dioxide reduction electrode of Example 14 was obtained by the same procedure as in Example 1, except that an oxygen-bridged dinuclear copper complex 13 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 14 was used instead of the carbon dioxide reduction electrode of Example 1.

<Production of Ethylene>

Ethylene was synthesized in the same procedure as in Example 2, except that the carbon dioxide reduction apparatus of Example 14 was used instead of the carbon dioxide reduction apparatus of Example 1.

<<Example 15>>

(Synthesis of Compound 22)

A compound 22 was synthesized according to the following reaction formula.

[Chem. 64]

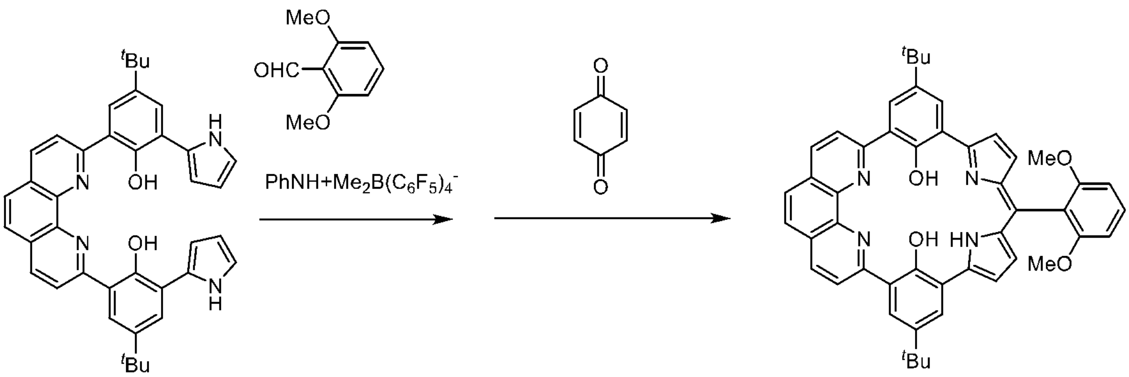


Compound 6 Compound 22

Under a nitrogen atmosphere, 37 ml of dehydrated toluene and 264 mg (0.33 mmol) of N,N-dimethylanilinium tetrakispentafluorophenyl borate were added, and the resulting solution was heated to 80°C with stirring using a rotor. A mixed solution of 0.60 g (3.63 mmol) of aldehyde, 2.0 g (3.30 mmol) of the compound 6, and 5 ml of toluene was added dropwise thereto. After stirring for 3 hours, the resulting solution was allowed to cool, and the temperature of the reaction solution was gradually adjusted to room temperature.

A solution prepared by dissolving 0.39 g (3.63 mmol) of benzoquinone in 5 ml of THF was added dropwise to the reaction solution. After completion of the reaction was confirmed, the resulting reaction solution was filtered to obtain a target compound 23 in an amount of 2.27 g and a yield of 91%. The identification data of the resulting compound 22 is shown below. The results of ESI-MS measurement were confirmed as follows.
ESI-MS$[M+H]^+$:m/z=753.3

(Synthesis of Oxygen-Bridged Dinuclear Copper Complex 14)

An oxygen-bridged dinuclear copper complex 14 (that is, a carbon dioxide reduction catalyst 14) was synthesized according to the following reaction formula.

[Chem. 65]

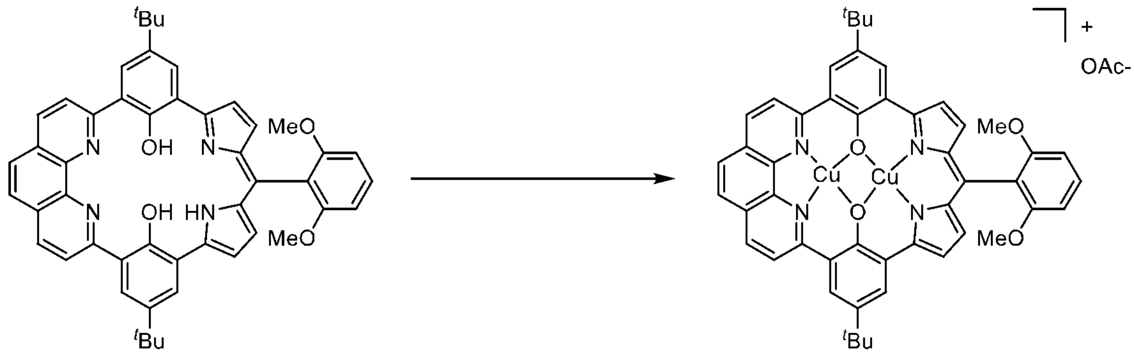

Compound 23

Oxygen-bridged dinuclear copper complex 14

The inside of a reaction vessel was set to a nitrogen gas atmosphere, and then, 6 mL of methanol which was previously degassed was added to and suspended in 0.29 g (1.46 mmol) of copper acetate monohydrate. 6 mL of chloroform was added thereto, and the temperature was raised to 50°C to obtain a copper acetate solution. The inside of another reaction vessel was set to a nitrogen gas atmosphere, and then, a suspension containing 0.5 g (0.66 mmol) of the compound 23 and 10 mL of chloroform was prepared. The suspension was added dropwise to the copper acetate solution, and then, the resulting suspension was heated to 55°C and stirred for 1 hour while being refluxed to obtain a reaction solution containing an oxygen-bridged dinuclear copper complex 5. The reaction solution was cooled to room temperature and then filtered. The resulting crystals were dried under reduced pressure to obtain an oxygen-bridged dinuclear copper complex 14 in an amount of 0.4 g and a yield of 64%. The identification data of the resulting oxygen-bridged dinuclear copper complex 14 (carbon dioxide reduction catalyst 14) is shown below. The results of ESI-MS measurement were confirmed as follows.

ESI-MS[M-OAc]$^+$:m/z=875.2

A copper interatomic distance of the oxygen-bridged dinuclear copper complex 14 was 2.67 (Å).

<Production of Carbon Dioxide Reduction Electrode>

A carbon dioxide reduction electrode of Example 15 was obtained by the same procedure as in Example 1, except that an oxygen-bridged dinuclear copper complex 14 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 15 was used instead of the carbon dioxide reduction electrode of Example 1.

<Production of Ethylene>

Ethylene was synthesized in the same procedure as in Example 2, except that the carbon dioxide reduction apparatus of Example 15 was used instead of the carbon dioxide reduction apparatus of Example 1.

<<Example 16>>

(Synthesis of Compound 23)

A compound 23 was synthesized according to the following reaction formula.

[Chem. 66]

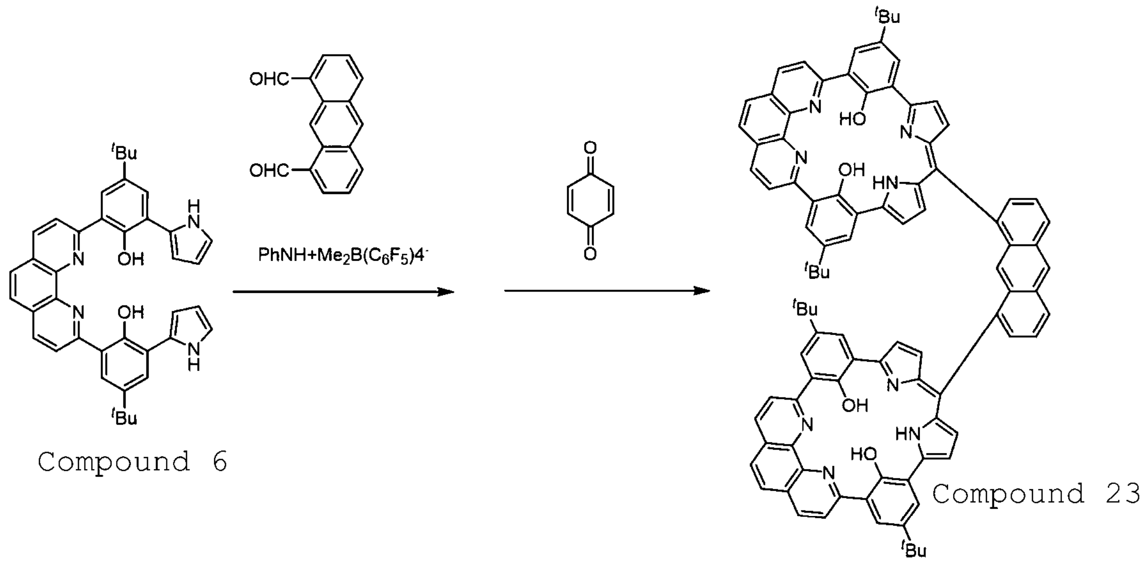


Compound 6

Compound 23

Under a nitrogen atmosphere, 10 ml of dehydrated chloroform and 66 mg (0.08 mmol) of N,N-dimethylanilinium tetrakispentafluorophenyl borate were added, and the resulting solution was heated to 80°C with stirring using a rotor. A mixed solution of 0.09 g (0.37 mmol) of aldehyde, 0.5 g (0.82 mmol) of the compound 6, and 12 ml of chloroform was added dropwise to the solution. After stirring for 3 hours, the resulting solution was allowed to cool, and the temperature of the reaction solution was gradually adjusted to room temperature.

A solution prepared by dissolving 0.1 g (0.906 mmol) of benzoquinone in 1 ml of THF was added dropwise to the reaction solution. After completion of the reaction was confirmed, the resulting reaction solution was filtered to obtain a target compound 23 in an amount of 0.51 g and a yield of 99%. The identification data of the resulting compound 23 is shown below. The results of ESI-MS measurement were confirmed as follows.
ESI-MS$[M+H]^+$:m/z=1407.2

(Synthesis of Oxygen-Bridged Tetranuclear Copper Complex 1)

An oxygen-bridged tetranuclear copper complex 1 (that is, a carbon dioxide reduction catalyst 15) was synthesized according to the following reaction formula.

[Chem. 67]

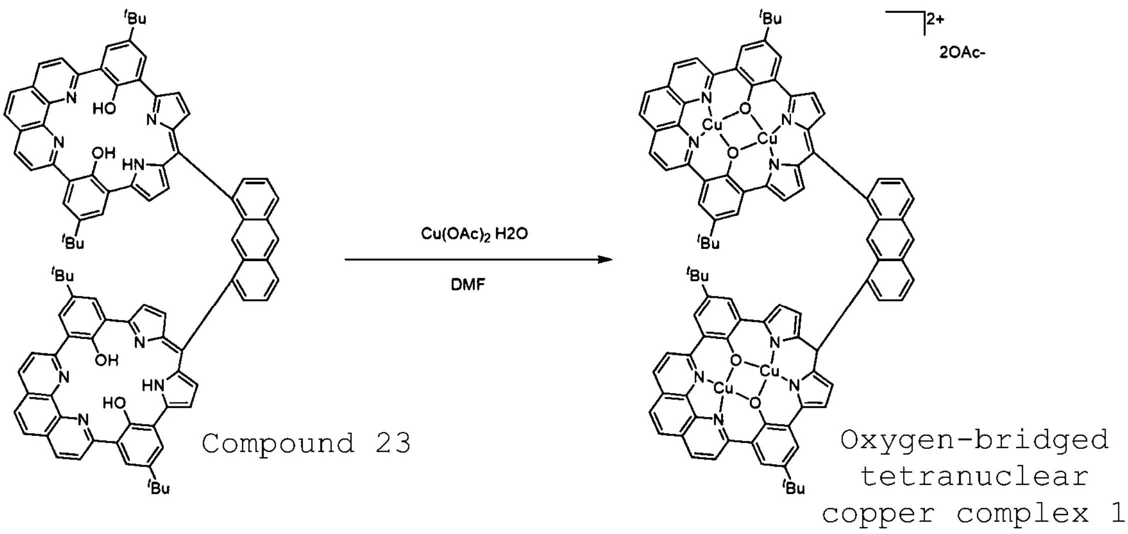


Compound 23

Oxygen-bridged tetranuclear copper complex 1

The inside of a reaction vessel was set to a nitrogen gas atmosphere, 5 mL of dimethylformamide which was

previously degassed was added to 0.2 g (0.142 mmol) of the compound 23 and 0.13 g (0.64 mmol) of copper acetate monohydrate, the temperature was raised to 100°C, and then, the resulting solution was stirred for 3 hours. Thereafter, the reaction solution was heated to 130°C and stirred for 6 hours. The reaction solution was cooled to room temperature, and then, chloroform and water were added to separate the reaction solution. An organic layer was dried with sodium sulfate and then filtered, heptane was added to the filtrate to precipitate an oxygen-bridged tetranuclear copper complex 1, and the precipitated oxygen-bridged tetranuclear copper complex 1 was collected by filtration. The amount was 0.25 g and a yield was 99%. The identification data of the resulting oxygen-bridged tetranuclear copper complex 1 (carbon dioxide reduction catalyst 15) is shown below. The results of ESI-MS measurement were confirmed as follows.
ESI-MS$[M\text{-}OAc]^+$:m/z=1654.3

A copper interatomic distance of the oxygen-bridged tetranuclear copper complex 1 was 2.70 (Å) and 2.69 (Å).

<Production of Carbon Dioxide Reduction Electrode>

A carbon dioxide reduction electrode of Example 16 was obtained by the same procedure as in Example 1, except that the oxygen-bridged tetranuclear copper complex 1 was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Example 16 was used instead of the carbon dioxide reduction electrode of Example 1.

<Production of Ethylene>

Ethylene was synthesized in the same procedure as in Example 2, except that the carbon dioxide reduction apparatus of Example 16 was used instead of the carbon dioxide reduction apparatus of Example 1.

<<Comparative Example 1>>

<Synthesis of Carbon Dioxide Reduction Catalyst>

A halogen-bridged dinuclear copper complex 1 was synthesized by the method described in JP-A-2021-109157 according to the following reaction formula. In the following reaction formula, "Ph" means a phenyl group.

[Chem. 68]

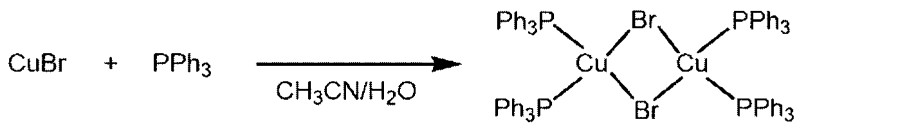


Halogen-bridged dinuclear copper complex 1

<Production of Carbon Dioxide Reduction Electrode>

In a reaction vessel, 105.12 mg (0.079 mmol) of a halogen-bridged dinuclear copper complex 1 was weighed, 52 mL of $H_2O$ and 33 mL of methanol were added, and stirring and ultrasonic irradiation were repeated to obtain a dispersion. In another reaction vessel, 200 mg of carbon black (Ketjen Black EC600JD manufactured by LION SPECIALTY CHEMICALS CO., Ltd.) as a conductive material was weighed, and the dispersion was added dropwise thereto to newly obtain a dispersion. By irradiating the dispersion with ultrasonic waves for 15 minutes, the conductive material on which the carbon dioxide reduction catalyst was supported was uniformly dispersed to obtain a suspension having a content of copper atoms of 5 mass% with respect to the mass of carbon black. In a subsequent operation, a carbon dioxide reduction electrode of Comparative Example 1 was obtained by the same procedure as in Example 1.

A copper interatomic distance of the halogen-bridged dinuclear copper complex 1 was 2.85 (Å).

<Manufacture of Carbon Dioxide Reduction Apparatus>

A carbon dioxide reduction apparatus was obtained in the same procedure as in Example 1, except that the carbon dioxide reduction electrode of Comparative Example 1 was used instead of the carbon dioxide reduction electrode of Example 1.

<Production of Ethylene>

Ethylene was synthesized in the same procedure as in Example 1, except that the carbon dioxide reduction apparatus of Comparative Example 1 was used instead of the carbon dioxide reduction apparatus of Example 1.

<<Comparative Example 2>>

<Preparation of Carbon Dioxide Reduction Catalyst>

212.83 mg (0.3147 mmmol) of copper tetraphenylporphyrin (manufactured by Tokyo Chemical Industry Co., Ltd.) was prepared and used as a carbon dioxide reduction catalyst of Comparative Example 2.

<Production of Carbon Dioxide Reduction Electrode>

A carbon dioxide reduction electrode of Comparative Example 2 was obtained by the same procedure as in Example 1, except that copper tetraphenylporphyrin was used instead of the oxygen-bridged dinuclear copper complex 1.

<Manufacture of Carbon Dioxide Reduction Apparatus>

A carbon dioxide reduction apparatus was obtained in the same procedure as in Example 1, except that the carbon dioxide reduction electrode of Comparative Example 2 was used instead of the carbon dioxide reduction electrode of Example 1.

<Production of Ethylene>

Ethylene was synthesized in the same procedure as in Example 1, except that the carbon dioxide reduction apparatus of Comparative Example 2 was used instead of the carbon dioxide reduction apparatus of Example 1.

<<Comparative Example 3>>

<Production of Ethylene>

Ethylene was synthesized in the same procedure as in Example 2, except that the carbon dioxide reduction apparatus of Comparative Example 1 was used instead of the carbon dioxide reduction apparatus of Example 1.

<<Comparative Example 4>>

<Production of Ethylene>

Ethylene was synthesized in the same procedure as in Example 2, except that the carbon dioxide reduction apparatus of Comparative Example 2 was used instead of the carbon dioxide reduction apparatus of Example 1.

<<Evaluation>>

<Calculation of Copper Interatomic Distance>

The copper interatomic distance was calculated according to the method described in "· Method for Calculating Copper Interatomic Distance" described above.

Note that since copper tetraphenylporphyrin is a mononuclear copper complex, the copper interatomic distance is not calculated.

Here, it is considered that the oxygen-bridged dinuclear copper complexes 1 to 7 and 14 have acetate anions as counter anions at the time of synthesis, but the copper centers are each reduced to monovalent during the carbon dioxide

reduction reaction, and the acetate anions are eliminated in a monoanion state. Therefore, for the oxygen-bridged dinuclear copper complexes 1 to 7 and 14, the copper interatomic distance in the monoanion state was calculated.

In addition, it is considered that the oxygen-bridged dinuclear copper complex 8 has two chlorine anions as counter anions at the time of synthesis, but the copper centers are each reduced to monovalent during the carbon dioxide reduction reaction, and the chlorine anions are eliminated in a neutral state. Therefore, for the oxygen-bridged dinuclear copper complex 8, the copper interatomic distance in the neutral state was calculated.

In addition, it is considered that the oxygen-bridged dinuclear copper complexes 10 to 13 have two acetate anions as counter anions at the time of synthesis, but the copper centers are each reduced to monovalent during the carbon dioxide reduction reaction, and the acetate anions are eliminated in a neutral state. Therefore, for the oxygen-bridged dinuclear copper complexes 10 to 13, the copper interatomic distance in the neutral state was calculated.

In addition, it is considered that the oxygen-bridged tetranuclear copper complex 1 has two acetate anions as counter anions at the time of synthesis, but the copper centers are each reduced to monovalent during the carbon dioxide reduction reaction, and the acetate anions are eliminated in a dianion state. Therefore, for the oxygen-bridged tetranuclear copper complex 1, the copper interatomic distance in the dianion state was calculated.

In addition, the copper centers of the halogen-bridged dinuclear copper complex 1 are monovalent, and are considered to be in a neutral state. Therefore, for the halogen-bridged dinuclear copper complex 1, the copper interatomic distance in the neutral state was calculated.

<Evaluation of Ethylene Selectivity>

Ethylene selectivity was evaluated by the following procedure using the carbon dioxide reduction apparatus obtained in each example.

Between 100 seconds and 200 seconds after the start of voltage application, 50 μL of outlet gas (gas flowing out from the reaction tank 16 in the direction of arrow B) was collected using a gas-tight syringe, and the product contained in the gas was quantitatively analyzed by a gas chromatograph apparatus (GC-2010/FID detector manufactured by Shimadzu Corporation, and GC-2014/TCD detector manufactured by Shimadzu Corporation). Faraday efficiency of each of the various products was calculated by the ratio of the observed charge amount used to generate each of the various products among all the charge amounts used in the reaction. The value is obtained by converting the current flowing through the reaction area of the carbon dioxide reduction electrode 10 as a current value per unit area ($mA/cm^2$) .

The conversion from the Ag/AgCl reference potential to the RHE reference potential was performed based on the following equation.

$$\text{Formula: } E(RHE)=E(Ag/AgCl)+0.198V+0.059\times pH$$

A higher value of the Faraday efficiency of ethylene indicates that ethylene is produced selectively, and means that ethylene selectivity is high.

[Table 1]

| | Carbon dioxide reduction catalyst | Applied potential vs RHE (V) | Faraday efficiency (%) | | | Current density ($mA/cm^2$) |
|---|---|---|---|---|---|---|
| | | | CO | $CH_4$ | $C_2H_4$ | |
| Example 1 | Oxygen-bridged dinuclear copper complex 1 | -2.2 | 27 | 23 | 44 | 265 |
| Example 2 | Oxygen-bridged dinuclear copper complex 1 | -3.2 | 23 | 21 | 54 | 494 |
| Example 3 | Oxygen-bridged dinuclear copper complex 2 | -3.2 | 17 | 27 | 40 | 480 |
| Example 4 | Oxygen-bridged dinuclear copper complex 3 | -3.2 | 12 | 12 | 41 | 475 |
| Example 5 | Oxygen-bridged dinuclear copper complex 4 | -3.2 | 18 | 18 | 55 | 467 |
| Example 6 | Oxygen-bridged dinuclear copper complex 5 | -3.2 | 17 | 31 | 46 | 463 |

(continued)

| | Carbon dioxide reduction catalyst | Applied potential vs RHE (V) | Faraday efficiency (%) | | | Current density (mA/ $cm^2$) |
|---|---|---|---|---|---|---|
| | | | CO | $CH_4$ | $C_2H_4$ | |
| Example 7 | Oxygen-bridged dinuclear copper complex 6 | -3.2 | 17 | 19 | 50 | 467 |
| Example 8 | Oxygen-bridged dinuclear copper complex 7 | -3.2 | 27 | 13 | 53 | 419 |
| Example 9 | Oxygen-bridged dinuclear copper complex 8 | -3.2 | 24 | 22 | 46 | 487 |
| Example 10 | Oxygen-bridged dinuclear copper complex 9 | -3.2 | 22 | 18 | 38 | 484 |
| Example 11 | Oxygen-bridged dinuclear copper complex 10 | -3.2 | 20 | 15 | 38 | 463 |
| Example 12 | Oxygen-bridged dinuclear copper complex 11 | -3.2 | 20 | 16 | 40 | 474 |
| Example 13 | Oxygen-bridged dinuclear copper complex 12 | -3.2 | 26 | 12 | 43 | 467 |
| Example 14 | Oxygen-bridged dinuclear copper complex 13 | -3.2 | 25 | 14 | 44 | 442 |
| Example 15 | Oxygen-bridged dinuclear copper complex 14 | -3.2 | 11 | 12 | 53 | 467 |
| Example 16 | Oxygen-bridged tetranuclear copper complex 1 | -3.2 | 34 | 17 | 39 | 382 |
| Comparative Example 1 | Halogen-bridged dinuclear copper complex 1 | -2.2 | 20 | 39 | 21 | 248 |
| Comparative Example 2 | Copper tetraphenylporphyrin | -2.2 | 10 | 26 | 10 | 224 |
| Comparative Example 3 | Halogen-bridged dinuclear copper complex 1 | -3.2 | 10 | 26 | 35 | 470 |
| Comparative Example 4 | Copper tetraphenylporphyrin | -3.2 | 19 | 42 | 24 | 466 |

From the above results, it is found that the ethylene production method of the present example has higher ethylene selectivity than that of Comparative Examples.

DESCRIPTION OF REFERENCE SIGNS

1 Layer containing conductive material on which carbon dioxide reduction catalyst is supported

2 Support

10 Carbon dioxide reduction electrode

11 Oxidation electrode

12 Membrane

13 Electrolyte solution

14 Power supply

15 Electrolytic bath

16 Reaction tank

100 Carbon dioxide reduction apparatus

## Claims

1. An ethylene production method comprising a step of reacting carbon dioxide with water in the presence of a carbon dioxide reduction catalyst that is a multinuclear copper complex and has a copper interatomic distance of 2.8 Å or less as determined by density functional theory.

2. The ethylene production method according to claim 1, wherein the carbon dioxide reduction catalyst is a multinuclear copper complex in which a copper atom and two oxygen atoms are coordinate-bonded.

3. An ethylene production method comprising a step of reacting carbon dioxide with water in the presence of a carbon dioxide reduction catalyst represented by the following Formula (1):

[Chem. 1]

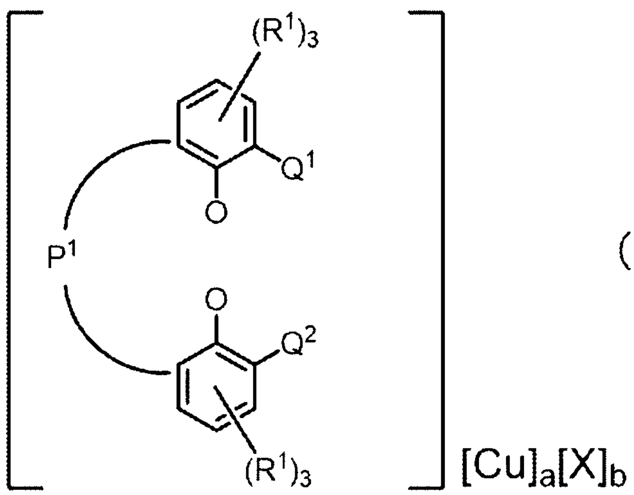

(1)

in Formula (1),
$R^1$ represents a hydrogen atom or a substituent, a plurality of $R^1$ may be the same as or different from each other, two adjacent $R^1$ may be bonded to each other to form a ring, $P^1$ represents a divalent group containing one or more aromatic rings, $Q^1$ and $Q^2$ represent a monovalent group containing one or more aromatic rings, $Q^1$ and $Q^2$ may be bonded to each other to form a ring structure, a is an integer of 2 or more and 4 or less, X is a counter ion or a neutral molecule, b is an integer of 0 or more, when a plurality of X are present, the plurality of X may be the same as or different from each other, and O is an oxygen atom and is bonded to at least one copper atom.

4. The ethylene production method according to claim 3, wherein $P^1$ is a divalent group represented by the following Formula ($P^a$) or the following Formula ($P^b$):

[Chem. 2]

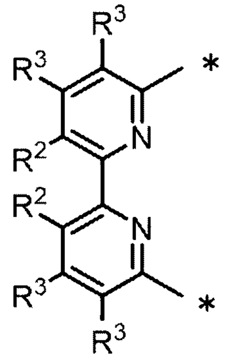

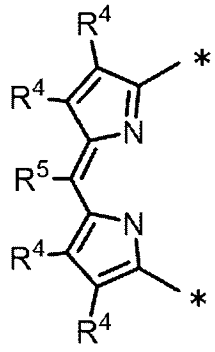

($P^a$) ($P^b$)

in Formula ($P^a$), $R^2$ and $R^3$ each independently represent a hydrogen atom or a substituent, two adjacent $R^2$ and two adjacent $R^3$ may be bonded to each other to form a ring structure, in Formula ($P^b$), $R^4$ and $R^5$ represent a hydrogen atom or a substituent, two adjacent $R^4$ and adjacent $R^4$ and $R^5$ may be bonded to each other to form a ring structure, a plurality of $R^4$ may be the same as or different from each other, and * represents a bond.

**5.** The ethylene production method according to claim 3, wherein the carbon dioxide reduction catalyst is a compound represented by the following Formula (2):

[Chem. 3]

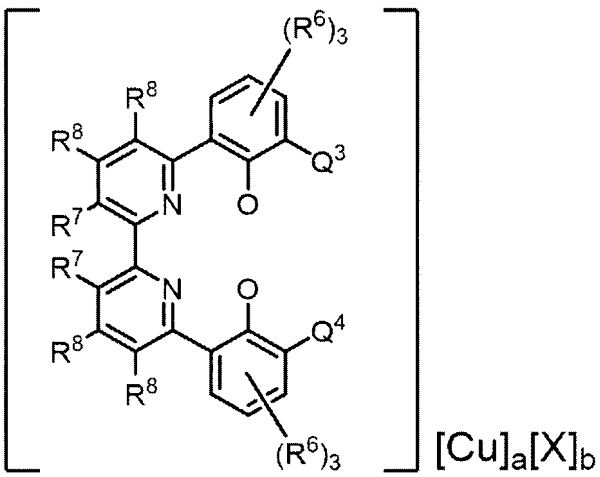

(2)

in Formula (2), $R^6$ to $R^8$ each independently represent a hydrogen atom or a substituent, two adjacent $R^6$, two adjacent $R^7$, and two adjacent $R^8$ may be bonded to each other to form a ring structure, a plurality of $R^6$ to $R^8$ may be the same as or different from each other, $Q^3$ and $Q^4$ represent a monovalent group containing one or more aromatic rings, $Q^3$ and $Q^4$ may be bonded to each other to form a ring structure, a is an integer of 2 or more and 4 or less, X is a counter ion or a neutral molecule, b is an integer of 0 or more, when a plurality of X are present, the plurality of X may be the same as or different from each other, and O is an oxygen atom and is bonded to at least one copper atom.

**6.** The ethylene production method according to claim 3, wherein the carbon dioxide reduction catalyst is a compound represented by the following Formula (3):

[Chem. 4]

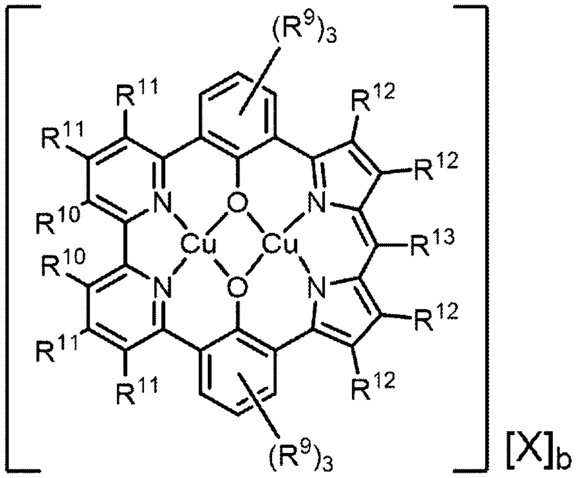

(3)

in Formula (3), $R^9$ to $R^{13}$ each independently represent a hydrogen atom, a substituent, or a divalent group, two adjacent $R^9$, two adjacent $R^{10}$, two adjacent $R^{11}$, two adjacent $R^{12}$, and adjacent $R^{12}$ and $R^{13}$ may be bonded to each other to form a ring structure, a plurality of $R^9$ to $R^{12}$ may be the same as or different from each other, when $R^{13}$ is a divalent group, the divalent group may form a bond with another compound represented by Formula (3) to form a dimer, X is a counter ion or a neutral molecule, b is an integer of 0 or more, and when a plurality of X are present, the plurality of X may be the same as or different from each other.

**7.** The ethylene production method according to claim 3, wherein the carbon dioxide reduction catalyst is a compound represented by the following Formula (4):

[Chem. 5]

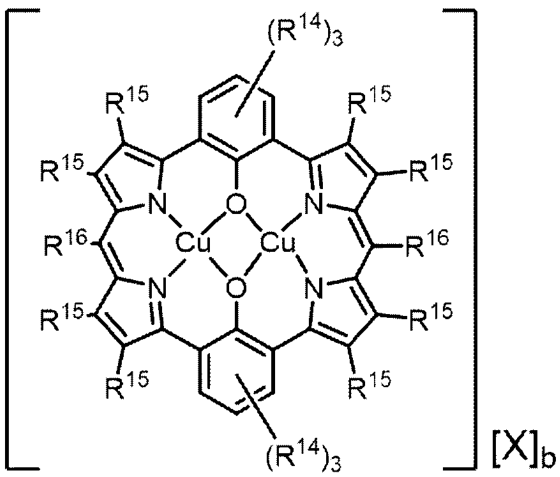

(4)

in Formula (4), $R^{14}$ to $R^{16}$ each independently represent a hydrogen atom or a substituent, two adjacent $R^{14}$, two adjacent $R^{15}$, and adjacent $R^{15}$ and $R^{16}$ may be bonded to each other to form a ring, a plurality of $R^{14}$ to $R^{16}$ may be the same as or different from each other, X is a counter ion or a neutral molecule, b is an integer of 0 or more, and when a plurality of X are present, the plurality of X may be the same as or different from each other.

**8.** The ethylene production method according to claim 3, wherein the carbon dioxide reduction catalyst is a compound represented by the following Formula (5):

[Chem. 6]

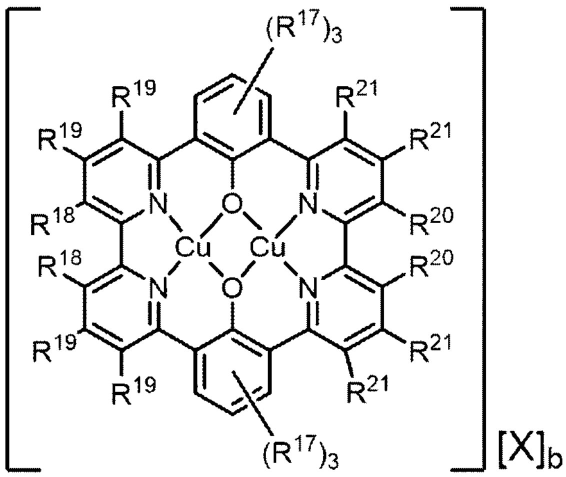

(5)

in Formula (5), $R^{17}$ to $R^{21}$ each independently represent a hydrogen atom or a substituent, two adjacent $R^{17}$, two adjacent $R^{18}$, two adjacent $R^{19}$, two adjacent $R^{20}$, and two adjacent $R^{21}$ may be bonded to each other to form a ring, a plurality of $R^{17}$ to $R^{21}$ may be the same as or different from each other, X is a counter ion or a neutral molecule, b is an integer of 0 or more, and when a plurality of X are present, the plurality of X may be the same as or different from each other.

**9.** The ethylene production method according to claim 3, wherein the carbon dioxide reduction catalyst is a compound represented by the following Formula (6):

[Chem. 7]

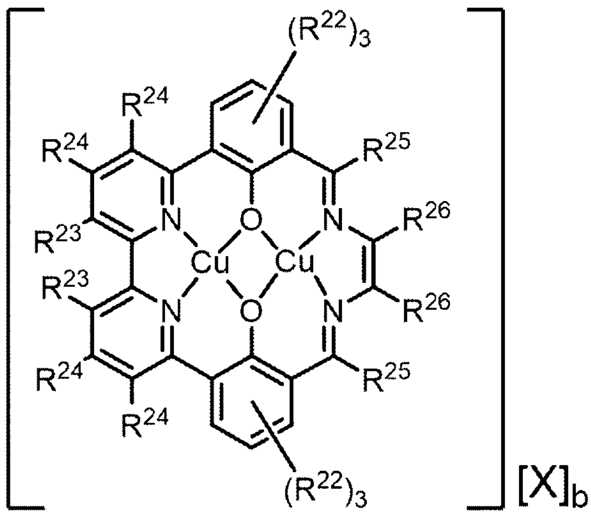

(6)

in Formula (6), $R^{22}$ to $R^{26}$ each independently represent a hydrogen atom or a substituent, two adjacent $R^{22}$, two adjacent $R^{23}$, two adjacent $R^{24}$, two adjacent $R^{26}$, and adjacent $R^{25}$ and $R^{26}$ may be bonded to each other to form a ring structure, a plurality of $R^{22}$ to $R^{26}$ may be the same as or different from each other, X is a counter ion or a neutral molecule, b is an integer of 0 or more, and when a plurality of X are present, the plurality of X may be the same as or different from each other.

**10.** The ethylene production method according to claim 3, wherein the carbon dioxide reduction catalyst is a compound represented by the following Formula (7):

[Chem. 8]

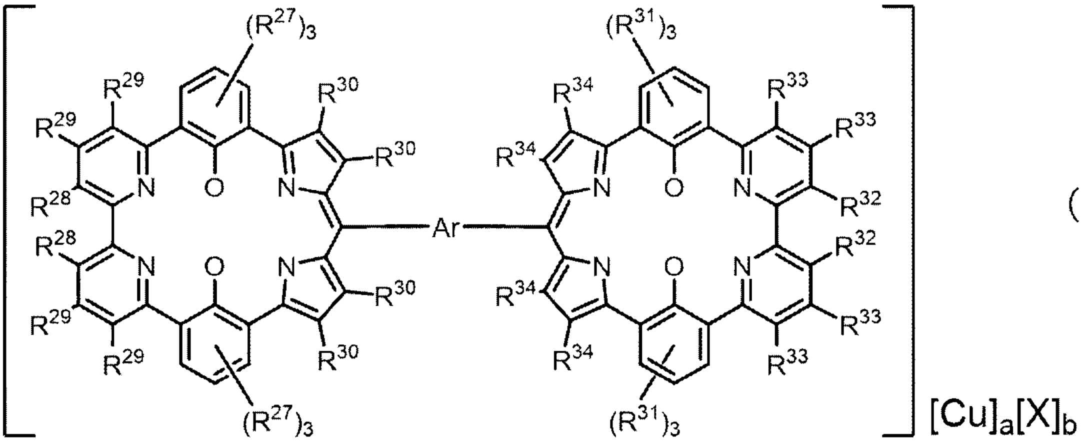

(7)

in Formula (7), $R^{27}$ to $R^{34}$ each independently represent a hydrogen atom or a substituent, two adjacent $R^{27}$, two adjacent $R^{28}$, two adjacent $R^{29}$, two adjacent $R^{30}$, two adj acent $R^{31}$, two adj acent $R^{32}$, two adj acent $R^{33}$, and two adjacent $R^{34}$ may be bonded to each other to form a ring structure, a plurality of $R^{27}$ to $R^{34}$ may be the same as or different from each other, Ar represents a divalent aromatic group which may have a substituent, a is an integer of 2 or more and 4 or less, X is a counter ion or a neutral molecule, b is an integer of 0 or more, when a plurality of X are present, the plurality of X may be the same as or different from each other, and O is an oxygen atom and is bonded to at least one copper atom.

**11.** A carbon dioxide reduction electrode comprising a carbon dioxide reduction catalyst that is a multinuclear copper complex and has a copper interatomic distance of 2.8 Å or less as determined by density functional theory or a conductive material on which a carbon dioxide reduction catalyst represented by the following Formula (1) is supported:

[Chem. 9]

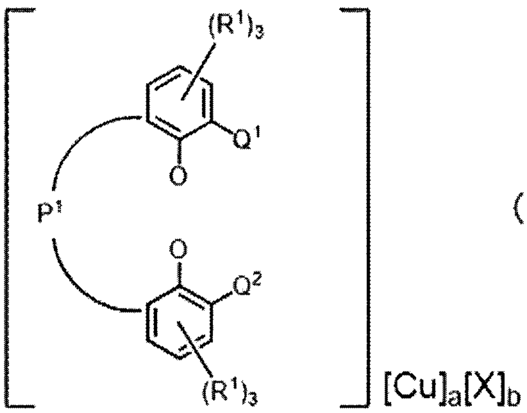

(1)

in Formula (1),

$R^1$ represents a hydrogen atom or a substituent, a plurality of $R^1$ may be the same as or different from each other, two adjacent $R^1$ may be bonded to each other to form a ring, $P^1$ represents a divalent group containing one or more aromatic rings, $Q^1$ and $Q^2$ represent a monovalent group containing one or more aromatic rings, $Q^1$ and $Q^2$ may be bonded to each other to form a ring structure, a is an integer of 2 or more and 4 or less, X is a counter ion or a neutral molecule, b is an integer of 0 or more, when a plurality of X are present, the plurality of X may be the same as or different from each other, and O is an oxygen atom and is bonded to at least one copper atom.

**12.** The carbon dioxide reduction electrode according to claim 11, further comprising a support that supports the conductive material.

**13.** The carbon dioxide reduction electrode according to claim 11 or 12, further comprising an ion conductor.

**14.** A carbon dioxide reduction apparatus comprising:

an oxidation electrode;
the carbon dioxide reduction electrode according to claim 11;
a membrane separating the oxidation electrode and the carbon dioxide reduction electrode;
an electrolyte solution; and
a power supply connected to the oxidation electrode and the carbon dioxide reduction electrode.

[Fig. 1]

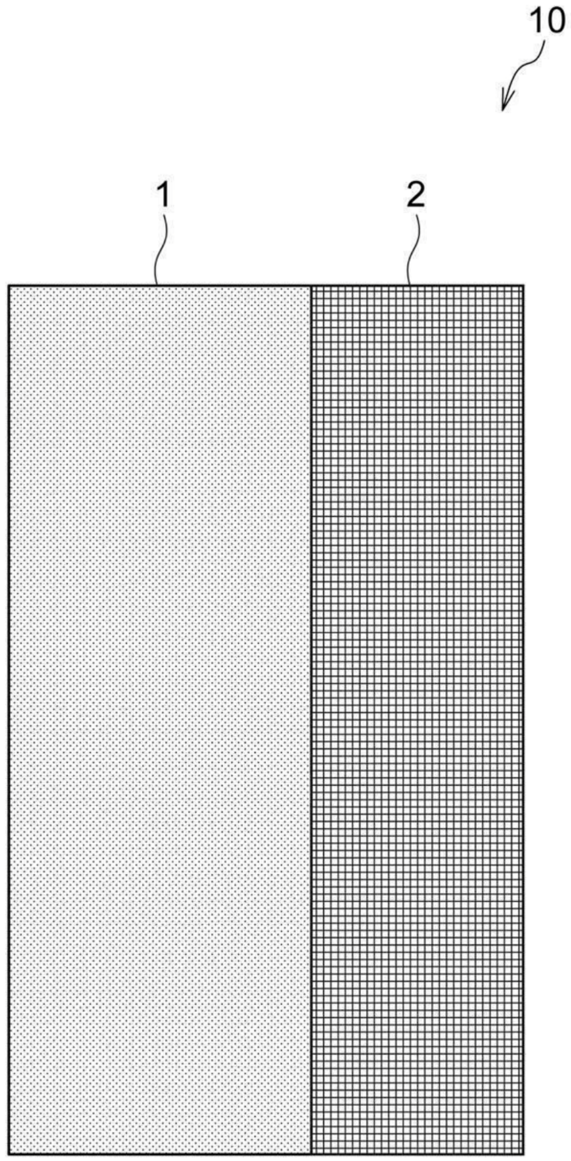

[Fig. 2]

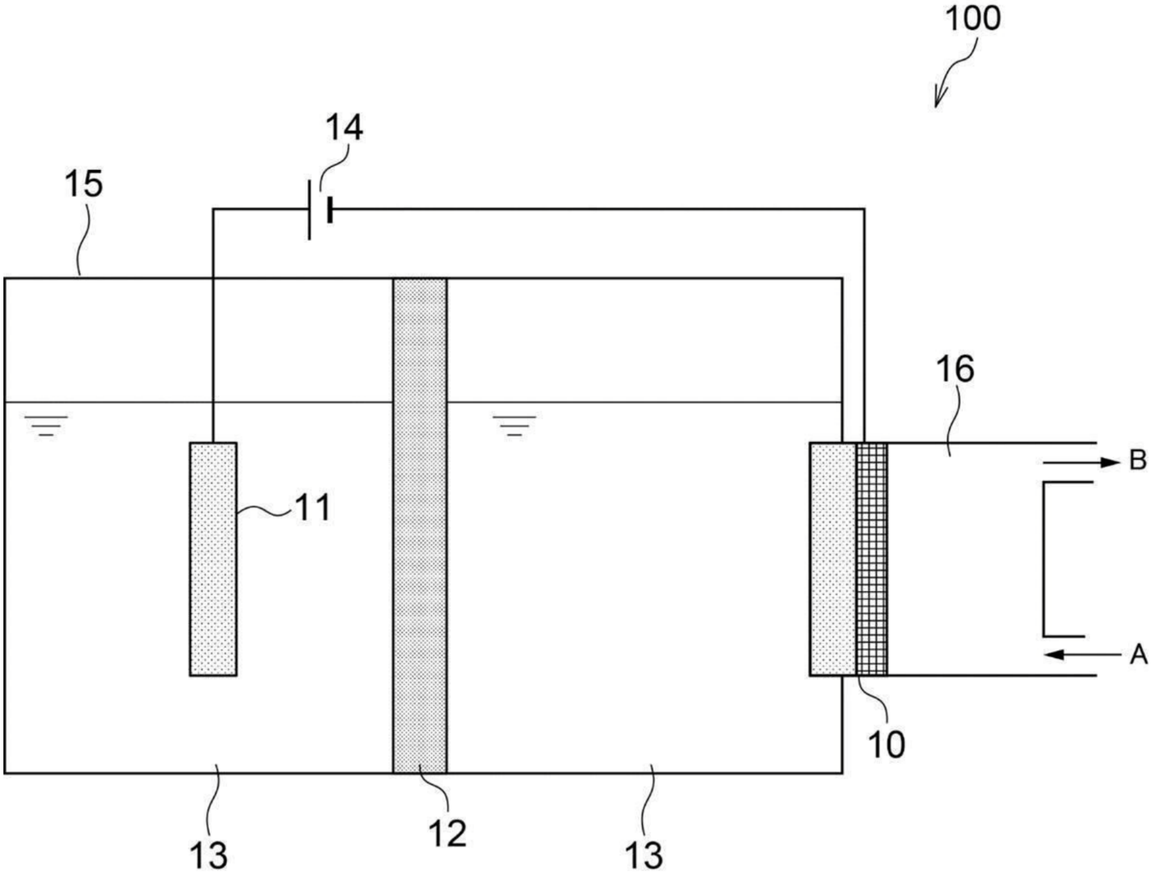

## INTERNATIONAL SEARCH REPORT

International application No.
**PCT/JP2024/001599**

**A. CLASSIFICATION OF SUBJECT MATTER**

***C25B 11/085***(2021.01)i; ***C07B 61/00***(2006.01)i; ***C07C 1/02***(2006.01)i; ***C07C 11/04***(2006.01)i; ***C07D 471/04***(2006.01)i; ***C07D 471/22***(2006.01)i; ***C07F 1/08***(2006.01)i; ***C25B 1/04***(2021.01)i; ***C25B 3/03***(2021.01)i; ***C25B 3/26***(2021.01)i; ***C25B 9/00***(2021.01)i; ***C25B 11/032***(2021.01)i; ***C25B 11/052***(2021.01)i; ***C25B 11/054***(2021.01)i; ***C25B 11/065***(2021.01)i; ***C25B 11/069***(2021.01)i

FI: C25B11/085; C07B61/00 300; C07C1/02; C07C11/04; C07D471/04 112; C07D471/22; C07F1/08 C; C25B1/04; C25B3/03; C25B3/26; C25B9/00 A; C25B9/00 G; C25B11/032; C25B11/052; C25B11/054; C25B11/065; C25B11/069

According to International Patent Classification (IPC) or to both national classification and IPC

**B. FIELDS SEARCHED**

Minimum documentation searched (classification system followed by classification symbols)

C25B11/085; C07B61/00; C07C1/02; C07C11/04; C07D471/04; C07D471/22; C07F1/08; C25B1/04; C25B3/03; C25B3/26; C25B9/00; C25B11/032; C25B11/052; C25B11/054; C25B11/065; C25B11/069

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); Scopus

**C. DOCUMENTS CONSIDERED TO BE RELEVANT**

| Category* | Citation of document, with indication, where appropriate, of the relevant passages | Relevant to claim No. |
|---|---|---|
| A | US 2020/0255961 A1 (RESEARCH TRIANGLE INSTITUTE) 13 August 2020 (2020-08-13) entire text | 1-14 |
| A | WO 2008/111569 A1 (NATIONAL INSTITUTE OF ADVANCED INDUSTRIAL & TECHNOLOGY) 18 September 2008 (2008-09-18) entire text | 1-14 |
| A | WO 2005/095408 A1 (NAGOYA INSTITUTE OF TECHNOLOGY) 13 October 2005 (2005-10-13) entire text | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 April 2024** | **16 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/001599**

**C. DOCUMENTS CONSIDERED TO BE RELEVANT**

| Category* | Citation of document, with indication, where appropriate, of the relevant passages | Relevant to claim No. |
|---|---|---|
| A | WO 2020/060887 A2 (THE REGENTS OF THE UNIVERSITY OF CALIFORNIA) 26 March 2020 (2020-03-26) entire text | 1-14 |
| A | KR 10-2014-0057057 A (SEOUL NATIONAL UNIVERSITY OF TECHNOLOGY CENTER FOR INDUSTRY COLLABORATION) 12 May 2014 (2014-05-12) entire text | 1-14 |

Form PCT/ISA/210 (second sheet) (July 2022)

**INTERNATIONAL SEARCH REPORT**
**Information on patent family members**

International application No.
**PCT/JP2024/001599**

| Patent document cited in search report | Publication date (day/month/year) | Patent family member(s) | Publication date (day/month/year) |
|---|---|---|---|
| US 2020/0255961 A1 | 13 August 2020 | (Family: none) | |
| WO 2008/111569 A1 | 18 September 2008 | US 2010/0129698 A1<br>entire text<br>EP 2133145 A1<br>CN 101674886 A<br>KR 10-2009-0127419 A | |
| WO 2005/095408 A1 | 13 October 2005 | (Family: none) | |
| WO 2020/060887 A2 | 26 March 2020 | (Family: none) | |
| KR 10-2014-0057057 A | 12 May 2014 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021109157 A
- WO 2019026883 A
- WO 2009084283 A
- JP 2009173627 A
- WO 2017073467 A

### Non-patent literature cited in the description

- *Tetrahedron*, 1999, vol. 55, 8377